(19) Europäisches Patentamt
European Patent Office
Office européen des brevets

(11) EP 4 755 924 A1

(12) EUROPEAN PATENT APPLICATION
published in accordance with Art. 153(4) EPC

(43) Date of publication:
**10.06.2026 Bulletin 2026/24**

(21) Application number: **24850478.9**

(22) Date of filing: **07.03.2024**

(51) International Patent Classification (IPC):
***C08B 37/00*** (2006.01) ***A61K 31/715*** (2006.01)
***A61K 36/074*** (2006.01) ***A61P 11/00*** (2006.01)
***A61P 35/00*** (2006.01)

(52) Cooperative Patent Classification (CPC):
**A61P 35/00; A61K 31/716; A61K 33/243; A61K 36/074; A61P 11/00; C08B 37/0003; C08B 37/0024;** A61K 2236/15; A61K 2236/331; A61K 2236/39; A61K 2236/51; Y02A 50/30

(86) International application number:
**PCT/CN2024/080444**

(87) International publication number:
**WO 2025/030818 (13.02.2025 Gazette 2025/07)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **04.08.2023 CN 202310990729**

(71) Applicant: **Shenzhen Yandai Investment Co., Ltd**
**Shenzhen, Guangdong 518000 (CN)**

(72) Inventors:
- **CHEN, Xuemin**
  **Shenzhen, Guangdong 518000 (CN)**
- **MO, Guan**
  **Shenzhen, Guangdong 518000 (CN)**
- **HE, Chunming**
  **Shenzhen, Guangdong 518000 (CN)**
- **CHEN, Jin**
  **Shenzhen, Guangdong 518000 (CN)**
- **LIU, Yunlei**
  **Shenzhen, Guangdong 518000 (CN)**
- **LI, Yanjun**
  **Shenzhen, Guangdong 518000 (CN)**
- **CHEN, Yao**
  **Shenzhen, Guangdong 518000 (CN)**
- **CHEN, Fengji**
  **Shenzhen, Guangdong 518000 (CN)**
- **LIANG, Mei**
  **Shenzhen, Guangdong 518000 (CN)**
- **FANG, Zhe**
  **Shenzhen, Guangdong 518000 (CN)**
- **SU, Yuanye**
  **Shenzhen, Guangdong 518000 (CN)**

(74) Representative: **Metida**
**Gyneju str. 16**
**01109 Vilnius (LT)**

# (54) POLYSACCHARIDE COMPOUND HAVING CLEAR MOLECULAR STRUCTURE AND SUITABLE FOR TREATING PULMONARY FIBROSIS

(57) This invention pertains to the field of plant extraction and separation technology and provides a polysaccharide compound, GLP-4, with a defined molecular structure, effective in treating emphysema and pulmonary fibrosis, and exhibiting antitumor effects. The molecular formula is: $(C_{162}H_{270}O_{135})_n$. The polysaccharide compound is obtained by extracting from a residue of Ganoderma lucidum processed with alkaline solution under high temperature and high pressure, followed by column chromatographic separation. The polysaccharide compound features excellent water solubility and is readily absorbed by the human body. The polysaccharide compound shows superior efficacy in treating emphysema and pulmonary fibrosis compared to pirfenidone and is also effective for patients with advanced cancer (those who are no longer surgical candidates, with a life expectancy of only three to six months, and still eligible for chemotherapy). Especially when used in combination with chemotherapy drugs, the polysaccharide compound can mitigate the toxic side effects caused by the chemotherapy drugs on the human body and has been supported by experimental data showing control and reduction of tumor masses, and reduction and elimination of cancer cells.

EP 4 755 924 A1

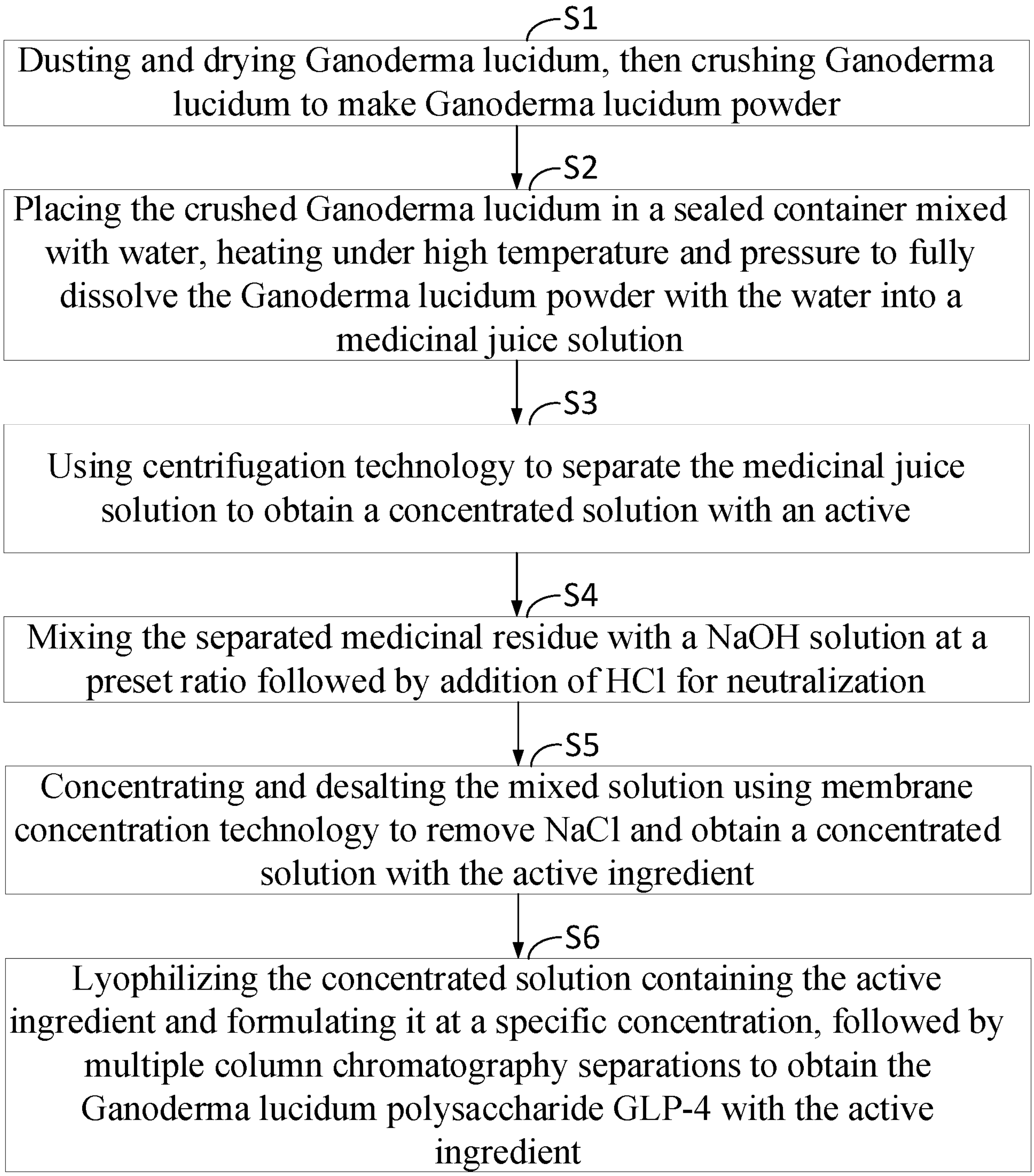
S1
Dusting and drying Ganoderma lucidum, then crushing Ganoderma lucidum to make Ganoderma lucidum powder
S2
Placing the crushed Ganoderma lucidum in a sealed container mixed with water, heating under high temperature and pressure to fully dissolve the Ganoderma lucidum powder with the water into a medicinal juice solution
S3
Using centrifugation technology to separate the medicinal juice solution to obtain a concentrated solution with an active
S4
Mixing the separated medicinal residue with a NaOH solution at a preset ratio followed by addition of HCl for neutralization
S5
Concentrating and desalting the mixed solution using membrane concentration technology to remove NaCl and obtain a concentrated solution with the active ingredient
S6
Lyophilizing the concentrated solution containing the active ingredient and formulating it at a specific concentration, followed by multiple column chromatography separations to obtain the Ganoderma lucidum polysaccharide GLP-4 with the active ingredient

Fig. 1

## Description

### Technical Field

The present invention relates to the field of improvements in plant extraction and separation technology, specifically relating to a method for extracting Ganoderma lucidum polysaccharide GLP-4 and its applications.

### Background

Ganoderma lucidum is a type of fungal plant with a long history of medicinal use in China and Japan. Ganoderma lucidum has a plurality of complex active ingredients, and over 150 compounds have been isolated from it, such as polysaccharides, triterpenes, sterols, alkaloids, furan derivatives, amino peptides, and inorganic elements. Geographic location (longitude and latitude), seed variation, growth environment, and differences in temperature, humidity, and light intensity can significantly affect the content, proportion, and presence of an active ingredient referred to in this invention in Ganoderma lucidum.

### Summary of Invention

The invention provides a polysaccharide compound with a defined molecular structure effective for treating emphysema, pulmonary fibrosis, and cancer.

The invention aims to address the current global challenge of treating emphysema and pulmonary fibrosis, for which no effective medication yet exists. The emergence of the Ganoderma lucidum polysaccharide compound GLP-4 holds promise to fill this gap. Moreover, this compound has shown excellent effects in the treatment of advanced cancer, particularly when used in combination with chemotherapy drugs as it can mitigate the toxic side effects caused by the chemotherapy drugs. It also plays a preventive, controlling, and therapeutic role in the transformation of pulmonary fibrosis and pulmonary nodules into lung cancer.

Another important function of the active ingredients specified in this invention is the prevention of mutations in normal human cells and the prevention of cancer cell formation.

The invention provides a method for extracting Ganoderma lucidum polysaccharide GLP-4, comprising the steps of:

S1. dusting and drying Ganoderma lucidum, then crushing Ganoderma lucidum to make Ganoderma lucidum powder;
S2. placing the crushed Ganoderma lucidum in a sealed container mixed with water, heating under high temperature and pressure to fully dissolve the Ganoderma lucidum powder with the water into a medicinal juice solution;
S3. using centrifugation technology to separate the medicinal juice solution to obtain a concentrated solution with an active ingredient and medicinal residue;
S4. mixing the separated medicinal residue with a NaOH solution at a preset ratio followed by addition of HCl for neutralization;
S5. concentrating and desalting the mixed solution using membrane concentration technology to remove NaCl and obtain a concentrated solution with the active ingredient; and
S6. lyophilizing the concentrated solution containing the active ingredient and formulating it at a specific concentration, followed by multiple column chromatography separations to obtain the Ganoderma lucidum polysaccharide GLP-4 with the active ingredient.

A further aspect of the invention: In step S2, the mixture of the Ganoderma lucidum powder and the water in the sealed container is fully stirred and heated at a high temperature to 105-200°C, with boiling time lasting for 2-6 h, during which the internal pressure in the sealed container gradually increases as the heating temperature rises, forming a high-temperature and high-pressure environment within the sealed container.

A further aspect of the invention: In step S2, the mixture of the Ganoderma lucidum powder and the water in the sealed container is heated at a high temperature to 105-170°C, with boiling time lasting for 3-6 h, during which the internal pressure in the sealed container gradually increases as the heating temperature rises, forming the high-temperature and high-pressure environment within the sealed container.

A further aspect of the invention: In step S6, pure water is added to the lyophilized powder of the concentrated solution containing the active ingredient to prepare a solution of 40-80 mg/mL, which then undergoes multiple column chromatography separations.

A further aspect of the invention: In step S4, the medicinal residue is mixed and soaked with a NaOH solution at a temperature of 40-100°C at a ratio of 1:10 to 1:40, with a soaking time of 1-5 h, where the concentration of NaOH is 0.05-0.5

mol/L.

A further aspect of the invention: In step S3, the Ganoderma lucidum residue is obtained from the extract by using centrifugation technology to separate the medicinal liquid and the Ganoderma lucidum residue for further extraction and purification.

A further aspect of the invention: In step S1, the Ganoderma lucidum is rinsed with clean water to remove surface dust and dried at 105°C, and the dried Ganoderma lucidum is crushed, with the crushed Ganoderma lucidum powder being larger than 60 mesh.

A further aspect of the invention: In step S2, the mixed liquid in the sealed container is heated at a high temperature to 105°C, 110°C, 115°C, 120°C, 125°C, 130°C, 135°C, 140°C, 145°C, 150°C, 155°C, 160°C, 165°C, 170°C, 175°C, 180°C, 185°C, 190°C, 195°C, or 200°C, with boiling times of 2 h, 2.5 h, 3 h, 3.5 h, 4 h, 4.5 h, 5 h, 5.5 h, or 6 h, during which the internal pressure in the sealed container gradually increases as the heating temperature rises, forming the high-temperature and high-pressure environment within the sealed container.

The invention also provides the Ganoderma lucidum polysaccharide GLP-4, wherein the structural formula of the Ganoderma lucidum polysaccharide GLP-4 is

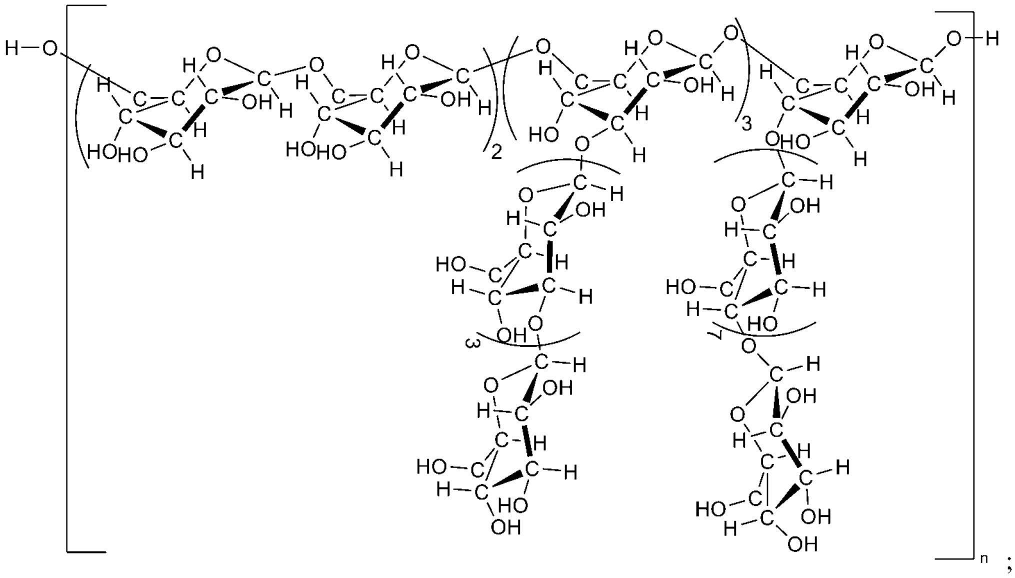

the molecular formula is $(C_{162}H_{270}O_{135})_n$, where n = 10-15.

A further aspect of the invention: n is 10, 11, 12, 13, 14, or 15.

This invention also provides an application of the Ganoderma lucidum polysaccharide GLP-4. The Ganoderma lucidum polysaccharide GLP-4 has good water solubility and is easily absorbed by the human body. It is effective in treating emphysema, pulmonary fibrosis, and has antitumor effects. Notably, its effects on treating emphysema and pulmonary fibrosis are exceptionally excellent, significantly better than those of pirfenidone. When used in combination with chemotherapy drugs, the Ganoderma lucidum polysaccharide GLP-4 can alleviate toxic side effects caused by the chemotherapy drugs on the human body, control and reduce tumor masses, and reduce and eliminate cancer cells.

The beneficial effects of this invention include a simple extraction process, sufficient use of the Ganoderma lucidum residue, low production cost, high polysaccharide yield, and ease of operation. The Ganoderma lucidum polysaccharide has good solubility, extremely low (or even no) toxic side effects, and is easily absorbed and degraded by the human body, thereby exerting therapeutic effects.

## Brief Description of the Drawings

Figure 1: Flowchart of the method for extracting Ganoderma lucidum polysaccharide GLP-4, provided by an embodiment of the invention.

Figure 2: Schematic diagram of lgMp-RT (peak molecular weight) calibration curves provided by an embodiment of the invention.

Figure 3: Schematic diagram of lgMp-RT (weight-average molecular weight) calibration curvew provided by an embodiment of the invention.

Figure 4: Schematic diagram of IgMp-RT (number-average molecular weight) calibration curves provided by an embodiment of the invention.

Figure 5: Schematic diagram of the molecular weight of Ganoderma lucidum polysaccharide GLP-4, provided by an embodiment of the invention.

Figure 6: Schematic diagram 1 of the ion chromatography of mixed standard 16 sugars, provided by an embodiment of the invention.

Figure 7: Schematic diagram 2 of the ion chromatography of mixed standard 16 sugars, provided by an embodiment of the invention.

Figure 8: GCMS chromatogram of the Ganoderma lucidum polysaccharide GLP-4 (PMAA) provided by an embodiment of the invention.

Figure 9: Schematic diagram 1 of the analysis results of permethylated alditol acetate (PMAA) of polysaccharide, provided by an embodiment of the invention.

Figure 10: Schematic diagram 2 of the analysis results of permethylated alditol acetate (PMAA) of polysaccharide, provided by an embodiment of the invention.

Figure 11: Schematic diagram 3 of the analysis results of permethylated alditol acetate (PMAA) of polysaccharide, provided by an embodiment of the invention.

Figure 12: Schematic diagram 4 of the analysis results of permethylated alditol acetate (PMAA) of polysaccharide, provided by an embodiment of the invention.

Figure 13: Schematic diagram 5 of the analysis results of permethylated alditol acetate (PMAA) of polysaccharide, provided by an embodiment of the invention.

Figure 14: Schematic diagram 6 of the analysis results of permethylated alditol acetate (PMAA) of polysaccharide, provided by an embodiment of the invention.

Figure 15: Schematic diagram of the hydrogen spectrum provided by an embodiment of the invention.

Figure 16: Schematic diagram of the carbon spectrum provided by an embodiment of the invention.

Figure 17: Schematic diagram of the Dept135 spectrum provided by an embodiment of the invention.

Figure 18: Schematic diagram of HH-COSY provided by an embodiment of the invention.

Figure 19: Schematic diagram of HSQC provided by an embodiment of the invention.

Figure 20: HMBC spectrum provided by an embodiment of the invention.

Figure 21: Schematic diagram of NOESY provided by an embodiment of the invention.

Figure 22: Schematic diagram of the effect of Ganoderma lucidum polysaccharide GLP-4 combined with chemotherapy on the tumor volume in LLC-bearing mice, provided by an embodiment of the invention.

Figure 23: Schematic diagram of the effect of Ganoderma lucidum polysaccharide GLP-4 combined with chemotherapy on the tumor pathology in LLC-bearing mice (×200), provided by an embodiment of the invention.

Figure 24: Schematic diagram of the effect of Ganoderma lucidum polysaccharide GLP-4 combined with chemotherapy on the renal pathology in LLC-bearing mice (×200), provided by an embodiment of the invention.

Figure 25: Schematic diagram of the effect of Ganoderma lucidum polysaccharide GLP-4 combined with chemotherapy on the gastric pathology in LLC-bearing mice (×200), provided by an embodiment of the invention.

Figure 26: Schematic diagram of the effect of Ganoderma lucidum polysaccharide GLP-4 combined with chemotherapy on the spleen pathology in LLC-bearing mice (×200), provided by an embodiment of the invention.

Figure 27: Schematic diagram of the control group of H22-bearing mouse model treated with Ganoderma lucidum polysaccharide GLP-4 combined with chemotherapy, provided by an embodiment of the invention.

Figure 28: Schematic diagram of the cisplatin group of H22-bearing mice treated with Ganoderma lucidum polysaccharide GLP-4 combined with chemotherapy, provided by an embodiment of the invention.

Figure 29: Schematic diagram of the cisplatin + low-dose GLP-4 group of H22-bearing mice treated with Ganoderma lucidum polysaccharide GLP-4 combined with chemotherapy, provided by an embodiment of the invention.

Figure 30: Schematic diagram of the cisplatin + high-dose GLP-4 group of H22-bearing mice treated with Ganoderma lucidum polysaccharide GLP-4 combined with chemotherapy, provided by an embodiment of the invention.

Figure 31: Schematic diagram of the control group of H22-bearing mouse tumor model treated with Ganoderma lucidum polysaccharide GLP-4 combined with chemotherapy, provided by an embodiment of the invention.

Figure 32: Schematic diagram of the cisplatin group of H22-bearing mouse tumor treated with Ganoderma lucidum polysaccharide GLP-4 combined with chemotherapy, provided by an embodiment of the invention.

Figure 33: Schematic diagram of the cisplatin + low-dose GLP-4 group of H22-bearing mouse tumor treated with Ganoderma lucidum polysaccharide GLP-4 combined with chemotherapy, provided by an embodiment of the invention.

Figure 34: Schematic diagram of the cisplatin + high-dose GLP-4 group of H22-bearing mouse tumor treated with Ganoderma lucidum polysaccharide GLP-4 combined with chemotherapy, provided by an embodiment of the invention.

Figure 35: Schematic diagram of the effect of Ganoderma lucidum polysaccharide GLP-4 combined with chemotherapy on the tumor of orthotopic H22-bearing mice (binning: 8 × 8; T = 30 s), provided by an embodiment of the invention.

Figure 36: Schematic diagram of the effect of Ganoderma lucidum polysaccharide GLP-4 combined with chemotherapy on the tumor of orthotopic H22-bearing mice, provided by an embodiment of the invention.

Figure 37: Schematic diagram of the effect of Ganoderma lucidum polysaccharide GLP-4 combined with chemotherapy on the liver of orthotopic H22-bearing mice (×200), provided by an embodiment of the invention.

Figure 38: Schematic diagram of the effect of Ganoderma lucidum polysaccharide GLP-4 combined with chemotherapy on the spleen of orthotopic H22-bearing mice (×200), provided by an embodiment of the invention.

Figure 39: Schematic diagram of the effect of Ganoderma lucidum polysaccharide GLP-4 combined with chemotherapy on the stomach of orthotopic H22-bearing mice (×200), provided by an embodiment of the invention.

Figure 40: Schematic diagram of the effect of Ganoderma lucidum polysaccharide GLP-4 combined with chemotherapy on the kidneys of orthotopic H22-bearing mice (×200), provided by an embodiment of the invention.

Figure 41: Confocal image of the RAW264.7 macrophage blank control group under 40x magnification, provided by an embodiment of the invention.

Figure 42: Confocal image of 0.25 mg/mL Ganoderma lucidum polysaccharide GLP-4 incubated with RAW264.7 macrophages for 24 h, under 40x magnification, provided by an embodiment of the invention.

Figure 43: Confocal image of 0.25 mg/mL Ganoderma lucidum polysaccharide GLP-4 incubated with RAW264.7 macrophages for 24 h, under 63x oil immersion magnification, provided by an embodiment of the invention.

Figure 44: Confocal image of 0.5 mg/mL Ganoderma lucidum polysaccharide GLP-4 incubated with RAW264.7 macrophages for 24 h, under 40x magnification, provided by an embodiment of the invention.

Figure 45: Confocal image of 0.5 mg/mL Ganoderma lucidum polysaccharide GLP-4 incubated with RAW264.7 macrophages for 24 h, under 63x oil immersion magnification, provided by an embodiment of the invention.

Figure 46: Schematic diagram of the effect of Ganoderma lucidum polysaccharide GLP-4 in combination with cisplatin on the necrosis and apoptosis rates of H22 cells, provided by an embodiment of the invention.

Figure 47: Schematic diagram of the control group of 4T1 breast tumor-bearing mouse model treated with Ganoderma lucidum polysaccharide GLP-4 combined with chemotherapy, provided by an embodiment of the invention.

Figure 48: Schematic diagram of the cisplatin group of 4T1 breast tumor-bearing mice treated with Ganoderma lucidum polysaccharide GLP-4 combined with chemotherapy, provided by an embodiment of the invention.

Figure 49: Schematic diagram of the cisplatin + low-dose GLP-4 group of 4T1 breast tumor-bearing mice treated with Ganoderma lucidum polysaccharide GLP-4 combined with chemotherapy, provided by an embodiment of the invention.

Figure 50: Schematic diagram of the cisplatin + high-dose GLP-4 group of 4T1 breast tumor-bearing mice treated with Ganoderma lucidum polysaccharide GLP-4 combined with chemotherapy, provided by an embodiment of the invention.

Figure 51: Schematic diagram of the control group of 4T1 breast tumor-bearing mouse tumor model treated with Ganoderma lucidum polysaccharide GLP-4 combined with chemotherapy, provided by an embodiment of the invention.

Figure 52: Schematic diagram of the cisplatin group of 4T1 breast tumor-bearing mouse tumor treated with Ganoderma lucidum polysaccharide GLP-4 combined with chemotherapy, provided by an embodiment of the invention.

Figure 53: Schematic diagram of the cisplatin + low-dose GLP-4 group of 4T1 breast tumor-bearing mouse tumor treated with Ganoderma lucidum polysaccharide GLP-4 combined with chemotherapy, provided by an embodiment of the invention.

Figure 54: Schematic diagram of the cisplatin + high-dose GLP-4 group of 4T1 breast tumor-bearing mouse tumor treated with Ganoderma lucidum polysaccharide GLP-4 combined with chemotherapy, provided by an embodiment of the invention.

Figure 55: Schematic diagram of mouse lung histopathology in the normal group (×40), provided by an embodiment of the invention.

Figure 56: Schematic diagram of mouse lung histopathology in the model control group (×40), provided by an embodiment of the invention.

Figure 57: Schematic diagram of mouse lung histopathology in the positive control group (×40), provided by an embodiment of the invention.

Figure 58: Schematic diagram of mouse lung histopathology in the Ganoderma lucidum polysaccharide GLP-4 low-dose group (×40 magnification), provided by an embodiment of the invention.

Figure 59: Schematic diagram of mouse lung histopathology in the Ganoderma lucidum polysaccharide GLP-4 high-dose group (×40 magnification), provided by an embodiment of the invention.

Detailed Description of the Embodiments

Embodiments of the present invention are described in detail below, and examples of the embodiments are shown in the drawings, wherein the same or similar reference numerals denote the same or similar elements or elements having the same or similar functions throughout. The embodiments below described by reference to the drawings are exemplary and are intended for the purpose of explaining the invention and should not be construed as limiting the scope of the invention.

As shown in Figure 1, the present invention provides a flowchart for the method for extracting the Ganoderma lucidum polysaccharide GLP-4, described in detail as follows:
In step S1, harvested Ganoderma lucidum, either raw or having undergone preliminary processing, is washed with clean water in washing equipment to remove surface dust. After dust removal, the Ganoderma lucidum is transferred to drying equipment for drying at a temperature of 105°C. The dried Ganoderma lucidum is then placed in a crusher to be broken down into Ganoderma lucidum powder. The Ganoderma lucidum powder is sieved, particles of the Ganoderma lucidum powder larger than 60 mesh are transferred to the next process, while particles of the Ganoderma lucidum powder smaller than 60 mesh are returned to the crusher for further crushing. This process is repeated multiple times until the crushed Ganoderma lucidum powder meets specified requirements.

In step S2, the Ganoderma lucidum powder that meets the requirements is mixed with pure water and placed in a sealed container. The sealed container is heated at high temperatures. As the temperature rises, the internal pressure of the sealed container gradually increases, creating a high-temperature and high-pressure environment that facilitates the thorough dissolution of the Ganoderma lucidum powder with the water to form a mixed solution. The sealed container is heated to a temperature between 105°C and 200°C, with a boiling time of 2-6 h, preferably between 105°C and 170°C, for 3-6 h. More preferably, the heating occurs at temperatures of 105°C, 110°C, 115°C, 120°C, 125°C, 130°C, 135°C, 140°C, 145°C, 150°C, 155°C, 160°C, 165°C, 170°C, 175°C, 180°C, 185°C, 190°C, 195°C, or 200°C, with a boiling time of 2 h, 2.5 h, 3 h, 3.5 h, 4 h, 4.5 h, 5 h, 5.5 h, or 6 h, ensuring full dissolution of the mixture to form a water solution containing the active ingredient.

In step S3, the water solution containing the active ingredient is separated from the Ganoderma lucidum residue using the centrifugation technology; the Ganoderma lucidum extract and the Ganoderma lucidum residue are obtained, with the Ganoderma lucidum extract used for further efficacy studies.

In step S4, the separated Ganoderma lucidum residue obtained after centrifugation is extracted by soaking in a NaOH solution at a solid-to-liquid ratio of 1:10 to 1:40, extraction temperature of 40-100°C, and extraction time of 1-5 h, where the concentration of sodium hydroxide is 0.05-0.5 mol/L. After extraction, hydrochloric acid is added for neutralization followed by centrifugation to remove the residue and obtain a mixed solution.

In step S5, the mixed solution is concentrated and desalted using the membrane concentration technology to remove NaCl and obtain a concentrated solution with the active ingredient.

In step S6, pure water is added to the lyophilized powder of the concentrated solution containing the active ingredient to prepare a solution of 40-80 mg/mL, and after multiple column chromatography separations and concentration lyophilization, Ganoderma lucidum polysaccharide GLP-4 is obtained.

This method features a simple extraction process with high polysaccharide yield, reuse of the Ganoderma lucidum residue, and low production costs.

The invention provides the Ganoderma lucidum polysaccharide GLP-4, wherein the structural formula of the Ganoderma lucidum polysaccharide GLP-4 is

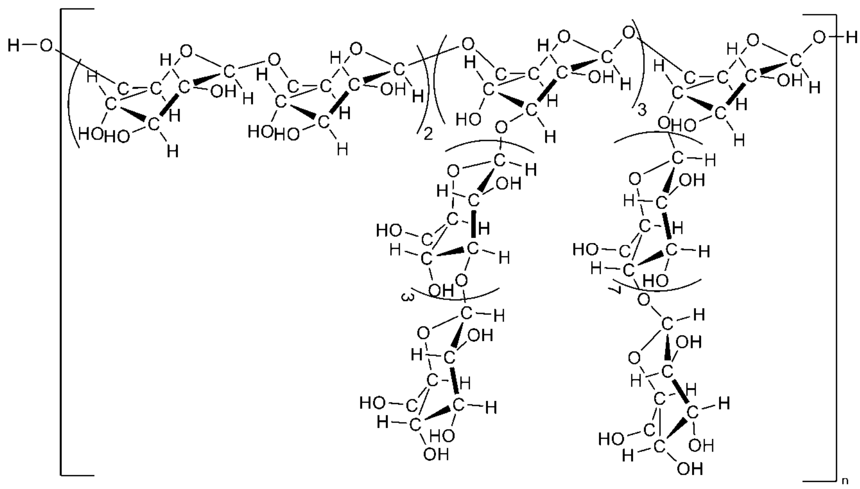

;

the molecular formula is $(C_{162}H_{270}O_{135})_n$, where n = 10-15.
n is 10, 11, 12, 13, 14, or 15.

Following the acquisition of the Ganoderma lucidum polysaccharide GLP-4 with the aforementioned structure, assay experiments were conducted and the following results are reported herein.

I. Molecular Weight Determination

1. Experimental Objective

To determine the molecular weight and purity of the polysaccharide GLP-4 using HPGPC.

2. Experimental Materials

2.1 Equipment

| Equipment name | Manufacturer | Model |
|---|---|---|
| High-performance liquid chromatography | Shimadzu | LC-10A |
| Differential refractometer | Shimadzu | RI-10A |
| BRT105-104-102 tandem gel permeation chromatography column | BoRui Saccharide | BRT105-104-102 (8 × 300 mm) |
| Electronic scale | Sartorius | CPA225D |
| Centrifuge | Eppendorf | Eppendorf5424 |
| Pipette | Sartorius | 200 uL, 1,000 uL |

2.2 Materials

| Reagent | Manufacturer | Batch No. | Catalog No. | Grade | Shelf life |
|---|---|---|---|---|---|
| NaCl | ACROS | A0356762 | 139725000 | ACROS | 2022 |

2.3 Standards

| Standard | Manufacturer | Batch No. | Storage conditions | Purity | Shelf life |
|---|---|---|---|---|---|
| Dextranstandards1152 | Yuanye BioTechnology | A16A8L41850 | Seal and store | 99% | 2 years |
| 5000 | Sigma | 102084138 | Seal and store | ≥99% | 2 years |
| 11600 | Sigma | 102136543 | Seal and store | 99% | 2 years |
| 23800 | Sigma | 102124529 | Seal and | ≥97% | 2 years |
| | | | store | | |
| 48600 | Sigma | 102104509 | Seal and store | ≥99% | 2 years |
| 80900 | Sigma | 102108375 | Seal and store | >98% | 2 years |
| 148000 | Sigma | 102089360 | Seal and store | >98% | 2 years |
| 273000 | Sigma | 102110878 | Seal and store | 98% | 2 years |
| 409800 | Sigma | 102124507 | Seal and store | >98% | 2 years |

(continued)

| Standard | Manufacturer | Batch No. | Storage conditions | Purity | Shelf life |
|---|---|---|---|---|---|
| 667800 | Sigma | 102104510 | Seal and store | >98% | 2 years |
| Molecular weight is measured in Daltons (Da). | | | | | |

3. Experimental Procedure

3.1 Reagent Preparation

| Reagent name | Preparation method | Storage conditions | Shelf life |
|---|---|---|---|
| 0.05M NaCl solution | Precisely prepared, filtered through a 0.45-μm membrane, degassed by sonication for 10 min | RT | 1 month |

3.2 Preparation of Sample and Standard Solutions

Samples and standards were precisely weighed to prepare a 5 mg/mL solution, which was centrifuged at 12,000 rpm for 10 min, and the supernatant was filtered through a 0.22-μm micropore filter. Then the sample was transferred to a 1.8-mL sample vial.

3.3 Chromatographic Method

Column: BRT105-104-102 tandem gel permeation chromatography column (8 × 300 mm); Mobile phase: 0.05M NaCl solution; Flow rate: 0.6 mL/min; Column temperature: 40°C; Injection volume: 20 μL; Detector: Differential refractometer RI-10A.

4. Experimental Results

As shown in Figures 2-4, calibration curves for lgMp-RT (peak molecular weight), lgMw-RT (weight-average molecular weight), and IgMn-RT (number-average molecular weight) were obtained.

The equation of the calibration curves for lgMp-RT is: $y = -0.1796x + 11.518R^2 = 0.9957$;
The equation of the calibration curves for lgMw-RT is: $y = -0.1914x + 12.069R^2 = 0.9944$;
The equation of the calibration curves for lgMn-RT is: $y = -0.1774x + 11.357R^2 = 0.9921$;

Using the standard curves, a formula was derived to calculate the molecular weight of each sample. The molecular weight chromatograms for the samples are shown in Figure 5, with the results detailed in the table below.

| Sample ID | RT (min) | lgMp | lgMw | lgMn | Mp | Mw | Mn | Peak Area Ratio (%) |
|---|---|---|---|---|---|---|---|---|
| | 37.931 | 4.7 | 4.8 | 4.6 | 50768 | 64418 | 42466 | 100 |
| Note: The peak at 46.1 min corresponds to the mobile phase. | | | | | | | | |

II. Monosaccharide Composition Determination Experiment

1. Experimental Objective

To determine the monosaccharide composition using an ion chromatograph.

2. Experimental Principle

This method is based on the electrochemical activity of sugar molecules and their ionization in strong alkaline solutions. Sugar compounds are weak acids with a pKa greater than 11. In high pH eluents, they partially or fully exist as

anions. Efficient anion exchange and separation of sugar compounds is achieved, which leverages differences in ion exchange due to variations in pKa of different sugars, as well as differences in hydrophobic interactions between some sugars and the anion exchange resin. Detection is then accomplished by measuring the current produced by the oxidation of hydroxyl groups in the sugar molecules at a gold electrode surface.

3. Experimental Materials

3.1 Equipment

| Equipment name | Manufacturer | Model |
|---|---|---|
| Ion chromatograph | ThermoFisher | ICS5000 |
| Electric thermostatic blast drying oven | LICHEN | 101-1BS |
| Nitrogen blower | LICHEN | UGC-24M |
| Electronic scale | Sartorius | BS 210 S |
| Centrifuge | ThermoFisher | D-37520 |
| Pipette | DRAGONLAB | 19050983 |

3.2 Reagents

| Reagent | Manufacturer | Batch No. | Catalog No. | Grade |
|---|---|---|---|---|
| Trifluoroacetic acid | ACROS | A0356762 | 139725000 | AR |
| 50% sodium hydroxide solution | Alfa Aesar | Z21E036 | 33382 | GR |
| Sodium acetate | ThermoFishe | 191126 | 059326 | GR |

3.3 Standards

| Standard | Manufacturer | Batch No. | Storage conditions | Purity |
|---|---|---|---|---|
| Mannose | Bo Rui Saccharide | C17D9H77586 | Seal and store | AR |
| Rhamnose | Bo Rui Saccharide | H10S9Z69863 | Seal and store | AR |
| Galacturonic acid | Bo Rui Saccharide | K02A9B66077 | Seal and store | AR |
| Galactose | Bo Rui Saccharide | E1927035 | Seal and store | AR |
| Glucose | Bo Rui Saccharide | Q18F10N80946 | Seal and store | AR |
| Glucuronic acid | Bo Rui Saccharide | K14M10S82777 | Seal and store | AR |
| Arabinose | Bo Rui Saccharide | S15A10G85850 | Seal and store | AR |
| Xylose | Bo Rui Saccharide | A22S6X3606 | Seal and store | AR |
| Fucose | Bo Rui Saccharide | X29D7Y27768 | Seal and store | AR |
| Glucosamine hydrochloride | Bo Rui Saccharide | A22S6X3606 | Seal and store | AR |
| N-acetyl-D-glucosamine | Bo Rui Saccharide | A21J8X40372 | Seal and store | AR |
| D-fructose | Bo Rui Saccharide | J01J10R89818 | Seal and store | AR |
| D-ribose | Bo Rui Saccharide | H26F10Z81556 | Seal and store | AR |

(continued)

| Standard | Manufacturer | Batch No. | Storage conditions | Purity |
|---|---|---|---|---|
| Galactosamine hydrochloride | Bo Rui Saccharide | B01J8S37079 | Seal and store | AR |
| L-guluronic acid | Bo Rui Saccharide | S200115AG1 | Seal and store | ≥98% |
| D-mannuronic acid | Bo Rui Saccharide | S200108AM1 | Seal and store | ≥98% |

4. Experimental Methods

4.1 Reagent Preparation

| Reagent name | Preparation method | Storage conditions |
|---|---|---|
| 15 mM NaOH solution | 2.4 g of 50% NaOH solution, 2 L of water | RT |
| 15 mM NaOH & 100 mM NaOAc solution | 1.2 g of 50% NaOH solution, 8.2 g of NaOAc, 1 L of water | RT |

4.2 Preparation and Calculation Method for Standard Solutions

Standard stock solutions were prepared using 16 different monosaccharide standards (fucose, rhamnose, arabinose, galactose, glucose, xylose, mannose, fructose, ribose, galacturonic acid, glucuronic acid, glucosamine hydrochloride, galactosamine hydrochloride, N-acetyl-D-glucosamine, guluronic acid, and mannuronic acid).

Concentration standards of each monosaccharide standard solution were accurately prepared and used as a mixed standard. The mass of different monosaccharides was determined using an absolute quantification method and the molar ratios were calculated based on the molar mass of each monosaccharide.

4.3 Sample Preparation

5 mg of the GLP-4 sample was accurately weighed in an ampule. 2 mL of 3M TFA was added and hydrolyzed at 120°C for 3 h. The acid hydrolysate was accurately transferred to a tube and evaporated to dryness under nitrogen. 5 mL water was added, vortexed to mix, and then 50 μL was taken and added to 950 μL of deionized water, and centrifuged at 12,000 rpm for 5 min. The supernatant was transferred for IC analysis.

4.4 Chromatographic Method

Column: Dionex Carbopac™ PA20 (3 × 150 mm); Mobile phase: A: $H_2O$; B: 15 mM NaOH; C: 15 mM NaOH & 100 mM NaOAc; Flow rate: 0.3 mL/min; Injection volume: 5 μL; Column temperature: 30°C; Detector: Electrochemical detector.

4.5 Standard Series

| No. | Name | ppm | Name | RT | Area |
|---|---|---|---|---|---|
| 1 | Fucose | 2.5 | Fuc | 6.05 | 10.197 |
| 2 | Galactosamine hydrochloride | 1.5 | GalN | 11.067 | 15.639 |
| 3 | Rhamnose | 2.5 | Rha | 11.592 | 5.514 |
| 4 | Arabinose | 1.1875 | Ara | 12.117 | 7.634 |
| 5 | Glucosamine hydrochloride | 2.5 | GlcN | 13.725 | 19.348 |
| 6 | Galactose | 2.5 | Gal | 15.092 | 9.099 |

(continued)

| No. | Name | ppm | Name | RT | Area |
|---|---|---|---|---|---|
| 7 | Glucose | 2.5 | Glc | 17.117 | 14.661 |
| 8 | N-acetyl-D-glucosamine 5 | 2.5 | GlcNAc | 19.05 | 9.446 |
| 9 | Xylose | 2.5 | Xyl | 19.825 | 14.858 |
| 10 | Mannose | 2.5 | Man | 20.492 | 8.594 |
| 11 | Fructose | 7.5 | Fru | 23.175 | 7.389 |
| 12 | Ribose | 5 | Rib | 25.65 | 5.959 |
| 13 | Galacturonic acid | 2.5 | GalA | 44.767 | 5.052 |
| 14 | Guluronic acid | 5 | GulA | 45.359 | 23.618 |
| 15 | Glucuronic acid | 2.5 | GlcA | 47.559 | 8.045 |
| 16 | Mannuronic acid | 5 | ManA | 50.3 | 10.868 |

$$C\ (\text{standard}) / A\ (\text{standard}) = C\ (\text{sample}) / A\ (\text{sample})$$

5. Experimental Results

Mixed standard: Solvent peaks at 2.0 min for sodium hydroxide and at 41 min for sodium acetate, as shown in Figures 6 and 7.

| **Name** | **Peak** area | **RT** | Molar Ratio |
|---|---|---|---|
| Fucose | 0 | 6.050 | 0.000 |
| Galactosamine hydrochloride | 0 | 11.067 | 0.000 |
| Rhamnose | 0 | 11.592 | 0.000 |
| Arabinose | 0 | 12.117 | 0.000 |
| Glucosamine hydrochloride | 0 | 13.725 | 0.000 |
| Galactose | 0 | 15.092 | 0.000 |
| Glucose | 67.385 | 16.925 | 0.905 |
| N-acetyl-D-glucosamine | 0 | 19.050 | 0.000 |
| Xylose | 0 | 19.825 | 0.000 |
| Mannose | 0 | 20.492 | 0.000 |
| Fructose | 0 | 23.175 | 0.000 |
| Ribose | 0 | 25.650 | 0.000 |
| Galacturonic acid | 0 | 44.767 | 0.000 |
| Guluronic acid | 0 | 45.359 | 0.000 |
| Glucuronic acid | 4.176 | 47.184 | 0.095 |
| Mannuronic acid | 0 | 50.300 | 0.000 |

III. Experiment on the Determination of Ganoderma Lucidum Polysaccharide GLP-4 Linkage

1. Experimental Objective

To determine the linkage patterns of Ganoderma lucidum polysaccharide GLP-4 samples through derivatization such as methylation by GC-MS analysis.

2. Experimental Materials

2.1 Equipment

| Equipment name | Manufacturer | Model |
|---|---|---|
| Rotary evaporator | Zhengzhou Greatwall Scientific Industrial and Trade Co., Ltd. | R-1001VN |
| Nitrogen blower | LICHEN | UGC-24M |
| Magnetic stirrer | DLAB | MS7-H550-Pro |
| Vacuum drying oven | LICHEN | 101-1BS |
| Gas chromatograph-mass spectrometer | Agilent | 6890-5973 |

2.2 Reagents

| Reagent | Manufacturer | Batch No. | Catalog No. | Grade |
|---|---|---|---|---|
| Trifluoroacetic | ACROS | A0356762 | 139725000 | AR |
| acid | | | | |
| Methyl iodide | Adamas | P1345479 | 01111630 | AR |
| Sodium borohydride | Aldrich | MKCD7945 | 205591 | AR |
| Ethyl acetate | Vokai | 08050003 | 40065982 | AR |
| Acetic anhydride | HUSHI | 20170314 | 10000318 | AR |
| Perchloric acid | Aldrich | SHBF7833V | 311421 | AR |
| Acetic acid | Fisher | 156174 | A35-500 | AR |
| Methanol | Merck | 10941735810 | 67-56-1 | AR |
| Sodium hydroxide | HUSHI | 20150429 | 10019718 | AR |
| Dimethyl sulfoxide | Adamas | P1265087 | 759270 | AR |
| Sodium hydride | Adamas | P1306059 | 81778A | AR |
| Methylation kit | Borui Saccharide | BRT-2020JJH | BRT-JJH | AR |

3. Experimental Methods

3.1 Reagent Preparation

| Reagent name | Preparation method | Storage conditions |
|---|---|---|
| 3M trifluoroacetic acid | 1V trifluoroacetic acid + 3V water | Store in refrigerator at 5°C |
| Sodium hydride dry powder | 60% sodium hydride washed with hexane | Store dry at room temperature |
| Sodium borodeuteride and sodium hydroxide solution | 20 mg + 20 mM NaOH solution | Seal and store |
| 20% acetic acid methanol solution | 1V glacial acetic acid + 4V water | Store in refrigerator at 5°C |
| Polysaccharide methylation kit | A: anhydrous alkaline solution | Store in refrigerator at 5°C |
| | B: methyl iodide solution | |

3.2 Sample Methylation

The sample underwent methylation, hydrolysis, and acetylation, followed by GC-MS analysis, which was compared with the standard mass spectral library.

2-3 mg of the Ganoderma lucidum polysaccharide GLP-4 sample was weighed and placed in a glass reaction vial, 1 mL of anhydrous DMSO was added, and methylation reagent A was added rapidly. The solution was sealed and dissolved under ultrasonication, then methylation reagent B was added. The reaction was allowed at 30°C in a magnetic stirring water bath for 60 min. Finally, 2 mL of ultrapure water was added to stop the methylation reaction.

The methylated polysaccharide was taken, 1 mL of 2M trifluoroacetic acid (TFA) was added, and hydrolyzed for 90 min. The rotary evaporator was used to dry. 2 mL of double-distilled water was added to the residue, which was reduced with 60 mg of sodium borohydride for 8 h, neutralized with glacial acetic acid, and evaporated using the rotary evaporator. Then, it was dried in a 101°C oven, then 1 mL of acetic anhydride was added for acetylation and reacted at 100°C for 1 h, and cooled. Then 3 mL of toluene was added, vacuum concentrated to dry, and this process was repeated 4-5 times to remove excess acetic anhydride.

The acetylated product was dissolved in 3 mL of $CH_2Cl_2$ and transferred to a separatory funnel. A small amount of distilled water was added and shaken thoroughly, then the upper aqueous layer was removed. This process was repeated four times. The $CH_2Cl_2$ layer was dried with an adequate amount of anhydrous sodium sulfate, brought to a volume of 10 mL, and placed in a vial for liquid analysis. The acetylated sample was analyzed using a Shimadzu GCMS-QP 2010 gas chromatograph-mass spectrometer;

GC-MS Conditions: RXI-5 SIL MS column 30 m × 0.25 mm × 0.25 μm; Temperature program: started at 120°C, increased at 3°C/min to 250°C, held for 5 min; Injector temperature at 250°C, detector temperature at 250°C, carrier gas was helium, flow rate was 1 mL/min.

4. Experimental Results

The GC-MS chromatogram of the sample (PMAA) is shown in Figure 8.

The permethylated alditol acetate (PMAA) analysis of the Ganoderma lucidum polysaccharide GLP-4 is presented in the following table and Figures 9-14.

| RT | Methylated sugar | Mass fragments (m/z) | Molar ratio | Type of linkage |
|---|---|---|---|---|
| 16.841 | 2,3,4,6-$Me_4$-Glcp | 45,71,87,101,117,129,145,161,205 | 0.279 | Glep-(1→ |
| 20.73 | 2,4,6-$Me_3$-Glcp | 45,87,99,101,117,129,161,173,233 | 0.269 | →3)-Glep-(1→ |
| 21.239 | 2,3,6-$Me_3$-Glcp | 45,87,99,101,113,117,129,131,161,173,233 | 0.204 | →4)-Glep-(1→ |
| 21.981 | 2,3,4-$Me_3$-Glcp | 43,87,99,101,117,129,161,189,233 | 0.110 | →6-Glep-(1→ |
| 26.446 | 2,3-$Me_2$-Glcp | 45,71,85,87,99,101,117,127,159,161,201,261 | 0.031 | →4,6)-Glep-(1→ |
| 26.75 | 2,4-$Me_2$-Glcp | 45,87,117,129,159,189,233 | 0.107 | →3,6)-Glep-(1→ |

IV. NMR Spectral Analysis and Interpretation

1. Experimental Materials and Equipment

Deuterium oxide ($D_2O$, 99.9%) and deuterated acetone as internal standard; freeze-dryer, Bruker 600M Nuclear Magnetic Resonance (NMR) spectrometer;

2. Experimental Procedure

50 mg of the polysaccharide GLP-4 sample was weighed and dissolved in 0.5 mL of deuterium oxide followed by freeze-drying. The lyophilized powder was redissolved in 0.5 mL of deuterium oxide and freeze-drying was continued. The process was repeated to ensure complete exchange of labile hydrogens. Subsequently, the sample was dissolve in 0.5 mL of deuterium oxide, and 1H NMR, 13C NMR, DEPT135 one-dimensional and two-dimensional spectral measurements were performed at 25°C using a 600 MHz NMR spectrometer.

3. Experimental Results

The hydrogen spectrum signals were primarily concentrated between 3.0 and 5.5 ppm. The signals for sugar ring protons were between δ3.2-4.0 ppm, with main terminal group protons peaks at δ4.68, 4.67, 4.46, 4.45, 4.44, and 4.43,

primarily distributed in the 4.3-5.5 ppm region as shown in Figure 15.

Carbon spectral analysis in $^{13}C$ NMR (201 MHz, $D_2O$): The NMR carbon spectrum signals were mainly concentrated between 60-120 ppm. Observations of the carbon spectrum indicated main anomeric carbon signal peaks at δ104.02, 103.92, 103.91, 103.83, and 103.66, primarily between 693-105. Other notable signal peaks were at δ71.47, 74.99, 69.23, 76.08, 62.09, 76.69, 74.09, 82.09, 76.43, 70.09, 74.61, 76.65, 76.01, 70.89, 70.13, 74.42, 85.43, 69.96, 77.31, 70.13, 74.16, 71.57, 82.28, 76.68, 61.66, 74.76, 85.64, 69.36, 77.11, and 62.03 ppm. Based on monosaccharide composition results, the polysaccharide is composed of glucose, indicating the polysaccharide GLP-4 is primarily glucan. This is depicted in Figure 16.

Dept135 spectral analysis showed inverted peaks at 60.70, 68.82, 61.66, 62.04, 70.28, and 68.79 ppm, indicative of chemical shifts for C6, This is depicted in Figure 17.

Figures 18-21 present HSQC spectra where the anomeric carbon signal at δ103.94 corresponds to anomeric hydrogen signal at δ4.68. HH-COSY identifies H1-2 signal at 4.68/3.44; H2-3 signal at 3.44/3.65; H3-4 signal at 3.65/3.41. We deduce H1, H2, H3, and H4 as δ4.68, 3.44, 3.65, and 3.41 respectively, corresponding to δ103.66, 74.76, 85.64, and 69.36. The corresponding C5 is at 77.11; the chemical shift of C6 is at δ62.03. Therefore, this signal is attributed to the glycosidic bond →3)-β-Glep-(1→.

Further HSQC observations show anomeric carbon signal at δ103.91, corresponding to anomeric hydrogen signal at δ4.44. HH-COSY identifies H1-2 signal at 4.44/3.23; H2-3 signal at 3.23/3.41; H3-4 signal at 3.41/3.55. We deduce H1, H2, H3, and H4 as δ4.44, 3.23, 3.41, 3.55 respectively, corresponding to C1-4 at δ103.92, 74.61, 76.65, and 76.01. NOESY spectra show correlated peaks at δ4.44 with 3.23, 3.41, 3.55, and 4.12. Dept135 combined with HSQC allows the attribution of δ3.77, 4.12 as peaks for H6a,b; H5 at 3.36 ppm. Corresponding C5 is at δ70.89; C6 chemical shift is at δ70.13, with H6a at δ3.77, 4.12. Therefore, this signal is attributed to the glycosidic bond →6)-β-Glep-(1→.

Using similar patterns and combining HMBC and NOESY, all glycosidic bond signals are assigned as shown in the following table:

Hydrogen and Carbon Signal Attribution

| Glycosyl residues | H1/C1 | H2/C2 | H3/C3 | H4/C4 | H5/C5 | H6a/C6 | H6b |
|---|---|---|---|---|---|---|---|
| β-D-Glep-(1→ →3)-β-D-Glep-(1→ →6)-β-D-Glep-(1→ →3,6)-β-D-Glep-(1→ →4,6)-β-D-Glep-(1→ →4)-β-D-Glep-(1→ | 4.67 | 3.52 | 3.68 | 3.74 | 3.65 | 3.83 | 3.64 |
| | 104.02 | 71.47 | 74.99 | 69.23 | 76.08 | 62.09 | |
| | 4.68 | 3.44 | 3.65 | 3.41 | 3.42 | 3.66 | 3.84 |
| | 103.66 | 74.76 | 85.64 | 69.36 | 77.11 | 62.03 | |
| | 4.44 | 3.23 | 3.41 | 3.55 | 3.36 | 3.77 | 4.12 |
| | 103.92 | 74.61 | 76.65 | 76.01 | 70.89 | 70.13 | |
| | 4.43 | 3.29 | 3.68 | 3.42 | 3.67 | 3.77 | 4.12 |
| | 103.91 | 74.42 | 85.43 | 69.96 | 77.31 | 70.13 | |
| | 4.46 | 3.26 | 3.43 | 3.58 | 3.79 | 3.77 | 4.12 |
| | 104.02 | 76.69 | 74.09 | 82.09 | 76.43 | 70.09 | |
| | 4.45 | 3.29 | 3.62 | 3.59 | 3.42 | 3.8 | 3.64 |
| | 103.83 | 74.16 | 71.57 | 82.28 | 76.68 | 61.66 | |

**Main Chain Analysis:**

From the HMBC spectra, based on the one-dimensional and two-dimensional NMR spectra, we have attributed the signals of the glycosidic bonds in the polysaccharide GLP-4. Analyzing the chemical shifts in the proton spectrum below 5 ppm, the monosaccharide composition is glucose, thus, it is a β-glucan. The anomeric hydrogen of the glycosidic bond →6)-β-D-Glcp-(1→ shows correlation signal peaks with its own C6, indicating the presence of a →6)-β-D-Glcp-(1→ 6)-β-D-Glcp-(1→ linkage.

The anomeric hydrogen of the glycosidic bond →6)-(3-D-Glcp-(1→ shows correlation signal peaks with the C6 of →3,6)-β-D-Glcp-(1→, indicating the presence of a →6)-β-D-Glcp-(1→3,6)-β-D-Glcp-(1→ linkage.

The anomeric hydrogen of the glycosidic bond →3,6)-β-D-Glcp-(1→ shows correlation signal peaks with its own C6, indicating the presence of a →3,6)-β-D-Glcp-(1→4,6)-β-D-Glcp-(1→ linkage.

The anomeric hydrogen of the glycosidic bond →3,6)-β-D-Glcp-(1→ shows correlation signal peaks with the C6 of →4,6)-β-D-Glcp-(1→, indicating the presence of a →3,6)-β-D-Glcp-(1→4,6)-β-D-Glcp-(1→ linkage.

**Branch Chain Analysis:**

From the HMBC spectra, the anomeric hydrogen of the glycosidic bond β-D-Glcp-(1→ shows correlation peaks with the H3 of →3)-β-D-Glcp-(1→, indicating the presence of a β-D-Glcp-(1→3)-β-D-Glcp-(1→ linkage.

The anomeric hydrogen of the glycosidic bond →3)-β-D-Glcp-(1→ shows correlation peaks with the H3 of →3)-β-D-Glcp-(1→, indicating the presence of a β-D-Glcp-(1→3)-β-D-Glcp-(1→ linkage.

The anomeric hydrogen of the glycosidic bond →3)-β-D-Glcp-(1→ shows correlation peaks with the H4 of →3,6)-β-D-Glcp-(1→, indicating the presence of a →3)-β-D-Glcp-(1→3,6)-β-D-Glcp-(1→ linkage.

The anomeric hydrogen of the glycosidic bond →4)-β-D-Glcp-(1→ shows correlation peaks with the H4 of →4,6)-β-D-Glcp-(1→, indicating the presence of a →4)-β-D-Glcp-(1→4,6)-β-D-Glcp-(1→ linkage.

Based on the above analysis, we can deduce that the main chain of this polysaccharide GLP-4 is a β-1,6 glucan. Additionally, the (3-D-Glcp-(1→3)-(3-D-Glcp-(1→ and P-D-Glcp-(1→4)-p-D-Glcp-(1→ are linked to the main chain through the O-3 bond of →3,6)-β-D-Glcp-(1→ and the O-4 bond of →4,6)-β-D-Glcp-(1→, respectively. The condensed molecular structure is illustrated below.

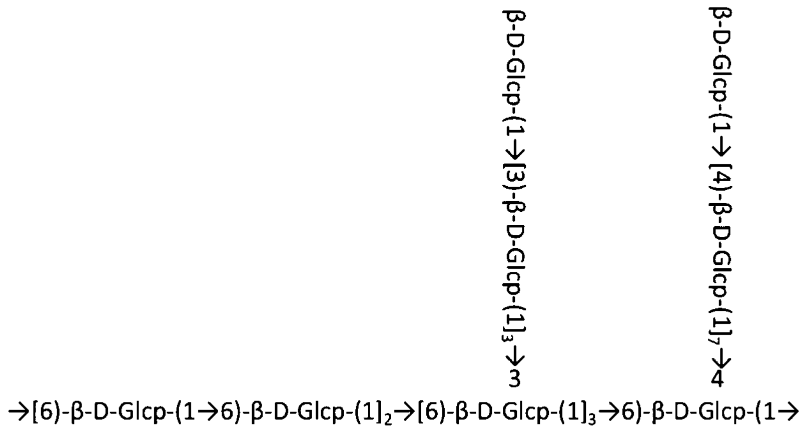

This invention also provides an application of the Ganoderma lucidum polysaccharide GLP-4. The Ganoderma lucidum polysaccharide GLP-4 has good water solubility and is easily absorbed by the human body. It is effective in treating emphysema, pulmonary fibrosis, and has antitumor effects. Notably, its effects on treating emphysema and pulmonary fibrosis are exceptionally excellent, significantly better than those of pirfenidone. When used in combination with chemotherapy drugs, the Ganoderma lucidum polysaccharide GLP-4 can alleviate toxic side effects caused by the chemotherapy drugs on the human body, control and reduce tumor masses, and reduce and eliminate cancer cells.

The cases describing the antitumor effects of the Ganoderma lucidum polysaccharide GLP-4 are as follows:

**Experimental Study on the Antitumor Effect of Ganoderma Lucidum Polysaccharide GLP-4 Combined with Chemotherapy on LLC-Bearing Mice and Study Data**

**Experimental Objective**

To study the antitumor effect of Ganoderma lucidum polysaccharide GLP-4 on lung cancer-bearing mice which are prepared by implanting LLC tumors in the right axilla of C57 mice. This study aims to provide experimental evidence for clinical studies of GLP-4.

**Experimental Materials**

**Test Sample**

Ganoderma lucidum polysaccharide GLP-4, batch number: OLMJT202108(2-4), provided by Shenzhen Aolimei Oncology Medical Technology Co., Ltd.

**Positive Control**

Kanglaite soft capsules, batch number: 20200802, Zhejiang Kanglaite Pharmaceutical Co., Ltd.; cisplatin injection, batch number: 601210104, Jiangsu Hansoh Pharmaceutical Co., Ltd.

### Laboratory Animals

80 SPF-grade male C57 mice, weighing 12-15 g, provided by Hunan SJA Laboratory Animal Co., Ltd. Laboratory animal production license number: SCXK (Xiang) 2019-0004; laboratory animal quality certification number: 430727211103153225. The animals were housed in Barrier Environment Laboratory D of Hunan Puruima Pharmaceutical Research Center Co., Ltd., under the laboratory animal use license number: SYXK (Xiang) 2020-0015.

### Main Reagents

0.9% sodium chloride injection, batch number: 21071401C, Hunan Kangyuan Pharmaceutical Co., Ltd.; ALP assay kit, batch number: 201223, Maccura Biotechnology; TP assay kit, batch number: 105467, ALB assay kit, batch number: 105468, ALT assay kit, batch number: 105475, AST assay kit, batch number: 104446, TBIL assay kit, batch number: 105465, GGT assay kit, batch number: 104447, all manufactured by Wako Pure Chemical Industries, Ltd. of Japan.

### Main Instruments

S10 Portable High-Speed Disperser, Scientz; AR223CN Electronic Scale, Ohaus Instruments (Changzhou) Co., Ltd.; LABOSPECT003 Automatic Biochemical Analyzer, Hitachi, Japan; TDZ5-WS Benchtop Multi-Tube Automatic Balance Centrifuge, Hunan Kaida Industrial Development Co., Ltd.; ME2002E Electronic Scale, Mettler-Toledo Instruments (Shanghai) Co., Ltd.; Digital Caliper, Yongkang Zhengfeng Hardware Co., Ltd.; Flow Cytometer, BD Biosciences; ASP200S Fully Automatic Tissue Dehydrator, ASP300S Fully Automatic Tissue Dehydrator, TP1020 Fully Automatic Dehydrator, HI1210 Slide Spreader, HI1220 Slide Dryer, RM2235 Paraffin Microtome, EG1150H+C Tissue Embedding Station, AutoStainer XL Automatic Slide Stainer + CV5030 Automatic Cover Slipper, Leica, Germany; BX43 Biological Microscope + MD50 Digital Imaging System, CX31 Biological Microscope, Olympus, Japan.

### Experimental Methods

LLC cells in logarithmic growth phase at $1 \times 10^7$/mL were inoculated at 0.2 mL into the right axilla of 10 purchased normal male C57 mice. Tumor growth was monitored, and tumor-bearing mice were prepared. Once the tumor volume in tumor-bearing mice exceeded 200 $mm^3$, the tumor tissue was aseptically harvested to prepare a tissue cell suspension. This was mixed at a 1:1 (V:V) ratio with culture medium to create a tumor tissue homogenate. This homogenate was injected into the right axilla of 60 male C57 mice at 0.2 mL per mouse. When the tumor volume in these tumor-bearing mice exceeded 100 $mm^3$, mice with well-grown tumors without ulceration were selected and randomized into groups based on tumor volume as follows: model control group, cisplatin group (4 mg/kg), Kanglaite soft capsule group (1,404 mg/kg), cisplatin + Kanglaite soft capsule group (4 + 1,404 mg/kg), cisplatin + GLP-4 low-dose group (4 + 130 mg/kg), and cisplatin + GLP-4 high-dose group (4 + 1,170 mg/kg), with 8 mice per group. Additionally, 8 mice were selected as a normal control group. The cisplatin group received intraperitoneal injections of cisplatin, and the other groups were administered their respective drug solutions by gavage (20 mL/kg) or intraperitoneal injection (10 mL/kg), once daily for 27 consecutive days. After the last dose, blood was collected from the orbital plexus to test blood WBC, RBC, liver and kidney function indicators (ALT, AST, BUN, CRE), and $CD3^+$, $CD4^+$, and $CD8^+$ levels. Bone marrow smears were examined, and spleens, thymuses, and tumors were weighed. Histopathological examinations were conducted on the tumor, spleen, stomach, and kidneys.

### Dosage Design

Based on preliminary study findings, Ganoderma lucidum polysaccharide GLP-4 was administered at a low dose of 130 mg/kg and a high dose of 1,170 mg/kg. The doses for each respective drug administered in this experiment are shown in Table 1, and the clinical intended dose was used in equivalent proportions.

The proposed clinical dose for Kanglaite soft capsules is 0.45 g per capsule, 6 capsules per dose, 4 doses per day, which totals 10.8 g per day. When converted to an equivalent mouse dose based on body surface area, it is calculated as 10.8 g/day $\times$ 0.0026 / 0.02 kg = 1,404 mg/kg. This study used the proposed clinical dose as a basis for the experiment.

Rationale for cisplatin dosage design: Based on the clinical dosage of cisplatin, which should not exceed 100 $mg/m^2$ per person per day, and considering the tolerance of mice to cisplatin, a dose of 4 mg/kg has been selected as the administration dosage.

Table 1: Experimental groups and dosage design

| Group | Dose (mg/kg) | Method of administration | Frequency of administration |
|---|---|---|---|
| Normal control group | - | Oral gavage | Once daily |
| Model control group | - | Intraperitoneal injection | Once every 2 days |
| Cisplatin group | 4 | Intraperitoneal injection | Once every 3 days |
| Kanglaite soft capsule group | 1404 | Oral gavage | Once daily |
| Cisplatin + Kanglaite soft capsule group | 4+1404 | Intraperitoneal injection + oral gavage | Cisplatin, once every 3 days; Kanglaite soft capsule, once daily |
| Cisplatin + GLP-4 low-dose group | 4+130 | Intraperitoneal injection + oral gavage | Cisplatin, once every 3 days; GLP-4, once daily |
| Cisplatin + GLP-4 high-dose group | 4+1170 | Intraperitoneal injection + oral gavage | Cisplatin, once every 3 days; GLP-4, once daily |

**Test Indicators**

**Efficacy Indicators**

**Evaluation of quality of life and survival time in animals:** Throughout the experimental process, detailed records were kept for each group of animals regarding weight, mortality, mental state, fur color, smoothness of fur, and fecal form, including incidences of diarrhea (if any).

**Tumor volume measurement:** Tumor dimensions (length and width) were measured with a caliper every three days post-dose, and the tumor volume was calculated as follows: tumor volume (TV) = 1 / 2 × a (length) × $b^2$ (width). Relative tumor volume (RTV) = $TV_t$ / $TV_0$, where $TV_0$ is the tumor volume at the time of administration, and $TV_1$ is the tumor volume at each measurement. Relative tumor growth rate T/C (%) = $T_{RTV}$ / $C_{RTV}$ × 100%, where $T_{RTV}$ is the RTV for the treatment group and $C_{RTV}$ is the RTV for the control group. Tumor growth inhibition rate (%) = (model control group tumor weight - treatment group tumor weight) / model control group tumor weight × 100%. A tumor growth inhibition rate (%) of < 40% is considered ineffective; a tumor growth inhibition rate (%) of ≥ 40% with $P \leq 0.05$ is considered effective.

**Spleen and thymus organ coefficients:** After the last dose, spleen, thymus, and tumor weights are measured, and organ coefficients are calculated. organ coefficient (%) = organ weight / fasting body weight × 100%.

**Potentiation and Detoxification**

**Hematological tests:** Blood samples for routine blood tests (WBC and RBC) and liver and kidney function tests (BUN and CRE) were collected from the orbital plexus after the last dose.

**Bone marrow examination:** After the last dose, bone marrow was collected for smear tests, and changes in bone marrow cells were observed in each group of mice.

**Histopathology examination:** After the last dose, tissues from tumors, spleens, stomachs, and kidneys were collected for H&E staining to observe histopathological changes.

**Blood $CD4^+$ and $CD8^+$ content measurement:** After the last dose, lymphocyte subtypes $CD4^+$ and $CD8^+$ in the blood were measured using flow cytometry.

**Data Processing and Statistical Analysis**

Significant figures of the study data were rounded according to the nearest whole number. Statistical analysis was conducted in accordance with the center's *SOP*, using SPSS software. Measurement data are expressed as mean ± standard deviation ($\overline{X} \pm s$), and normality and homogeneity of variance were tested using Leven's test. If not statistically significant ($P > 0.05$), one-way ANOVA was used for statistical analysis. If ANOVA was statistically significant ($P \leq 0.05$), the LSD test (parametric method) was used for comparison analysis. If the variance was not homogeneous ($P \leq 0.05$), the Kruskal-Wallis test was employed. If the Kruskal-Wallis test was statistically significant ($P \leq 0.05$), the Dunnett's Test (non-parametric method) was used for comparison analysis. Statistical significance was determined at $\alpha = 0.05$, where $P \leq 0.05$ indicates statistical significance and $P \leq 0.01$ indicates highly significant differences.

**Experimental Results**

**General Clinical Observation and Mortality in Animals**

Prior to administration, the mice exhibited normal activity and gait. In the model control group, mice 2M06 and 2M03 died on January 20, 2022 and January 21, 2022, respectively. In the Kanglaite soft capsule group, mouse 4M01 died on January 19, 2022. In the cisplatin + Kanglaite soft capsule group, mice 5M01 and 5M04 died on January 14, 2022 and January 22, 2022, respectively. In the cisplatin + GLP-4 low-dose group, mice 8M06/08 and 8M05/07 died on January 12, 2022 and January 15, 2022, respectively. In the cisplatin + GLP-4 high-dose group, mice 9M05/06 and 9M08 died on January 8, 2022 and January 23, 2022, respectively.

As shown in Table 2, the mortality rates for each group are 0%, 25.0%, 0%, 12.5%, 25.0%, 50.0%, and 37.5%, respectively.

Table 2: Statistics on the number of surviving animals and mortality rate in each group

| Group | Dose (mg/kg) | Number of animals | Number of deaths | Number of surviving animals | Mortalit y rate (%) |
|---|---|---|---|---|---|
| Normal control group | - | 8 | 0 | 8 | 0 |
| Model control group | - | 8 | 2 | 6 | 25.0 |
| Cisplatin group | 4 | 8 | 0 | 8 | 0 |
| Kanglaite soft capsule group | 1404 | 8 | 1 | 7 | 12.5 |
| Cisplatin + Kanglaite soft capsule group | 4+1404 | 8 | 2 | 6 | 25.0 |
| Cisplatin + GLP-4 low-dose group | 4+130 | 8 | 4 | 4 | 50.0 |
| Cisplatin + GLP-4 high-dose group | 4+1170 | 8 | 3 | 5 | 37.5 |

**Effect of Ganoderma Lucidum Polysaccharide GLP-4 Combined with Chemotherapy on Body Weight of LLC-Bearing Mice**

As shown in Table 3, compared to the normal control group, the body weight of the mice in the model control group significantly decreased after administration on D4 ($P \leq 0.05$). Compared to the model control group, the body weight of mice in the cisplatin group and cisplatin + Kanglaite soft capsule group significantly decreased after administration from D7 to D25 ($P \leq 0.01$); the body weight of mice in the cisplatin + GLP-4 low-dose and high-dose groups significantly decreased after administration from D4 to D25 ($P \leq 0.05$ or $P \leq 0.01$); the body weight of mice in the Kanglaite soft capsule group significantly decreased after administration from D10 to D19. Compared to the cisplatin group, the body weight of mice in the Kanglaite soft capsule group significantly increased after administration from D7 to D25 ($P \leq 0.05$ or $P \leq 0.01$); the body weight of mice in the cisplatin + GLP-4 high-dose group significantly decreased after administration on D7 ($P \leq 0.05$); the body weight of mice in the cisplatin + GLP-4 low-dose group significantly decreased after administration from D7 to D13 ($P \leq 0.05$ or $P \leq 0.01$). Compared to the Kanglaite soft capsule group, the body weight of mice in the cisplatin + Kanglaite soft capsule group and GLP-4 low-dose and high-dose groups significantly decreased after administration from D7 to D25 ($P \leq 0.05$ or $P \leq 0.01$). Compared to the cisplatin + Kanglaite soft capsule group, the body weight of mice in the cisplatin + GLP-4 low-dose group significantly decreased after administration from D7 to D13 ($P \leq 0.05$ or $P \leq 0.01$), with no statistical significance in the other groups.

Table 3: Effect of Ganoderma lucidum polysaccharide GLP-4 combined with chemotherapy on body weight of LLC-bearing mice ( $\bar{x} \pm s$ )

| Group | Weight (g) | | | | | | | | |
|---|---|---|---|---|---|---|---|---|---|
| | D1 | D4 | D7 | D10 | D13 | D16 | D19 | D22 | D25 |
| Normal control group | 15.2 ± 0.6 | 16.8 = 0.8 | 17.6 ± 1.0 | 17.9 ± 0.6 | 19.7 ± 0.8 | 21.0 ± 1.1 | 22.1 = 1.5 | 21.4 ± 1.1 | 22.1 ± 1.2 |
| Model control group | 14.3 ± 1.1 | **15.6 ± 1.2+** | 17.1 ± 1.5 | 17.9 ± 1.6 | 19.3 ± 1.3 | 20.9 ± 1.2 | 21.9 ± 1.0 | 21.7 ± 1.0 | 22.4 ± 2.0 |
| Cisplatin group | 141 ± 0.7 | 14.5 = 1.1 | **14.1 ± 1.7**** | **13.5 ± 1.5**** | **13.0 ± 1.9**** | **13.8 ± 2.3**** | **133 ± 2.3**** | **133 ± 2.0**** | **14.0 ± 2.3**** |

(continued)

| Group | Weight (g) | | | | | | | | |
|---|---|---|---|---|---|---|---|---|---|
| | D1 | D4 | D7 | D10 | D13 | D16 | D19 | D22 | D25 |
| Kanglaite soft capsule group | 13-4 ± 1 5 | 14.8 = 1.5 | **16.1 ± 1.5**$^{\#\#}$ | **15.6 ± 1.7**$^{**\#}$ | **16.9 ± 2.1**$^{*\#\#}$ | **18.3 ± 1.7**$^{*\#\#}$ | **18.8 ± 2.1**$^{**\#\#}$ | **20.2 ± 1.5**$^{\#\#}$ | **215 ± 1.4"** |
| Cisplatin + Kanglaite soft capsule group | 14-7 ± 0.7 | 14.6 ± 0.7 | **13.8 ± 0.7**$^{**\&\&}$ | **13.4 ± 1.3**$^{**\&\&}$ | **13.2 ± 1.6**$^{**\&\&}$ | **13.7 ± 1.7**$^{**\&\&}$ | **139 ± 1.6**$^{**\&\&}$ | **135 ± 1.7**$^{**\&\&}$ | **14.0 ± 1.5**$^{**\&\&}$ |
| Cisplatin + GLP-4 low-dose group | 14.0 ±1.2 | **13.8 ± 1.1**$^{**}$ | **11.8±1.1**$^{**\#\#\&\&\star\star}$ | **11.2 ± 1.4**$^{**\#\#\&\&\star}$ | **11.0 ± 1.8**$^{**\#\&\&\star}$ | **12.9 ± 4.4**$^{**\&\&}$ | **15.0 ± 3.6**$^{**\&\&}$ | **12.0 ± 2.3**$^{**\&\&}$ | **123 ± 23**$^{**\&\&}$ |
| Cisplatin + GLP-4 high-dose group | 14.3 ± 1.2 | **13.9 ± 1.9**$^{**}$ | **12.4 ± 1.6**$^{***\&\&}$ | **12.6 ± 2.0**$^{**\&\&}$ | **13.0 ± 2.6**$^{**\&\&}$ | **12.7 ± 2.9**$^{**\&\&}$ | **139 ± 3.6**$^{**\&\&}$ | **123 ± 2.9**$^{**\&\&\$}$ | **13.0 ± 3.1**$^{**\&\&}$ |

Note: Compared to the normal control group, $^{+}P \le 0.05$; compared to the model control group, $^{*}P \le 0.05$, $^{**}P \le 0.01$; compared to the cisplatin group, $^{\#}P \le 0.05$, $^{\#\#}P \le 0.01$; compared to the Kanglaite soft capsule group, $^{\&}P \le 0.05$, $^{\&\&}P \le 0.01$; compared to the cisplatin + Kanglaite soft capsule group, $^{\star}P \le 0.05$, $^{**}P \le 0.01$.

**Effect of Ganoderma Lucidum Polysaccharide GLP-4 Combined with Chemotherapy on Tumor Volume and Relative Tumor Volume in LLC-Bearing Mice**

As shown in Table 4, compared to the model control group, the tumor volume of mice in the cisplatin + GLP-4 high-dose group significantly decreased after administration from D10 to D25 (*P* ≤ 0.05 or *P* ≤ 0.01), the tumor volume of mice in the cisplatin + GLP-4 low-dose group significantly decreased after administration on D4 and from D10 to D25 *(*P ≤ 0.05 or *P* ≤ 0.01), the tumor volume of mice in the cisplatin group significantly decreased after administration on D10 and from D16 to D25 (*P* ≤ 0.05 or *P* ≤ 0.01), and the tumor volume of mice in the cisplatin + Kanglaite soft capsule group significantly decreased after administration from D13 to D25 (*P* ≤ 0.05 or *P* ≤ 0.01). Compared to the cisplatin group, the tumor volume of mice in the cisplatin + GLP-4 low-dose group significantly decreased after administration from D4 to D16 *(P* ≤ 0.05), and the tumor volume of mice in the Kanglaite soft capsule group significantly increased after administration from D7 to D25 (*P* ≤ 0.05 or *P* ≤ 0.01). Compared to the Kanglaite soft capsule group, the tumor volume of mice in the cisplatin + GLP-4 high-dose group and the Kanglaite soft capsule group significantly decreased after administration from D13 to D25 (*P* ≤ 0.05 or *P* ≤ 0.01), and the tumor volume of mice in the cisplatin + GLP-4 low-dose group significantly decreased after administration from D4 to D7 and from D13 to D25 (*P* ≤ 0.05 or *P* ≤ 0.01).

As shown in Table 5, compared to the model control group, the relative tumor volume of mice in the cisplatin group, the cisplatin + Kanglaite soft capsule group, and the cisplatin + GLP-4 low-dose and high-dose groups significantly decreased after administration from D13 to D25 (*P* ≤ 0.05 or *P* ≤ 0.01). Compared to the cisplatin + Kanglaite soft capsule group, the tumor volume of mice in the cisplatin + GLP-4 low-dose group significantly decreased after administration from D4 to D13 *(P* ≤ 0.05 or *P* ≤ 0.01), with no statistical significance in the other groups.

Table 4: Effect of Ganoderma lucidum polysaccharide GLP-4 combined with chemotherapy on tumor volume in LLC-Bearing Mice ( $\bar{x} \pm s$)

| Group | Tumor volume ($mm^3$) ($mm^3$) | | | | | | | | |
|---|---|---|---|---|---|---|---|---|---|
| | D1 | D4 | D7 | D10 | D13 | D16 | D19 | D22 | D25 |
| Model control group | 94.63 ± 20.97 | 178.51 ± 57.95 | 19.72 ± 12.58 | 26.4 ± 18.02 | 42.45 ± 20.82 | 145.78 ± 62.96 | 218.08 ± 69.66 | 3025.26 ± 804.69 | 4173.23 ± 1009.80 |
| Cisplatin group | 94.99 ± 1116 | 150.58 ± 34.33 | 8.91 ± 2.56 | **6.51 ± 4.74**$^{*}$ | 27.36 ± 24.4 | **36.09 ± 27.67**$^{**}$ | **71.00 ± 49.93**$^{**}$ | **824.01 ± 435.48**$^{**}$ | **976.79 ± 427.46**$^{**}$ |
| Kanglaite soft capsule group | 94.81 ± 13.17 | 146.63 ± 27.20 | **31.53 ± 12.76**$^{\#}$ | **17.13 ± 3.68**$^{\#}$ | **33.8 ± 14.5**$^{\#}$ | **106.86 ± 26.94**$^{\#}$ | **164.23 ± 33.41**$^{\#}$ | **2559.02 ±439.54**$^{\#}$ | **4270.10** ± 883.45$^{\#\#}$ |
| Cisplatin + Kanglaite soft capsule group | 95.88 ± 14.41 | 172.38 ± 31.19 | **18.53 = 6.71** | **10.34 ± 6,25** | **2.56 ± 1.08**$^{**\&}$ | **4.21 ± 2.78**$^{**\&\&}$ | **18.59 ± 9.44**$^{**\&\&}$ | **218.87 ± 120.00**$^{**\&\&}$ | **781.75 ± 520.71**$^{**\&\&}$ |
| Cisplatin + GLP-4 low-dose group | 95.78 ± 15.62 | **66.64±14.49**$^{*\#\&\star\star}$ | **5.76 ± 3.65**$^{\#\&\star}$ | **0.44 ± 0.44**$^{**\#\star}$ | **0.00 ± 0.00**$^{**\#\&\star}$ | **0.00 ± 0.00**$^{**\#\&\&}$ | **2.21 ± 1.28**$^{**\&}$ | **70.84 ± 47.07**$^{****}$ | **116.95 ± 65.48**$^{**\&\&}$ |
| Cisplatin + GLP-4 high-dose group | 94.97 ± 13.21 | 104.42 ± 12.06 | 5.57 ± 1.67 | **0.63 ± 0.63**$^{**}$ | **0.39 ± 0.39**$^{**\&}$ | **0.00 ± 0.00**$^{**\&\&}$ | **3.84 ± 2.66**$^{**\&\&}$ | **85.0747**$^{**\&\&}$ **74%** $^{24k}$ | **138.09±63.05**$^{**\&\&}$ |

Note: Compared to the model control group, $^{*}P \leq 0.05$, $^{**}P \leq 0.01$; compared to the cisplatin group, $^{\#}P \leq 0.05$, $^{\#\#}P \leq 0.01$; compared to the Kanglaite soft capsule group, $^{\&}P \leq 0.05$, $^{\&\&}P \leq 0.01$; compared to the cisplatin + Kanglaite soft capsule group, $^{\star}P \leq 0.05$, $^{\star\star}P \leq 0.01$.

Table 5: Effect of Ganoderma lucidum polysaccharide GLP-4 combined with chemotherapy on relative tumor volume in LLC-Bearing Mice ($\bar{x} \pm s$)

| Group | Relative tumor volume | | | | | | | |
|---|---|---|---|---|---|---|---|---|
| | D4 | D7 | **D10** | D13 | D16 | D19 | D22 | D25 |
| Model control group | 2.26 ± 2.05 | 0.22 ± 0.43 | 0.33 ± 0.62 | 0.57 ± 0.79 | 1.81 ± 2.36 | 2.63 ± 2.77 | 34.34 29.91 | 51.36 ± 28.59 |
| Cisplatin group | 1.84 ± 1.42 | 0.10 ± 0.07 | 0.09 ± 0.20 | **0.40 ± 1.04*** | **0.51 ± 1.18*** | **0.97 ± 2.12*** | **10.62 ± 18.36*** | **12.10 ± 17.51*** |
| Kanglaite soft capsule group | 2.03 ± 1.89 | 0.53 ± 0.79 | 0.22 ± 0.15 | 0.42 ± 0.54 | 1.20 ± 0.93 | 1.83 ± 1.15 | **29.27** ± 11.91 | 48.39 ± 17.21 |
| Cisplatin + Kanglaite soft capsule group | 2.32 ± 1.75 | 0.31 ± 0.39 | 0.22 ± 0.36 | **0.04 ± 0.08**** | **0.06 ± 0.09**** | **0.41 ± 0.86*** | **2.51 ± 3.38**** | **± 8.02 ± 13.07*** |
| Cisplatin + GLP-4 low-dose group | 0.95 ± 0.76 | 0.06 ± 0.07 | 0.01 ± 0.01 | **0.00 ± 0.00**** | **0.00 ± 0.00**** | **0.02 ± 0.02**** | **0.62 ± 0.80**** | **1.10 ± 1.06*** |
| Cisplatin + GLP-4 high-dose group | 1.29 ± 0.82 | 0.06 ± 0.06 | 0.01 ± 0.02 | **0.01 ± 0.01**** | **0.00± 0.00**** | **0.07 ± 0.12**** | **1.37 ± 2.11**** | **2.12 ± 2.72**** |

Note: Compared to the model control group, *$P \leq 0.05$, **$P \leq 0.01$.

**Effect of Ganoderma Lucidum Polysaccharide GLP-4 Combined with Chemotherapy on Relative Tumor Growth Rate (T/C) in LLC-Bearing Mice**

As shown in Table 6, the T/C ratios for mice in the cisplatin + GLP-4 low-dose from D4 to D25 after administration were: 42.0%, 27.3%, 3.0%, 0.0%, 0.0%, 0.8%, 1.8%, and 2.1%, all values ≤ 60.0%. For the cisplatin + GLP-4 high-dose group, the T/C ratios from D4 to D25 were: 57.1%, 27.3%, 3.0%, 0.0%, 0.0%, 2.7%, 4.0%, and 4.1%, all values ≤ 60.0%. For the cisplatin group, T/C ratios from D7 to D10 and D16 to D25 were: 45.5%, 27.3%, 28.2%, 36.9%, 30.9%, and 23.6%, all values ≤ 60.0%. For the cisplatin + Kanglaite soft capsule group, T/C ratios from D13 to D25 were: 7.3%, 3.3%, 15.6%, 7.3%, and 15.6%, all values ≤ 60.0%.

Table 6: Effect of Ganoderma lucidum polysaccharide GLP-4 combined with chemotherapy on relative tumor growth rate in LLC-Bearing Mice ($\bar{x} \pm s$)

| Group | Relative tumor growth rate (%) | | | | | | | |
|---|---|---|---|---|---|---|---|---|
| | D4 | D7 | D10 | D13 | D16 | D19 | D22 | D25 |
| Model control group | -- | -- | -- | -- | -- | -- | -- | -- |
| Cisplatin group | 81.4 | **45.5** | **27.3** | 70.2 | **28.2** | **36.9** | **30.9** | **23.6** |
| Kanglaite soft capsule group | 89.8 | 240.9 | 66.7 | 73.7 | 66.3 | 69.6 | 85.3 | 94.2 |
| Cisplatin + Kanglaite soft capsule group | 102.7 | 140.9 | 63.6 | **7.0** | **3.3** | **15.6** | **7.3** | **15.6** |
| Cisplatin + GLP-4 low-dose group | **42.0** | **27.3** | **3.0** | **0.0** | **0.0** | **0.8** | **1.8** | **2.1** |
| Cisplatin + GLP-4 high-dose group | **57.1** | **27.3** | **3.0** | **0.0** | **0.0** | **2.7** | **4.0** | **4.1** |

Effect of Ganoderma Lucidum Polysaccharide GLP-4 Combined with Chemotherapy on Organ Coefficients and Tumor Growth Inhibition Rates in LLC-Bearing Mice

As shown in Table 7, compared to the normal control group, the spleen and tumor coefficients in the model control group significantly increased ($P \leq 0.01$), while the thymus coefficient significantly decreased ($P \leq 0.01$). Compared to the model control group, the spleen, thymus, and tumor coefficients in the cisplatin group, cisplatin + GLP-4 low-dose group, and Kanglaite soft capsule group all significantly decreased ($P \leq 0.05$ or $P \leq 0.01$), and the spleen and tumor coefficients in the cisplatin + GLP-4 high-dose group significantly decreased ($P \leq 0.01$). Compared to the cisplatin group, the spleen and

tumor coefficients in the Kanglaite soft capsule group significantly increased (P ≤ 0.01). Compared to the Kanglaite soft capsule group, the spleen and tumor coefficients in the cisplatin + GLP-4 high-dose group significantly decreased (*P* ≤ 0.01), and the spleen, thymus, and tumor coefficients in the cisplatin + GLP-4 low-dose group significantly decreased (*P* ≤ 0.05 or P ≤ 0.01). There was no significant statistical difference in the organ-to-body weight ratios between groups compared to the cisplatin + Kanglaite soft capsule group.

The tumor growth inhibition rate in the Kanglaite soft capsule group was -6.8%, all values < 40.0%; for the cisplatin group, cisplatin + Kanglaite soft capsule group, cisplatin + GLP-4 low-dose and high-dose groups, the tumor growth inhibition rates were 61.6%, 78.3%, 88.6%, and 94.2% respectively, all values ≥ 40.0%.

Table 7: Effect of Ganoderma lucidum polysaccharide GLP-4 combined with chemotherapy on organ coefficients and tumor growth inhibition rates in LLC-bearing mice ($\bar{x} \pm s$ , n = 4-8)

| Group | Spleen coefficient (%) | Thymus coefficient (%) | Tumor coefficient (%) | Tumor growth inhibition rate (%) |
|---|---|---|---|---|
| Normal control group | 0.368 ±0.009 | 0.286 ±0.018 | 0.000 ± 0.000 | |
| Model control group | **1.336 ± 0.280$^{++}$** | **0.128 ± 0.042$^{++}$** | **19.581 ± 3.559$^{++}$** | |
| Cisplatin group | **0.456 ± 0.102$^{**}$** | **0.044 ± 0.020$^{*}$** | **7.528 ±2.567$^{**}$** | **61.6** |
| Kanglaite soft capsule group | **1.307 ± 0.195$^{\#\#}$** | 0.102 ±0.034 | **20.918 ± 3.245$^{\#\#}$** | -6.8 |
| Cisplatin + Kanglaite soft capsule group | **0.396 ± 0.064$^{**\&\&}$** | **0.053 ± 0.014$^{*}$** | **4.247 ± 2.338$^{**\&\&}$** | **78.3** |
| Cisplatin + GLP-4 low-dose group | **0.324 ± 0.072$^{**\&\&}$** | **0.014 ± 0.014$^{**\&}$** | **2.231 ± 1.714$^{**\&\&}$** | **88.6** |
| Cisplatin + GLP-4 high-dose group | **0.567 ± 0.271$^{**\&\&}$** | 0.088 ±0.009 | **1.139 ± 0.300$^{**\&\&}$** | **94.2** |

Note: Compared to the normal control group, $^{++}P \leq 0.01$; compared to the model control group, $^{*}P \leq 0.05$, $^{**}P \leq 0.01$; compared to the cisplatin group, $^{\#}P \leq 0.05$, $^{\#\#}P \leq 0.01$; compared to the Kanglaite soft capsule group, $^{\&}P \leq 0.05$, $^{\&\&}P \leq 0.01$; compared to the cisplatin + Kanglaite soft capsule group, $^{\star}P \leq 0.05$, $^{**}P \leq 0.01$.

In Figure 22: 1: Model control group; 2: Cisplatin group; 3: Kanglaite soft capsule group; 4: Cisplatin + Kanglaite soft capsule group; 7: Cisplatin + GLP-4 low-dose group, 8: Cisplatin + GLP-4 high-dose group

**Effect of Ganoderma Lucidum Polysaccharide GLP-4 Combined with Chemotherapy on Blood Biochemical Indicators in LLC-Bearing Mice**

As shown in Table 8, compared to the normal control group, the blood WBC, ALT, AST, and BUN levels in the mice of the model control group significantly increased (*P* ≤ 0.05 or *P* ≤ 0.01), and the RBC level significantly decreased (P ≤ 0.05). Compared to the model control group, the blood WBC and AST levels in the mice of cisplatin + GLP-4 low-dose and high-dose groups significantly decreased *(P* ≤ 0.05 or *P* ≤ 0.01), and the WBC level in the cisplatin group and the cisplatin + Kanglaite soft capsule group both significantly decreased (*P* ≤ 0.05). Compared to the cisplatin group, the blood BUN level in the mice of the Kanglaite soft capsule group significantly increased (*P* ≤ 0.05), and the RBC level in the mice of the cisplatin + Kanglaite soft capsule group significantly decreased (*P* ≤ 0.05). Compared to the Kanglaite soft capsule group, the blood WBC level in the mice of the cisplatin + Kanglaite soft capsule group significantly decreased (*P* ≤ 0.05) There was no significant statistical difference between groups compared to the cisplatin + Kanglaite soft capsule group.

Table 8: Effect of Ganoderma lucidum polysaccharide GLP-4 combined with chemotherapy on blood biochemical indicators in LLC-bearing mice ( $\bar{x} \pm s$ )

| Group | WBC ($10^9$/L) | RBC ($10^{12}$/L) | ALT (U/L) | AST (U/L) | BUN (mmol/L) | CRE (umol/L) |
|---|---|---|---|---|---|---|
| Normal control group | 7.65 ±0.71 | 9.71 + 0.17 | 33.44 ±2.27 | 124.78 ± 26.67 | 12.02 ±2.33 | 14.69 ±0.67 |
| Model control group | **53.26 ± 27.69$^{++}$** | **5.66 ± 0.99$^{++}$** | **105.60 ± 39.48$^{++}$** | **577.00 ± 218.58$^{++}$** | **18.42 ± 5.69$^{+}$** | 17.62 ±3.57 |

(continued)

| Group | WBC ($10^9$/L) | RBC ($10^{12}$/L) | ALT (U/L) | AST (U/L) | BUN (mmol/L) | CRE (umol/L) |
|---|---|---|---|---|---|---|
| Cisplatin group | **11.09 ± 3.64*** | 6.01 ± 0.82 | 58.25 ± 10.63 | 393.25 ± 114.89 | 7.91 ±2.36 | 20.45 ±3.43 |
| Kanglaite soft capsule group | 38.41 ±12.59 | 4.60 ± 0.66 | 85.00 ±47.35 | 313.50 ± 87.43 | **20.60 ± 5.07#** | 16.05 ± 1.51 |
| Cisplatin + Kanglaite soft capsule group | **2.61 ± 0.64*&** | **3.94 ± 0.83#** | 45.33 ±10.40 | 296.00 ± 118.49 | 11.66 ±4.94 | 18.07 ±2.54 |
| Cisplatin + GLP-4 low-dose group | **5.26 ±2.12*** | 5.56 ± 1.34 | 43.67 ± 4.18 | 271.33 ± **50.40*** | 11.59 ±1.76 | 16.47 ± 0.94 |
| Cisplatin + GLP-4 high-dose group | **1.48 ± 0.51**** | 5.37 ± 3.00 | 48.00 ± 4.00 | **208.50** ± **30.50*** | 15.98 ± 0.93 | 17.40 ± 0.90 |

Note: Compared to the normal control group, $^+P$:S 0.05, $^{++}P \leq 0.01$; compared to the model control group, $^*P \leq 0.05$, $^{**}P \leq 0.01$; compared to the cisplatin group, $^{\#}P \leq 0.05$, $^{\#\#}P \leq 0.01$; compared to the Kanglaite soft capsule group, $^{\&}P \leq 0.05$; compared to the cisplatin + Kanglaite soft capsule group, $^*P \leq 0.05$.

**Effect of Ganoderma Lucidum Polysaccharide GLP-4 Combined with Chemotherapy on $CD3^+/CD4^+$ and $CD3^+/CD8^+$ Ratios in LLC-Bearing Mice**

As shown in Table 9, compared to the normal control group, the blood CD3+/CD8+ ratio in the mice of the model control group significantly increased ($P \leq 0.05$). Compared to the model control group, the blood CD3+/CD8+ ratio in the mice of the cisplatin + GLP-4 low-dose group significantly decreased ($P \leq 0.05$). Compared to the cisplatin group, the blood CD3+/CD4+ and CD3+/CD8+ ratios in the cisplatin + GLP-4 low-dose group significantly decreased ($P \leq 0.05$); the blood CD3+/CD8+ ratio in the Kanglaite soft capsule group significantly decreased ($P \leq 0.05$). Compared to the Kanglaite soft capsule group, the blood CD3+/CD8+ ratio in the cisplatin + GLP-4 low dose-group significantly decreased ($P \leq 0.05$), and the blood CD3+/CD8+ ratio in the cisplatin + Kanglaite soft capsule group significantly increased ($P \leq 0.05$). Compared to the cisplatin + Kanglaite soft capsule group, the blood **$CD3^+/CD4^+$** and **$CD3^+/CD8^+$** ratios in the cisplatin + GLP-4 low dose-group significantly decreased ($P \leq 0.05$), and there were no statistically significant differences among the other treatment groups.

Table 9: Effect of Ganoderma lucidum polysaccharide GLP-4 combined with chemotherapy on **$CD3^+/CD4^+$** and **$CD3^+/CD8^+$** Ratios in LLC-Bearing Mice ( $\bar{x} \pm s$ )

| Group | **$CD3^+/CD4^+$** | **$CD3^+/CD8^+$** |
|---|---|---|
| Normal control group | 0.062 ±0.008 | 0.283 ±0.066 |
| Model control group | 0.124 ±0.042 | **1.460 ± 0.706+** |
| Cisplatin group | 0.093 ± 0.025 | 1.571 ±0.406 |
| Kanglaite soft capsule group | 0.091 ± 0.005 | **0.561 ± 0.095#** |
| Cisplatin + Kanglaite soft capsule group | 0.174 ± 0.094 | **1.050 ± 0.172&** |
| Cisplatin + GLP-4 low-dose group | **0.029 ± 0.014*#&★** | **0.378 ± 0.201#★** |
| Cisplatin + GLP-4 high-dose group | 0.434 ±0.331 | 2.302 ±1.472 |

Note: Compared to the normal control group, $^+P \leq 0.05$; compared to the model control group, $^*P \leq 0.05$; compared to the cisplatin group, $^{\#}P \leq 0.05$; compared to the Kanglaite soft capsule group, $^{\&}P \leq 0.05$, $^{\&\&}P \leq 0.01$; compared to the cisplatin + Kanglaite soft capsule group, $^*P \leq 0.05$, $^{**}P \leq 0.01$.

**Effect of Ganoderma Lucidum Polysaccharide GLP-4 Combined with Chemotherapy on Bone Marrow Cells in LLC-Bearing Mice**

As shown in Table 10, compared to the normal control group, the model control group showed a significant increase in mononuclear and other cell systems in the bone marrow ($P \leq 0.05$), a trend toward an increase in the myeloid and erythroid system and the myeloid/erythroid ratio, and a decreasing trend in the red cell and lymphatic systems, though these changes were not statistically significant. Compared to the model control group, the cisplatin + Kanglaite soft

capsule group showed a significant increase in the red cell system ($P \leq 0.05$) and a significant decrease in the myeloid/erythroid ratio and mononuclear cell system in the bone marrow of mice ($P \leq 0.05$). The Kanglaite soft capsule group and the cisplatin + GLP-4 low-dose group showed a significant decrease in the mononuclear cell system in the bone marrow of mice ($P \leq 0.01$). The cisplatin + GLP-4 high-dose group showed a significant decrease in both the mononuclear cell and other cell systems in the bone marrow of mice ($P \leq 0.05$ or $P \leq 0.01$). Compared to the cisplatin group, the cisplatin + GLP-4 low-dose and high-dose groups showed a significant decrease in the mononuclear cell system in the bone marrow of mice ($P \leq 0.05$). Compared to the Kanglaite soft capsule group, the cisplatin + GLP-4 high-dose group showed a significant decrease in other cells in the bone marrow of mice ($P \leq 0.05$). Compared to the cisplatin + Kanglaite soft capsule group, the cisplatin + GLP-4 low-dose group showed a significant increase in the myeloid and erythroid system and myeloid/erythroid ratio in the bone marrow of mice ($P \leq 0.05$), with no statistical significance in other groups.

Table 10: Effect of Ganoderma lucidum polysaccharide GLP-4 combined with chemotherapy on bone marrow cells in LLC-bearing mice ( $\bar{x} \pm s$ )

| Group | Myeloid and erythroid system | Red cell system | Myeloid/erythroid ratio | Lymphatic system | Mononuclear cell system | Other cells |
|---|---|---|---|---|---|---|
| Normal control group | 52.00 ± 4.91 | 20.50 ± 5.63 | 2.90 ± 0.69 | 25.50 ± 9.00 | 1.50 ± 0.29 | 0.50 ± 0.00 |
| Model control group | 62.17 ± 12.71 | 15.00 ± 5.27 | 7.93 ± 5.44 | 17.50 ± 9.45 | **3.50 ±0.76$^{+}$** | 1.83 ± 0.33 |
| Cisplatin group | 56.13 ± 4.95 | 22.75 ±5.67 | 3.68 ± 1.68 | 15.75 ± 3.44 | 2.88 ± 0.55 | 2.50 ± 2.01 |
| Kanglaite soft capsule group | 5600 ± 7.54 | 30.63 ±7.77 | 2.38 ± 0.76 | **8.13 ± 1.11$^{\#}$** | **2.00 ± 0.46$^{*}$** | 3.25 ± 0.97 |
| Cisplatin + Kanglaite soft capsule group | 41.00 ± 8.58 | **38.50 ± 10.11$^{*}$** | 1.33 ± 0.50 | 15.83 ± 3.88 | **2.00 ± 0.76$^{*}$** | 2.67 ± 0.33 |
| Cisplatin + GLP-4 low-dose group | **63.50 ±9.41$^{*}$** | 23.33 ± 9.24 | **5.10 ± 3.23$^{*}$** | 10.50 ± 1.32 | **1.50 ±0.29$^{**\#}$** | 1.17 ± 0.60 |
| Cisplatin + GLP-4 high-dose group | 53.83 ±5.17 | 3083 ± 8.54 | 2.10 ± 0.69 | 13.33 ± 4.00 | **1.00 ± 0.00$^{**\#}$** | **1.00 ± 0.00$^{*\&}$** |

Note: Compared to the normal control group, $^{+}P \leq 0.05$; compared to the model control group, $^{*}P \leq 0.05$, $^{**}P \leq 0.01$; compared to the cisplatin group, $^{\#}P \leq 0.05$, $^{\#\#}P \leq 0.01$; compared to the Kanglaite soft capsule group, $^{\&}P \leq 0.05$, $^{\&\&}P \leq 0.01$; compared to the cisplatin + Kanglaite soft capsule group, $^{\bigstar}P \leq 0.05$, $^{**}P \leq 0.01$.

**Effect of Ganoderma Lucidum Polysaccharide GLP-4 Combined with Chemotherapy on Tumor, Spleen, Kidney, and Stomach Histopathology in LLC-Bearing Mice**

As shown in Figure 23, the model control group displayed dense tumor cells around the tumor, closely arranged with large cell volume, high nucleus-to-cytoplasm ratio, and frequent mitotic figures. In the cisplatin group, large areas of necrosis and hemorrhage were visible at the tumor center, with abundant tumor cells; in the cisplatin + Kanglaite soft capsule group and cisplatin + GLP-4 low-dose group, necrosis was visible at the tumor center with reduced peripheral tumor cell numbers; the cisplatin + GLP-4 high-dose group showed vacuolation in the tumor tissue.

As shown in Figures 24 and 25, kidney and stomach pathology in all groups showed no significant abnormalities.

As shown in Figure 26, in the spleen, significant hematopoiesis and diverse cell types were noted in the red marrow of the model control group. The cisplatin + Kanglaite soft capsule group showed no significant abnormalities in the spleen, while varying degrees of hematopoiesis and diverse cell types were observed in the spleen of the other groups.

In Figures 23-26: A: Normal group; B: Model control group; C: Cisplatin group; D: Kanglaite soft capsule group; E: Cisplatin + Kanglaite soft capsule group; H: Cisplatin + GLP-4 low-dose group, I: Cisplatin + GLP-4 high-dose group

Conclusion

Ganoderma Lucidum Polysaccharide GLP-4 combined with cisplatin significantly inhibits tumor growth in LLC-bearing mice and exhibits a significant synergistic effect.

**Experimental Study on the Antitumor Effect of Ganoderma Lucidum Polysaccharide GLP-4 Combined with Chemotherapy on H22-Bearing Mice and Study Data**

**Experimental Objective**

To study the effect of Ganoderma lucidum polysaccharide GLP-4 on hepatoma-bearing mice which are prepared by implanting H22 hepatoma tumor homogenate in the right axilla of C57 mice. This study aims to provide experimental evidence for clinical studies of GLP-4.

**Experimental Materials**

**Test Sample**

Ganoderma lucidum polysaccharide GLP-4, batch number: OMLJT202108(2-4), provided by Shenzhen Aolimei Oncology Medical Technology Co., Ltd.

**Positive Control**

Cisplatin, batch number: E21268081, product of Shanghai Aladdin Biochemical Technology Co., Ltd.

**Laboratory Animals**

70 SPF male C57 mice, weighing 16-18 g, supplied by Guangdong Medical Laboratory Animal Center. Laboratory animal production license number: SCXK (Yue) 2022-0002; laboratory animal quality certification number: 44007200111236.

**Main Reagents**

PBS buffer, prepared by Shenzhen Aolimei Oncology Medical Technology Co., Ltd.; fetal bovine serum, product of Zhejiang Tianhang Biotechnology Co., Ltd.; 1640 culture medium, product of Gibco; 0.25% trypsin, product of Gibco.

**Main Instruments**

Vernier caliper, product of Shanghai Tool Works Co., Ltd.; I-2000 scale, Dongguan Nancheng Changxie Electronic Products Factory; ophthalmic scissors and tweezers, products of Shanghai Jinzhong Medical Instrument Co., Ltd.; CCL-170B-8 $CO_2$ incubator, product of ESCO, Singapore; Luna-II cell counter, product of Nanjing Hengqiao Instrument Co., Ltd.; JRA-35S handheld homogenizer, product of Wuxi Jieruian Instrument Equipment Co., Ltd.

**Experimental Methods**

A homogenate suspension of H22 hepatoma solid tumors was injected subcutaneously under the axilla of 60 healthy male C57 mice to create a solid tumor model. Once all mouse tumors averaged a volume of about 180 $mm^3$, mice were randomized into groups based on tumor volume and were administered the respective drugs or drug solvents via oral gavage or intraperitoneal injection for 27 consecutive days. Longest and shortest diameters of tumors were measured every three days to calculate tumor volume, and mouse weights were recorded every three days. At the end of the experiment, tumors, spleens, and thymuses were harvested and weighed to calculate tumor, spleen, and thymus indices.

**Dosage Design**

Based on previous experimental results, Ganoderma lucidum polysaccharide GLP-4 was administered at a low dose of 50 mg/kg and a high dose of 150 mg/kg as shown in Table 11.

Rationale for cisplatin dosage design: Based on the clinical dosage of cisplatin, which should not exceed 100 $mg/m^2$ per person per day, and considering the tolerance of mice to cisplatin, a dose of 3 mg/kg has been selected as the administration dosage.

Table 11: Experimental groups and dosage design

| Group | Dose (mg/kg) | Method of administration | Administration volume (mL/10 g) | Frequency of administration |
|---|---|---|---|---|
| Model control group | - | Oral gavage | 0.2 | Once daily |
| Cisplatin | 3 | Intraperitoneal injection | 0.2 | Once every 3 days |
| Cisplatin + GLP-4 low-dose group | 3+50 | Intraperitoneal injection + oral gavage | 0.2+0.2 | Cisplatin, once every 3 days; GLP-4, once daily |
| Cisplatin + GLP-4 high-dose group | 3+150 injection + oral gavage | Intraperitoneal | 0.2+0.2 days; GLP-4, once daily | Cisplatin, once every 3 |
| Normal group- | Oral gavage | 0.2 | Once daily | |

**Test Indicators**

**Efficacy Indicators**

**Relative tumor growth inhibition rate**

Relative tumor growth inhibition rate (%) = (1 - $T_{RTV}$ / $C_{RTV}$) × 100%. Where $T_{RTV}$ is the relative tumor volume in the experimental group, and $C_{RTV}$ is the relative tumor volume in the model control group. Relative tumor volume (RTV) = $V_t$ / $V_0$, where $V_t$ is the tumor volume on day t of dosing, and $V_0$ is the tumor volume at the time of grouping. Evaluation criteria: A relative tumor growth inhibition rate of ≥ 40% and a statistical analysis with P < 0.05 indicate effective inhibition.

**Tumor growth inhibition rate**

Tumor growth inhibition rate (%) = (1 - T / C) × 100%. Where T represents the average tumor weight in the treatment group, and C represents the average tumor weight in the model control group. Evaluation criteria: A tumor growth inhibition rate of ≥ 40% and a statistical analysis with P < 0.05 indicate effective inhibition.

**Spleen and thymus organ coefficients:** After the last dose, spleen, thymus, and tumor weights are measured, and organ coefficients are calculated.

$$\text{Tumor index (\%)} = (\text{tumor weight / body weight}) \times 100\%.$$

$$\text{Immune organ index (mg/g)} = (\text{organ mass / body weight}) \times 1000.$$

**Data Processing and Statistical Analysis**

Statistical analyses were performed using SPSS 17.0, with the significance level set at P ≤ 0.05. Measurement data were expressed as mean ±standard deviation ( $\bar{x} \pm s$ ). Normality and homogeneity of variance were tested using Leven's test. If data met normality and homogeneity of variance (P > 0.05), one-way ANOVA and LSD test were used for statistical analysis. If data did not meet normality and homogeneity of variance (P < 0.05), the Kruskal-Wallis test was used. If the Kruskal-Wallis test was statistically significant (P < 0.05), comparison analysis was performed using Dunnett's Test (a non-parametric method). Evaluation considered statistical differences and biological significance.

**Experimental Results**

**Animal Mortality**

As shown in Table 12, the mortality rate for all groups of mice was 0.

Table 12: Statistics on the number of surviving animals and mortality rate in each group

| Group | Total number of animals | Number of deaths | Mortality rate (%) | Survival time (days) |
|---|---|---|---|---|
| Model control group | 8 | 0 | 0 | 27±0 |
| Cisplatin | 8 | 0 | 0 | 27±0 |
| Cisplatin + GLP-4 low-dose group | 8 | 0 | 0 | 27±0 |
| Cisplatin + GLP-4 high-dose group | 8 | 0 | 0 | 27±0 |
| Normal group | 8 | 0 | 0 | 27±0 |

**Effect of Ganoderma Lucidum Polysaccharide GLP-4 Combined with Chemotherapy on Body Weight of H22-Bearing Mice**

As indicated in Table 13, compared to the normal group, the body weight of mice in the model control group significantly increased from D0 to D27; the body weight of mice in the cisplatin group significantly increased on D0 and D3 and significantly decreased from D15 to D27; the body weight of mice in the cisplatin + GLP-4 low-dose group significantly decreased from D12 to D27; and the body weight of mice in the cisplatin + GLP-4 high-dose group significantly decreased from D9 to D27.

Compared to the model control group, the body weight of mice in the cisplatin group significantly decreased from D6 to D27, and the body weight of mice in the cisplatin + GLP-4 low-dose and high-dose groups significantly decreased from D3 to D27.

Compared to the cisplatin group, the body weight of mice in the cisplatin + GLP-4 high-dose group significantly decreased on D15 and D24.

Table 13: Effect of Ganoderma lucidum polysaccharide GLP-4 combined with chemotherapy on body weight of H22-bearing mice ( x ± s)

| Group | Weight (g) | | | | | | | | | |
|---|---|---|---|---|---|---|---|---|---|---|
| | D0 | D3 | D6 | D9 | D12 | D15 | D18 | D21 | D24 | D27 |
| Model control group | **23±1.6$^{*}$** | **24.1±1.5$^{*}$** | **24.7±16$^{*}$** | **24.4±1.9$^{*}$** | **25.8±2.1$^{*}$** | **26.5±2.3$^{*}$** | **27.3±2.4$^{*}$** | **28.3±2.9$^{*}$** | **29.5±3.4$^{*}$** | **18.6±3.5$^{*}$** |
| Cisplatin | **22.8±1$^{*}$** | **23.4±0.7$^{*}$** | **22.7±0.7$^{+}$** | **22.2±0.7$^{+}$** | **21.9±0.5$^{+}$** | **213±1$^{*+}$** | **20.3±0.8$^{*+}$** | **19.5±0.9$^{*+}$** | **19.1±1$^{*+}$** | **18±1$^{*+}$** |
| Cisplatin + GLP-4 low-dose group | **22.8±0.9$^{*}$** | **22.6±0.9$^{+}$** | **22.1±1.1$^{+}$** | **21.7±1.2$^{+}$** | **21.2±0.9$^{*+}$** | **20.2±1.3$^{*+}$** | **18.7±2$^{*+}$** | **19.8±1.9$^{*+}$** | **19.2±2$^{*+}$** | **18.3±2$^{*+}$** |
| Cisplatin + GLP-4 high-dose group | **22.2±0.7$^{*}$** | **22.5±1$^{+}$** | **22.2±1.3$^{+}$** | **21.1±1$^{*+}$** | **20.7±0.5$^{*+}$** | **19±1.6$^{*+\#}$** | **19.7±1.6$^{*+}$** | **19.9±1.5$^{*+}$** | **19.1+1.7$^{*+\#}$** | **18±1.4$^{*+}$** |
| Normal group | **21.1±0.5$^{+}$** | **22.1±0.8$^{+}$** | **22.4±0.8$^{+}$** | **22.6±0.8$^{+}$** | **233±1$^{+}$** | **23.6±1.1$^{+}$** | **23.7±1$^{+}$** | **24.1±1.1$^{+}$** | **24.4±1.2$^{+}$** | **23.4±1.1$^{+}$** |

Note: Compared to the model control group, $^{+}P < 0.05$; Compared to the cisplatin group, $^{\#}P < 0.05$; Compared to the normal group, $^{*}P < 0.05$.

**Effect of Ganoderma Lucidum Polysaccharide GLP-4 Combined with Chemotherapy on Tumor Volume in H22-Bearing Mice**

As shown in Table 14, compared to the model control group, the tumor volume in the cisplatin group was significantly reduced from D9 to D27, the tumor volume in the cisplatin + GLP-4 low-dose group was significantly reduced from D6 to D27, and the tumor volume in the cisplatin + GLP-4 high-dose group was significantly reduced from D9 to D27.

Compared to the cisplatin group, the tumor volume in the cisplatin + GLP-4 low-dose group was significantly reduced from D12 to D18 and on D27.

Table 14: Effect of Ganoderma lucidum polysaccharide GLP-4 combined with chemotherapy on tumor volume in H22-bearing mice ( x ± s)

| Group | Tumor volume ($mm^3$) | | | | | | | | | |
|---|---|---|---|---|---|---|---|---|---|---|
| | D0 | D3 | D6 | D9 | D12 | D15 | D18 | D21 | D24 | D27 |
| Model control group | 166.2±47.6 | 195.2±112.6 | 399.7±267.4 | 610.1±435.2 | 900.5±667 | 1206.1±785.9 | 2108.7±1786.9 | 2740.4±1926.4 | 3539.2±2376.5 | 4810.1±3267.8 |
| Cisplatin | 179.2±72 | 169.3±123.3 | **269.3±142.2** | **248.3±140.1$^{+}$** | **317.3±173.8$^{+}$** | **427.3±199.7$^{+}$** | **471.1±2253$^{+}$** | **502.1±263.8$^{+}$** | **6115±389.5$^{+}$** | **887.7±638.2$^{+}$** |
| Cisplatin + GLP-4 low-dose group | 176.3±51.2 | 117.8±79.7 | **197±89.7$^{+}$** | **171.6±127.8$^{+}$** | **179.7±122.7$^{\#+}$** | **179.3±133$^{*+}$** | **224.9±174$^{*+}$** | **299.8±253.5$^{+}$** | **394.5±355.4$^{+}$** | **357.4±349.4$^{*+}$** |
| Cisplatin + GLP-4 high-dose group | 179.9±71.5 | 159.5±84.8 | 312±251.4 | **260.9±205.8$^{+}$** | **280±253.4$^{+}$** | **293.8±306.7$^{+}$** | **399.7±315.5$^{+}$** | **484.6±405.1$^{+}$** | **532.8±541.9$^{+}$** | **586.2±566.9$^{+}$** |

Note: Compared to the model control group, $^{+}P < 0.05$; Compared to the cisplatin group, $^{\#}P < 0.05$.

As indicated in Tables 15 and 16, compared to the model control group, the relative tumor growth inhibition rate in the cisplatin group from D9 to D27 was over 60%, specifically 66.5%, 70.6%, 69.1%, 81.3%, 83.9%, 85.3%, and 84.3% ($P < 0.05$); the relative tumor growth inhibition rate in the cisplatin + GLP-4 low-dose group from D6 to D27 was over 55%, specifically 56.1%, 77.1%, 83.6%, 87.9%, 91.7%, 91.2%, 91.1%, and 94.2% ($P < 0.05$); the relative tumor growth inhibition rate in the cisplatin + GLP-4 high-dose group from D9 to D27 was over 60%, specifically 65.2%, 76.6%, 81.9%, 85.8%, 86.4%, 88.7%, and 90.9% ($P < 0.05$).

Compared to the cisplatin group, the relative tumor growth inhibition rate in the cisplatin + GLP-4 low-dose group from D12 to D18 and on D27 was 44.4%, 60.9%, 55.6%, and 63.3% ($P < 0.05$), and the relative tumor growth inhibition rate in the cisplatin + GLP-4 high-dose group on D15 and D27 was 41.6% and 42.3%.

Table 15: Effect of Ganoderma lucidum polysaccharide GLP-4 combined with chemotherapy on relative tumor growth inhibition rate in H22-bearing mice (vs. the model control group)

| Group | Relative tumor growth inhibition rate (compared to model control group) (%) | | | | | | | | |
|---|---|---|---|---|---|---|---|---|---|
| | D3 | D6 | D9 | D12 | D15 | D18 | D21 | D24 | D27 |
| Model control group | - | - | - | - | - | - | - | - | - |
| Cisplatin | 32.9 | 39.7 | **66.5**$^+$ | **70.6**$^+$ | **69.1**$^+$ | **81.3**$^+$ | **83.9**$^+$ | **85.3**$^+$ | **84.3**$^+$ |
| Cisplatin + GLP-4 low-dose group | 47.4 | **56.1**$^+$ | **77.1**$^+$ | **83.6**$^+$ | **87.9**$^+$ | **91.7**$^+$ | **91.2**$^+$ | **91.1**$^+$ | **94.2**$^+$ |
| Cisplatin + GLP-4 high-dose group | 26.5 | 35.7 | **65.2**$^+$ | **76.6**$^+$ | **81.9**$^+$ | **85.8**$^+$ | **86.4**$^+$ | **88.7**$^+$ | **90.9**$^+$ |

Note: Compared to the model control group, $^+P < 0.05$.

Table 16: Effect of Ganoderma lucidum polysaccharide GLP-4 combined with chemotherapy on relative tumor growth inhibition rate in H22-bearing mice (vs. the cisplatin group)

| Group | Relative tumor growth inhibition rate (compared to model control group) (%) | | | | | | | | |
|---|---|---|---|---|---|---|---|---|---|
| | D3 | D6 | D9 | D12 | D15 | D18 | D21 | D24 | D27 |
| Cisplatin | - | - | - | - | - | - | - | - | - |
| Cisplatin + GLP-4 low-dose group | 21.7 | 27.2 | 31.7 | **44.4**$^\#$ | **60.9**$^\#$ | **55.6**$^\#$ | 45.3 | 39.5 | **63.3**$^\#$ |
| Cisplatin + GLP-4 high-dose group | -9.4 | -6.6 | -3.6 | 20.4 | 41.6 | 24.4 | 15.4 | 23.1 | 42.3 |

Note: Compared to the cisplatin group, $^\#P < 0.05$.

As indicated in Table 17, compared to the model group, the tumor index, spleen index, and thymus index were significantly reduced in the cisplatin group, the cisplatin + GLP-4 low-dose group, and the cisplatin + GLP-4 high-dose group. Compared to the cisplatin group, the thymus index was significantly lower in the cisplatin + GLP-4 low-dose group. Compared to the normal group, the spleen index was significantly reduced in the model control group; the thymus index was significantly reduced in the cisplatin group, the cisplatin + GLP-4 low-dose group, and the cisplatin + GLP-4 high-dose group.

Compared to the model control group, the tumor growth inhibition rate was 88.7% in the cisplatin group, and 91.5% and 88.2% in the cisplatin + GLP-4 low-dose and high-dose groups, respectively. Compared to the cisplatin group, the tumor growth inhibition rate was 25.1% and -4.4% in the cisplatin + GLP-4 low-dose and high-dose groups, respectively.

Table 17: Effect of Ganoderma lucidum polysaccharide GLP-4 combined with chemotherapy on organ indices and tumor growth inhibition rates in H22-bearing mice

| Group | Tumor index (%) | Spleen index (mg/g) | Thymus index (mg/g) | Tumor growth inhibition rate (%) vs. the model control group | Tumor growth inhibition rate (%) vs. the cisplatin group |
|---|---|---|---|---|---|
| Model control group | 14.9±10.4 | **9.4±4.9**$^*$ | 1.3±0.4 | - | - |
| Cisplatin | **2.8±2.1**$^+$ | **2.5±0.1**$^+$ | **0.3±0.1**$^{*+}$ | 88.7 | - |
| Cisplatin + GLP-4 low-dose group | **1.9±2**$^+$ | **2.4±0.2**$^+$ | **0.7±0.3**$^{*+}$ | 91.5 | 25.1 |

(continued)

| Group | Tumor index (%) | Spleen index (mg/g) | Thymus index (mg/g) | Tumor growth inhibition rate (%) vs. the model control group | Tumor growth inhibition rate (%) vs. the cisplatin group |
|---|---|---|---|---|---|
| Cisplatin + GLP-4 high-dose group | **2.9±3.5⁺** | **2.5±0.2⁺** | **0.4±0.2*⁺** | 88.2 | -4.4 |
| Normal group | - | **2.8±0.2⁺** | 1.6±0.2 | - | - |
| Note: Compared to the model control group, ⁺P < 0.05; Compared to the cisplatin group, #P < 0.05; Compared to the normal group, *P < 0.05. | | | | | |

Figures 27-30 show images of the tumor-bearing mice, corresponding to the groups as follows: Figure 27: model control group, Figure 28: cisplatin group, Figure 29: cisplatin + GLP-4 low-dose group, Figure 30: cisplatin + GLP-4 high-dose group.

Figures 31-34 show images of the tumors in the tumor-bearing mice, corresponding to the groups as follows: Figure 31: model control group, Figure 32: cisplatin group, Figure 33: cisplatin + GLP-4 low-dose group, Figure 34: cisplatin + GLP-4 high-dose group.

Conclusion

Ganoderma Lucidum Polysaccharide GLP-4 combined with cisplatin significantly inhibits tumor growth in H22-bearing mice and exhibits a significant synergistic effect.

**Experimental Study on the Antitumor Effect of Ganoderma Lucidum Polysaccharide GLP-4 Combined with Chemotherapy on Orthotopic H22-Bearing Mice and Study Data**

**Experimental Objective**

To study the effect of Ganoderma lucidum polysaccharide GLP-4 combined with chemotherapy on orthotopic H22-bearing mice which are prepared by implanting H22 hepatoma tumors in the right axilla of C57 mice. This study aims to provide experimental evidence for clinical studies of GLP-4.

**Experimental Materials**

**Test Sample**

Ganoderma lucidum polysaccharide GLP-4, batch number: OLMJT202108(2-4), provided by Shenzhen Aolimei Oncology Medical Technology Co., Ltd.

**Control**

Kanglaite soft capsules, batch number: 20211006, Zhejiang Kanglaite Pharmaceutical Co., Ltd.; cisplatin injection, batch number: 601211204, Jiangsu Hansoh Pharmaceutical Co., Ltd.

**Laboratory Animals**

65 SPF-grade male C57 mice, weighing 12-15 g, provided by Hunan SJA Laboratory Animal Co., Ltd. Laboratory animal production license number: SCXK (Xiang) 2019-0004. The animals were housed in Barrier Environment Laboratory D of Hunan Puruima Pharmaceutical Research Center Co., Ltd., under the laboratory animal use license number: SYXK (Xiang) 2020-0015.

**Main Reagents**

0.9% sodium chloride injection, batch number: 21071401C, Hunan Kangyuan Pharmaceutical Co., Ltd.; ALT assay kit, batch number: 201751; AST assay kit, batch number: 110620; CRE assay kit, batch number: 111644; BUN assay kit, batch number: 201749, all manufactured by Wako Pure Chemical Industries, Ltd. of Japan.

**Main Instruments**

AR223CN Electronic Scale, Ohaus Instruments (Changzhou) Co., Ltd.; LABOSPECT003 Automatic Biochemical Analyzer, Hitachi, Japan; AniView100 Multimodal Animal In Vivo Imaging System, ANDOR; TDZ5-WS Benchtop Multi-Tube Automatic Balance Centrifuge, Hunan Kaida Industrial Development Co., Ltd.; ME2002E Electronic Scale, Shimadzu Corporation, Japan; Flow Cytometer, BD Biosciences; ASP200S Fully Automatic Tissue Dehydrator, ASP300S Fully Automatic Tissue Dehydrator, TP1020 Fully Automatic Dehydrator, HI1210 Slide Spreader, HI1220 Slide Dryer, RM2235 Paraffin Microtome, EG1150H+C Tissue Embedding Station, AutoStainer XL Automatic Slide Stainer + CV5030 Automatic Cover Slipper, BX43 Biological Microscope + MD50 Digital Imaging System, CX31 Biological Microscope, all from Leica, Germany.

**Experimental Methods**

Liver cancer (H22) cells, labeled with Luc fluorescent marker at a concentration of $1\times10^7$ cells/mL, were initially inoculated into the peritoneal cavity of 8 male C57 mice. After the development of ascites, the ascitic fluid was aseptically extracted, washed with HBSS buffer, centrifuged to discard the supernatant, and stained with Trypan Blue, and cells in the ascitic fluid were counted under a microscope. The cell concentration was adjusted to $1\times10^{13}$/mL with HBSS buffer and inoculated into the right hepatic region of 51 male C57 mice, at a volume of 10 μL per mouse, to prepare the orthotopic tumor-bearing mice. One week later, the liver tumor formation in mice was detected using a small animal in vivo imaging system. Based on tumor size, the mice were randomized into groups: model control group, cisplatin group (4 mg/kg), Kanglaite soft capsule group (1,404 mg/kg), cisplatin + Kanglaite soft capsule group (4 + 1,404 mg/kg), cisplatin + GLP-4 low-dose group (4 + 130 mg/kg), and cisplatin + GLP-4 high-dose group (4 + 1,170 mg/kg), with 6 mice per group. An additional 6 mice served as the normal control group. The normal control group and the model control group were administered pure water by oral gavage. The chemotherapy group received intraperitoneal injections of cisplatin, and the other groups were administered their respective drug solutions by oral gavage (20 mL/kg) or intraperitoneal injection (10 mL/kg), once daily for 14 consecutive days. After the last dose, blood was collected from the orbital plexus to test blood WBC, RBC, liver and kidney function indicators (ALT, AST, BUN, CRE), and $CD3^+/CD4^+$, $CD3^+/CD8^+$ ratios. The spleen, thymus, and tumors were weighed to calculate organ coefficients.

Dosage Design

Based on previous experimental results, Ganoderma lucidum polysaccharide GLP-4 was administered at a low dose of 130 mg/kg and a high dose of 1170 mg/kg as shown in Table 18.

The proposed clinical dose for Kanglaite soft capsules is 0.45 g per capsule, 6 capsules per dose, 4 doses per day, which totals 10.8 g per day. When converted to an equivalent mouse dose based on body surface area, it is calculated as 10.8 g/day × 0.0026 / 0.02 kg = 1,404 mg/kg. This study used the proposed clinical dose as a basis for the experiment.

Rationale for cisplatin dosage design: Based on the clinical dosage of cisplatin, which should not exceed 100 $mg/m^2$ per person per day, and considering the tolerance of mice to cisplatin, a dose of 4 mg/kg has been selected as the administration dosage.

Table 18: Experimental groups and dosage design

| Group | Dose (mg/kg) | Method of administration | Frequency of administration |
|---|---|---|---|
| Normal control group | - | Oral gavage | Once daily |
| Model control group | - | Oral gavage | Once daily |
| Cisplatin group | 4 | Intraperitoneal injection | Once every 3 days |
| Kanglaite soft capsule group | 1404 | Oral gavage | Once daily |
| Cisplatin + Kanglaite soft capsule group | 4+1404 | Intraperitoneal injection + oral gavage | Cisplatin, once every 3 days; Kanglaite soft capsule, once daily |
| Cisplatin + GLP-4 low-dose group | 4+130 | Intraperitoneal injection + oral gavage | Cisplatin, once every 3 days; GLP-4, once daily |
| Cisplatin + GLP-4 high-dose group | 4+1170 | Intraperitoneal injection + oral gavage | Cisplatin, once every 3 days; GLP-4, once daily |

**Test Indicators**

**Efficacy Indicators**

**Animal survival and general condition:** Weight was recorded weekly, along with any deaths among the animals.

**Tumor volume measurement:** Tumor volume changes were measured weekly using small animal in vivo imaging technology.

**Hematological tests:** After the last dose, routine blood tests (WBC and RBC) and biochemical tests (liver and kidney functions) were performed.

**Immune organs:** The thymus, spleen, tumor, and liver were weighed, and organ coefficients were calculated as follows: organ coefficient (%) = organ weight / fasting body weight $\times$ 100%

**$CD4^+$ and $CD8^+$ content measurement:** After the last dose, lymphocyte subtypes $CD3^+/CD4^+$ and $CD3^+/CD8^+$ in the blood were measured using flow cytometry.

**Data Processing and Statistical Analysis**

Significant figures of the study data were rounded according to the nearest whole number. Statistical analysis was conducted in accordance with the center's *SOP,* using SPSS software. Measurement data are expressed as mean $\pm$ standard deviation ( $\bar{x} \pm s$), and normality and homogeneity of variance were tested using Leven's test. If not statistically significant ($P > 0.05$), one-way ANOVA was used for statistical analysis. If ANOVA was statistically significant ($P \leq 0.05$), the LSD test (parametric method) was used for comparison analysis. If the variance was not homogeneous ($P \leq 0.05$), the Kruskal-Wallis test was employed. If the Kruskal-Wallis test was statistically significant ($P \leq 0.05$), the Dunnett's Test (non-parametric method) was used for comparison analysis. Statistical significance was determined at $\alpha = 0.05$, where $P \leq 0.05$ indicates statistical significance and $P \leq 0.01$ indicates highly significant differences.

**Experimental Results**

**General Clinical Observation and Mortality in Animals**

As shown in Table 19, before administration, the mice exhibited normal activity, movement, and gait; after administration, a reduction in activity was observed, and tumor growth impacted their food and water intake, leading to minimal weight gain. In the model control group, 2M01 died on D12; in the cisplatin group, 3M01/3M05 and 3M02 died on D9 and D10, respectively; in the Kanglaite soft capsule group, 4M01 died on D9; in the cisplatin + Kanglaite soft capsule group, 5M01 and 5M06 died on D8 and D11, respectively; in the cisplatin + GLP-4 low-dose group, 8M02 died on D8.

The mortality rates for each group were 0%, 16.7%, 50.0%, 16.7%, 16.7%, 16.7%, and 0%, respectively.

Table 19: Statistics on the number of surviving animals and mortality rate in each group

| Group | Dose (mg/kg) | Number of animals | Number of deaths | Number of surviving animals | Mortality rate (%) |
|---|---|---|---|---|---|
| Normal control group | - | 6 | 0 | 6 | 0 |
| Model control group | - | 6 | 1 | 5 | 16.7 |
| Cisplatin group | 4 | 6 | 3 | 3 | 50.0 |
| Kanglaite soft capsule group | 1404 | 6 | 1 | 5 | 16.7 |
| Cisplatin + Kanglaite soft capsule group | 4+1404 | 6 | 1 | 5 | 16.7 |
| Cisplatin + GLP-4 low-dose group | 4+130 | 6 | 1 | 5 | 16.7 |
| Cisplatin + GLP-4 high-dose group | 4+1170 | 6 | 0 | 6 | 0 |

**Effect of Ganoderma Lucidum Polysaccharide GLP-4 Combined with Chemotherapy on Body Weight of Orthotopic H22-Bearing Mice**

As shown in Table 20, compared to the normal control group, the body weight of mice in the model control group significantly decreased on W2 after administration ($P \leq 0.01$). Compared to the model control group, the body weight of mice in both the cisplatin + GLP-4 low-dose group and the cisplatin + GLP-4 high-dose group significantly decreased on W1 and W2 after administration ($P \leq 0.05$ or $P \leq 0.01$), and the body weight of mice in the cisplatin group and the cisplatin + Kanglaite soft capsule group significantly decreased on W1 and W2 after administration ($P \leq 0.05$ or $P \leq 0.01$). Compared to the cisplatin group, the body weight of mice in the Kanglaite soft capsule group significantly increased on W2 after

administration ($P \leq 0.05$ or $P \leq 0.01$). Compared to the Kanglaite soft capsule group, the body weight of mice in the cisplatin + GLP-4 low-dose and high-dose groups significantly decreased on W1 and W2 after administration ($P \leq 0.05$ or $P \leq 0.01$). Compared to the cisplatin + Kanglaite soft capsule group, the body weight of mice in the cisplatin + GLP-4 high-dose group significantly decreased on W2 after administration ($P \leq 0.05$ or $P \leq 0.01$).

Table 20: Effect of Ganoderma lucidum polysaccharide GLP-4 combined with chemotherapy on body weight of orthotopic H22-bearing mice ( $\bar{x} \pm s$ )

| Group | Weight (g) | | |
|---|---|---|---|
| | WO | W1 | W2 |
| Normal control group | 19.9 ± 1.2 | 18.9 ± 1.6 | 22.4 ± 1.7 |
| Model control group | 18.3 ± 1.9 | 19.1 ± 1.9 | **19.6 ± 1.8$^{++}$** |
| Cisplatin group | 18.5 ± 1.3 | **16.9 ± 1.8$^{*}$** | **15.1 ± 1.6$^{**}$** |
| Kanglaite soft capsule group | 18.2 ± 1.5 | 18.7 ± 2.9 | **19.0 ± 1.3$^{\#\#}$** |
| Cisplatin + Kanglaite soft capsule group | 18.6 ± 0.9 | **15.5 ± 2.3$^{**\&\&}$** | **16.6 ± 3.7$^{**\&}$** |
| Cisplatin + GLP-4 low-dose group | 18.4 ± 3.4 | **16.0 ± 1.9$^{**\&\&}$** | **16.0 ± 1.8$^{**\&\&}$** |
| Cisplatin + GLP-4 high-dose group | 18.7 ± 1.1 | **15.3 ± 0.8$^{**\&\&}$** | **14.3 ± 0.8$^{**\&\&\star}$** |

Note: Compared to the normal control group, $^{++}P \leq 0.01$; compared to the model control group, $^{*}P \leq 0.05$, $^{**}P \leq 0.01$; compared to the cisplatin group, $^{\#}P \leq 0.05$, $^{\#\#}P \leq 0.01$; compared to the Kanglaite soft capsule group, $^{\&}P \leq 0.05$, $^{\&\&}P \leq 0.01$; compared to the cisplatin + Kanglaite soft capsule group, $^{\star}P \leq 0.05$, $^{\star\star}P \leq 0.01$.

**Effect of Ganoderma Lucidum Polysaccharide GLP-4 Combined with Chemotherapy on Tumors in Orthotopic H22-Bearing Mice**

As shown in Figures 35, 36, and Table 21, compared to the normal control group, the tumors in the mice of the model control group significantly increased ($P \leq 0.01$); compared to the model control group, the tumors in the mice of the Kanglaite soft capsule group significantly decreased on W1 and W2 after administration ($P \leq 0.05$ or $P \leq 0.01$); the tumors in the mice of the cisplatin + GLP-4 low-dose and high-dose groups, the cisplatin group, and the cisplatin + Kanglaite soft capsule group significantly decreased on W2 after administration ($P \leq 0.05$ or $P \leq 0.01$). There were no significant differences across the treatment groups when compared to the cisplatin group. Compared to the Kanglaite soft capsule group, the tumors in the mice of the cisplatin + GLP-4 high-dose group and Kanglaite soft capsule group significantly increased on W1 after administration ($P \leq 0.05$ or $P \leq 0.01$). There was no statistical difference between groups compared to the cisplatin + Kanglaite soft capsule group.

As shown in Figure 35, the corresponding groups are as follows: A: normal group, B: model control group, C: cisplatin group, D: Kanglaite soft capsule group, E: cisplatin + Kanglaite soft capsule group, H: cisplatin + GLP-4 low-dose group, I: cisplatin + GLP-4 high-dose group.

As shown in Figure 36, the corresponding groups are as follows: 1: normal group, 2: model control group, 3: cisplatin group, 4: Kanglaite soft capsule group, 5: cisplatin + Kanglaite soft capsule group, 8: cisplatin + GLP-4 low-dose group, 9: cisplatin + GLP-4 high-dose group.

Table 21: Effect of Ganoderma lucidum polysaccharide GLP-4 combined with chemotherapy on tumors in orthotopic H22-bearing mice ($\bar{x} \pm s$)

| Group | WO | | W1 | | W2 | |
|---|---|---|---|---|---|---|
| | Tumor (P/s/cm$^2$/sr) | Number of animals | Tumor (P/s/cm$^2$/sr) | Number of animals | Tumor (P/s/cm$^2$/sr) | Number of animals |
| Normal control group | 0 ± 0 | 6 | 0 ± 0 | 6 | 0 ± 0 | 6 |
| Model control group | **3897500 ± 2651893$^{++}$** | 6 | **17515000 ± 2702626$^{++}$** | 6 | **20012000 ± 3407219$^{++}$** | 5 |
| Cisplatin group | 3756000 ± 2439052 | 6 | 12898500 ± 3588676 | 6 | **11871333 ± 2780016$^{*}$** | 3 |
| Kanglaite soft capsule group | 3888167 ± 2341708 | 6 | **5906333 ± 2710545$^{**}$** | 6 | **6040000 ± 2159132$^{**}$** | 5 |
| Cisplatin + Kanglaite soft capsule group | 3968117 ± 2511453 | 6 | **17516667 ± 3438257$^{\&\&}$** | 6 | **10266000 ± 2143720$^{**}$** | 5 |

(continued)

| Group | WO | | W1 | | W2 | |
|---|---|---|---|---|---|---|
| | Tumor (P/s/cm2/sr) | Number of animals | Tumor (P/s/cm2/sr) | Number of animals | Tumor (P/s/cm2/sr) | Number of animals |
| Cisplatin + GLP-4 low-dose group | 3770333 ± 2142218 | 6 | 11598333 ± 2267784 | 6 | **7400000 ± 1891380**** | 5 |
| Cisplatin + GLP-4 high-dose group | 3802667 ± 1879800 | 6 | **15061667 ± 1430176&** | 6 | **4803333 ± 1268118**** | 6 |

Note: Compared to the normal control group, ++*P* ≤ 0.01; compared to the model control group, **P* ≤ 0.05, ***P* ≤ 0.01; compared to the cisplatin group, #*P* ≤ 0.05, ##*P* ≤ 0.01; compared to the Kanglaite soft capsule group, &*P* ≤ 0.05, &&*P* ≤ 0.01; compared to the cisplatin + Kanglaite soft capsule group, ★*P* ≤ 0.05, ★★*P* ≤ 0.01.

**Effect of Ganoderma Lucidum Polysaccharide GLP-4 Combined with Chemotherapy on Organ Coefficients and Tumor Growth Inhibition Rates in Orthotopic H22-Bearing Mice**

As shown in Table 22, compared to the normal control group, the organ coefficients of the spleen and liver tissues in the model control group significantly increased (*P* ≤ 0.05 or *P* ≤ 0.01). Compared to the model control group, the thymus and spleen coefficients in both the cisplatin + GLP-4 low-dose and high-dose groups significantly decreased (*P* ≤ 0.05 or *P* ≤ 0.01), and the thymus and spleen coefficients in both the cisplatin group and the cisplatin + Kanglaite soft capsule group significantly decreased (*P* ≤ 0.05). There were no significant differences across the groups when compared to the cisplatin group. Compared to the Kanglaite soft capsule group, the thymus and spleen coefficients in the cisplatin + GLP-4 low-dose group significantly decreased (*P* ≤ 0.05 or *P* ≤ 0.01), and the spleen coefficients in the cisplatin + GLP-4 high-dose group and the Kanglaite soft capsule group also significantly decreased (P ≤ 0.05 or *P* ≤ 0.01).

Table 22: Effect of Ganoderma lucidum polysaccharide GLP-4 combined with chemotherapy on organ coefficients in orthotopic H22-bearing mice ()

| Group | Thymus coefficient | Spleen coefficient | Liver coefficient |
|---|---|---|---|
| Normal control group | 0.191 ± 0.039 | 0.372 ± 0.066 | 5.325 ± 0.205 |
| Model control group | 0.191 ± 0.162 | **1.151 ± 0.713++** | **12.497 ± 6.659+** |
| Cisplatin group | **0.078 ± 0.023*** | **0.524 ± 0.295*** | 11.365 ± 3.599 |
| Kanglaite soft capsule group | 0.144 ± 0.079 | 1.116 ± 0.537 | 14.206 ± 8.001 |
| Cisplatin + Kanglaite soft capsule group | **0.074 ± 0.063*** | **0.548 ± 0.351*&** | 9.405 ± 2.800 |
| Cisplatin + GLP-4 low-dose group | **0.047 ± 0.015**&** | **0.577 ± 0.220*&** | 10.849 ± 2.947 |
| Cisplatin + GLP-4 high-dose group | **0.072 ± 0.030**** | **0.513 ± 0.095**&&** | 15.137 ± 5.425 |

Note: Compared to the normal control group, +*P* ≤ 0.05, ++*P* ≤ 0.01; compared to the model control group, **P ≤ 0.05,* **P ≤ 0.01; compared to the cisplatin group, #*P* ≤ 0.05; compared to the Kanglaite soft capsule group, &*P* ≤ 0.05, &&*P* ≤ 0.01.

**Effect of Ganoderma Lucidum Polysaccharide GLP-4 Combined with Chemotherapy on Blood Biochemical Indicators in Orthotopic H22-Bearing Mice**

As shown in Table 23, compared to the normal control group, the blood AST and CRE levels in the mice of the model control group significantly increased (*P* ≤ 0.01). Compared to the model control group, the blood WBC level in the mice of the cisplatin + Kanglaite soft capsule group significantly decreased (*P* ≤ 0.05), the blood WBC and BUN levels in the mice of the cisplatin + GLP-4 low-dose group significantly decreased (*P* ≤ 0.05 or *P* ≤ 0.01), and the blood WBC and RBC levels in the mice of the cisplatin + GLP-4 high-dose group significantly decreased (*P* ≤ 0.05 or *P* ≤ 0.01). Compared to the cisplatin group, the blood BUN level in the mice of the cisplatin + GLP-4 low-dose group significantly decreased (*P* ≤ 0.05). Compared to the Kanglaite soft capsule group, the blood RBC level in the mice of the cisplatin + GLP-4 low-dose group significantly decreased (*P* ≤ 0.05 or *P* ≤ 0.01), and the blood CRE level in the mice of the cisplatin + GLP-4 high-dose group significantly increased (P ≤ 0.05). There were no statistically significant differences in other groups.

Table 23: Effect of Ganoderma lucidum polysaccharide GLP-4 combined with chemotherapy on blood biochemical indicators in orthotopic H22-bearing mice ($\bar{x} \pm s$)

| Group | WBC ($10^9$/L) | RBC ($10^{12}$/L) | ALT (U/L) | AST (U/L) | BUN (mmol/L) | CRE (umol/L) |
|---|---|---|---|---|---|---|
| Normal control group | 6.81 ± 0.83 | 9.62 ± 0.16 | 49 ± 4 | 134 ± 18 | 8.10 ± 1.33 | 10.5 ± 0.9 |
| Model control group | 9.33 ± 1.29 | 9.18 ± 0.44 | 84 ± 31 | **761 ± 104$^{++}$** | 9.75 ± 1.51 | **19.8 ± 2.7$^{+}$** |
| Cisplatin group | 4.41 ± 1.83 | 8.91 ± 0.22 | 115± 39 | 771 ± 153 | 13.52 ± 4.94 | 18.9 ± 2.7 |
| Kanglaite soft capsule group | 6.16 ± 1.60 | 8.80 ± 0.15 | 72 ± 10 | 831 ± 148 | 8.42 ± 1.86 | 15.1 ± 1.7 |
| Cisplatin + Kanglaite soft capsule group | **3.10 ± 0.51$^{*}$** | 8.54 ± 0.27 | 63 ± 21 | 431 ± 103 | 4.97 ± 2.08 | 22.2 ± 0.9 |
| Cisplatin + GLP-4 low-dose group | **2.50 ± 0.32$^{**}$** | **8.21 ± 0.16$^{\&}$** | 62 ± 11 | 740 ± 81 | **4.12 ± 0.82$^{*\#}$** | 17.6 ± 4.0 |
| Cisplatin + GLP-4 high-dose group | **4.08 ± 0.28$^{**}$** | **7.70 ± 0.57$^{*}$** | 69 ± 11 | 1129 ± 211 | 10.93 ± 2.51 | **25.5 ± 2.8$^{\&}$** |

Note: Compared to the normal control group, $^{+}P \leq 0.05$, $^{++}P \leq 0.01$; compared to the model control group, $^{*}P \leq 0.05$, $^{**}P \leq 0.01$; compared to the cisplatin group, $^{\#}P \leq 0.05$, $^{\#\#}P \leq 0.01$; compared to the Kanglaite soft capsule group, $^{\&}P \leq 0.05$, $^{\&\&}P \leq 0.01$; compared to the cisplatin + Kanglaite soft capsule group, $^{\star}P \leq 0.05$, $^{\star\star}P \leq 0.01$.

As shown in Table 24, compared to the normal control group, there was a trend of increased blood $CD3^+/CD4^+$ and $CD3^+/CD8^+$ ratios in the model control group of mice, though the differences were not statistically significant. Compared to the model control group, the blood $CD3^+/CD8^+$ ratio in the mice of the cisplatin + GLP-4 high-dose group significantly increased (P ≤ 0.05); the blood $CD3^+/CD4^+$ and $CD3^+/CD8^+$ ratios in the mice of the cisplatin + GLP-4 high-dose group significantly increased (P ≤ 0.05). Compared to the cisplatin group, the blood $CD3^+/CD4^+$ and $CD3^+/CD8^+$ ratios in the mice of the cisplatin + GLP-4 low-dose and high-dose groups significantly increased (P ≤ 0.05). Compared to the Kanglaite soft capsule group, the blood $CD3^+/CD4^+$ and $CD3^+/CD8^+$ ratios in the mice of the cisplatin + GLP-4 low-dose group significantly increased ($P \leq 0.05$), and the blood $CD3^+/CD4^+$ ratio in the mice of the cisplatin + GLP-4 high-dose group significantly increased (P ≤ 0.05). Compared to the cisplatin + Kanglaite soft capsule group, the blood $CD3^+/CD4^+$ ratio in the mice of the cisplatin + GLP-4 low-dose and high-dose groups significantly increased ($P \leq 0.05$).

Table 24: Effect of Ganoderma lucidum polysaccharide GLP-4 combined with chemotherapy on blood $CD3^+/CD4^+$ and $CD3^+/CD8^+$ ratios in orthotopic H22-bearing mice ( $\bar{x} \pm s$ )

| Group | $CD3^+/CD4^+$ | $CD3^+/CD8^+$ |
|---|---|---|
| Normal control group | 2.97 ± 0.12 | 3.62 ± 0.38 |
| Model control group | 4.76 ± 1.37 | 4.73 ± 0.75 |
| Cisplatin group | 4.26 ± 0.04 | 4.21 ± 0.81 |
| Kanglaite soft capsule group | 4.20 ± 0.70 | 6.58 ± 2.91 |
| Cisplatin + Kanglaite soft capsule group | 3.88 ± 0.88 | 9.58 ± 3.62 |
| Cisplatin + GLP-4 low-dose group | **11.51 ± 0.45$^{*\#\&\star}$** | **21.10 ± 2.69$^{*\#\&}$** |
| Cisplatin + GLP-4 high-dose group | **9.50 ± 0.08$^{\#\&\star}$** | **17.79 ± 1.15$^{*\#}$** |

Note: Compared to the model control group, *P ≤ 0.05; compared to the cisplatin group, $^{\#}P \leq 0.05$; compared to the Kanglaite soft capsule group, $^{\&}P \leq 0.05$; compared to the cisplatin + Kanglaite soft capsule group, *P ≤ 0.05.

As shown in Figures 37-40, the mice in the model control group exhibited extensive hepatocellular carcinoma cell infiltration and necrosis, increased number of sinusoidal cells, and inflammatory cell infiltration in the liver; evident extramedullary hematopoiesis and diffuse red pulp in the spleen, liver cancer cell infiltration in the stomach and lesions on the serosa, with widespread atrophy of the gastric mucosa. After administration of Ganoderma lucidum polysaccharide GLP-4 combined with cisplatin, the extent of liver cancer cell infiltration and necrosis decreased in all groups, with increased splenic extramedullary hematopoiesis, and no significant lesions observed in the stomach and kidneys.

As shown in Figures 37-40, the corresponding groups are as follows: A: normal group, B: model control group, C: cisplatin group, D: Kanglaite soft capsule group, E: cisplatin + Kanglaite soft capsule group, F: cisplatin + GLP-4 low-dose group, G: cisplatin + GLP-4 high-dose group

Conclusion

Ganoderma lucidum polysaccharide GLP-4 combined with cisplatin significantly inhibits the growth of tumors in orthotopic H22-bearing mice and demonstrates a notable synergistic effect.

**Discussion and Conclusion**

Liver cancer is a highly lethal malignancy, where current treatments involving surgical resection, chemotherapy, and radiotherapy merely delay symptoms, making complete cure extremely difficult. Liver cancer is notably resistant to chemotherapy, especially in cases of advanced liver cancer, where there is no reliable evidence proving that systemic chemotherapy can improve overall survival of patients with advanced liver cancer.

The results of this study showed that in the model control group of mice, tumor volume significantly increased, spleen and liver indices significantly increased, red blood cell counts in the blood significantly increased, white blood cell counts significantly decreased, and liver and kidney functions were significantly abnormal. These findings indicate a decline in lymphatic system function during tumor progression in the model control group of mice. After the last dose, Ganoderma lucidum polysaccharide GLP-4 combined with cisplatin significantly inhibited the growth of tumors in mice, significantly reduced the thymus index and spleen index, significantly reduced the number of red blood cells in the blood of mice, significantly increased the number of white blood cells, and significantly lowered kidney function indicators and liver function indicators. $CD4^+$T cells and $CD8^+$T cells mediate tumor immune responses, where $CD8^+$T cells are the main effector cells of tumor immunity. Results indicate that Ganoderma lucidum polysaccharide GLP-4 combined with cisplatin significantly increased $CD3^+/CD4^+$ and $CD3^+/CD8^+$ ratios in the mice's blood, suggesting that Ganoderma lucidum polysaccharide GLP-4 combined with cisplatin can protect immune organs and enhance the body's own immune function, thereby playing a role in reducing toxicity and enhancing antitumor effects. Histopathological results also showed that Ganoderma lucidum polysaccharide GLP-4 can enhance the body's immunity. Additionally, when compared to Kanglaite soft capsule combined with cisplatin, Ganoderma lucidum polysaccharide GLP-4 combined with cisplatin significantly reduced tumor, spleen indices, and thymus indices, indicating that Ganoderma lucidum polysaccharide GLP-4 combined with cisplatin has a stronger antitumor effect than Kanglaite soft capsule combined with cisplatin.

In conclusion, Ganoderma lucidum polysaccharide GLP-4 combined with cisplatin significantly inhibits the growth of tumors in orthotopic H22-bearing mice and demonstrates a notable synergistic effect.

**Ganoderma Lucidum Polysaccharide GLP-4 Colocalization Experiment with RAW264.7 Macrophage Lysosomes and Study Data**

**Experimental Objective:**

To investigate the colocalization of Ganoderma lucidum polysaccharide GLP-4 with lysosomes in RAW264.7 macrophages to elucidate the mechanism of action of this compound and provide experimental evidence for clinical research.

**Experimental Materials**

**Test Sample**

Ganoderma lucidum polysaccharide GLP-4, batch number: OLMJT202108(2-4), provided by Shenzhen Aolimei Oncology Medical Technology Co., Ltd.

**Cell Line**

RAW264.7 macrophage cell line, purchased from the Shanghai Institutes for Biological Sciences, Chinese Academy of Sciences.

**Main Reagents**

PBS, prepared by Shenzhen Aolimei Oncology Medical Technology Co., Ltd.; DMEM culture medium, product of Gibco; Cell Navigator Lysosome Staining Kit, product of Beijing Solarbio Science & Technology Co., Ltd.; DAPI dye, product of Tianjin Baima Technology Co., Ltd.; HBSS buffer, product of Gibco.

**Main Instruments**

20 μL, 200 μL, and 1 mL pipettes, products of Mettler-Toledo Ltd.; Luna-II Cell Counter, product of Nanjing Hengqiao Instrument Co., Ltd.; Laser scanning confocal microscope, product of Leica.

**Experimental Methods:**

RAW264.7 macrophages were cultured in DMEM containing 10% serum at 37°C in a 5% $CO_2$ incubator until logarithmic growth phase was reached. 150,000 cells were seeded in six-well plates and cultured for 1-2 days until cell density exceeded 50%. Fluorescently labeled GLP-4 was added and incubated for 24 h, followed by the addition of lysosome staining agent and DAPI dye. Images were captured using a laser scanning confocal microscope under 488 nm, 550 nm, and UV light.

**Experimental Results**

As shown in Figures 41-45, after incubation of Ganoderma lucidum polysaccharide GLP-4 with RAW264.7 macrophages for 24 h, most of the cells contained the drug, indicating that GLP-4 could be phagocytized by macrophages. Colocalization images of the fluorescently labeled GLP-4 with lysosomes demonstrated that the drug enters macrophages via the endolysosomal pathway. In Figure 44, cells stained with DAPI that had died contained fluorescently labeled GLP-4 but no lysosomes, suggesting that the macrophage lysosomes could not fully digest the drug, allowing GLP-4 to be presented by macrophages to other immune cells, successfully activating the immune system and enhancing immunity.

Figure 41 shows a confocal image of the RAW264.7 macrophage control group under 40x magnification. A: Combined image of bright field and DAPI staining; B: Image under the FITC fluorescent channel; C: Image of lysosomes labeled with lysosome fluorescent dye; D: Combined image of C and bright field.

Figure 42 shows the confocal image of 0.25 mg/mL GLP-4 with RAW264.7 macrophages after 24 h incubation at 40x magnification. A: Image combining bright field and DAPI staining; B: Image of GLP-4 labeled with FITC; C: Image of lysosomes labeled with lysosomal fluorescent dye; D: Combined image of B and C; arrows indicate the colocalization of GLP-4 and lysosomes.

Figure 43 shows the confocal image of 0.25 mg/mL GLP-4 with RAW264.7 macrophages after 24 h incubation at 63x magnification. A: Image combining bright field and DAPI staining; B: Image of GLP-4 labeled with FITC; C: Image of lysosomes labeled with lysosomal fluorescent dye; D: Combined image of B and C; arrows indicate the colocalization of GLP-4 and lysosomes.

Figure 44 shows the confocal image of 0.5 mg/mL GLP-4 with RAW264.7 macrophages after 24 h incubation at 40x magnification. A: Image combining bright field and DAPI staining; B: Image of GLP-4 labeled with FITC; C: Image of lysosomes labeled with lysosomal fluorescent dye; D: Combined image of B and C; arrows indicate the colocalization of GLP-4 and lysosomes.

Figure 45 shows the confocal image of 0.5 mg/mL GLP-4 with RAW264.7 macrophages after 24 h incubation at 63x magnification. A: Image combining bright field and DAPI staining; B: Image of GLP-4 labeled with FITC; C: Image of lysosomes labeled with lysosomal fluorescent dye; D: Combined image of B and C; arrows indicate the colocalization of GLP-4 and lysosomes.

Conclusion

Ganoderma lucidum polysaccharide GLP-4 is capable of being phagocytized by RAW264.7 macrophages and can activate the body's immune response.

**Experimental Study on the Cytotoxic Effects of Ganoderma Lucidum Polysaccharide GLP-4 Combined with Cisplatin on H22 Cells and Study Data**

**Experimental Objective:**

To investigate the cytotoxic effects of Ganoderma lucidum polysaccharide GLP-4 on H22 cells, providing experimental basis for its clinical research.

**Experimental Materials**

**Test Sample**

Ganoderma lucidum polysaccharide GLP-4, batch number: OLMJT202108(2-4), provided by Shenzhen Aolimei Oncology Medical Technology Co., Ltd.

**Positive Control**

Cisplatin, batch number: E2128081, product of Shanghai Aladdin Biochemical Technology Co., Ltd.

**Cell Line**

H22 cell line, purchased from the Shanghai Institutes for Biological Sciences, Chinese Academy of Sciences.

**Main Reagents**

PBS, prepared by Shenzhen Aolimei Oncology Medical Technology Co., Ltd.; 0.25% Trypsin, product of Gibco; 1640 medium, product of Gibco.

**Main Instruments**

20 μL, 200 μL, and 1 mL pipettes, products of Mettler-Toledo Ltd.; Luna-II Cell Counter, product of Nanjing Hengqiao Instrument Co., Ltd.; Laser scanning confocal microscope, product of Leica.

**Experimental Methods:**

H22 cells were cultured in 1640 medium supplemented with 10% serum at 37°C in a 5% CO2 incubator until logarithmic growth phase was reached. The culture medium was discarded, cells were washed with PBS and digested with 0.125% trypsin. After digestion, the reaction was stopped with medium, and cells were transferred to EP tubes for counting using a cell counter. After counting, 40,000 cells per well were seeded into a 12-well plate and incubated at 37°C in a 5% $CO_2$ incubator. 24 h later, once cells adhered, drugs were added. 48 h after drug administration, cells were digested with trypsin, washed with PBS, and recounted. The corresponding volume of staining solution was added based on cell count, then incubated for 30 min in an incubator. After incubation, cells were chilled to stop staining and maintain viability, followed by imaging using a confocal microscope.

**Dosage Design**

Based on preliminary study results, the dosing concentration for Ganoderma lucidum polysaccharide GLP-4 was set at 2 mg/mL, and for cisplatin (DDP) at 5 μg/mL.

**Experimental Results**

As shown in Table 25 and Figure 46, GLP-4 induced necrosis in H22 cells. When combined with cisplatin, it reduced the apoptosis and necrosis caused by cisplatin, thereby diminishing the direct toxicity of cisplatin.

Table 25: Effects of Ganoderma lucidum polysaccharide GLP-4 combined with cisplatin on necrosis and apoptosis of H22 cells

| Group | Apoptosis rate | | | Mean ± SD | Necrosis rate | | | Mean ± SD | Total mortality rate | | | Mean ± SD |
|---|---|---|---|---|---|---|---|---|---|---|---|---|
| Blank group | 0.1 | 0.3 | 0.3 | 0.2±0 | 4.5 | 8.5 | 3.9 | 5.6±2 | 4.7 | 8.8 | 4.2 | 5.8±2 |
| GLP-4 | 0.0 | 0.0 | 0.0 | 0±0 | 9.4 | 4.1 | 9.5 | 7.6±2.5 | 9.4 | 4.1 | 9.5 | 7.6±2.5 |
| Cisplatin + GLP-4 | 0.8 | 0.1 | 0.2 | 0.3±0.3 | 22.2 | 28.3 | 22.9 | 24.4±2.7 | 23.0 | 28.4 | 23.1 | 24.8±2.5 |
| Cisplatin group | 11.3 | 8.8 | 12.9 | 10.9±1.6 | 27.3 | 28.6 | 45.4 | 33.7±8.2 | 38.7 | 37.3 | 58.2 | 44.7±9.5 |

Figure 46 illustrates the effects of Ganoderma lucidum polysaccharide GLP-4 combined with cisplatin on the necrosis and apoptosis rates of H22 cells.

Conclusion

Ganoderma lucidum polysaccharide GLP-4 induces necrosis in H22 cells and, when used in combination with cisplatin, reduces the apoptosis and necrosis caused by cisplatin, thus lowering cisplatin's direct toxicity.

**Experimental Study on the Antitumor Effect of Ganoderma Lucidum Polysaccharide GLP-3 Combined with Chemotherapy on 4T1 Breast Tumor-Bearing Mice and Study Data**

**Experimental Objective**

To study the effects of Ganoderma lucidum polysaccharide GLP-4 on breast cancer-bearing mice using BALB/c mice subcutaneously inoculated with 4T1 breast cancer cells on their left shoulder back, providing experimental evidence for its clinical research.

**Experimental Materials**

**Test Sample**

Ganoderma lucidum polysaccharide GLP-4, batch number: OLM2019071617, provided by Shenzhen Aolimei Oncology Medical Technology Co., Ltd.

**Positive Control**

Cisplatin, batch number: E2128081, product of Shanghai Aladdin Biochemical Technology Co., Ltd.

**Laboratory Animals**

65 SPF female BALB/c mice, weighing 16-18 g, supplied by Guangdong Medical Laboratory Animal Center. Laboratory animal production license number: SCXK (Yue) 2022-0002; laboratory animal quality certification number: 44007200100339.

**Main Reagents**

PBS buffer, prepared by Shenzhen Aolimei Oncology Medical Technology Co., Ltd.; fetal bovine serum, product of Zhejiang Tianhang Biotechnology Co., Ltd.; 1640 culture medium, product of Gibco; 0.25% trypsin, product of Gibco.

**Main Instruments**

Vernier caliper, product of Shanghai Tool Works Co., Ltd.; I-2000 scale, Dongguan Nancheng Changxie Electronic Products Factory; ophthalmic scissors and tweezers, products of Shanghai Jinzhong Medical Instrument Co., Ltd.; CCL-170B-8 $CO_2$ incubator, product of ESCO, Singapore; Luna-II cell counter, product of Nanjing Hengqiao Instrument Co., Ltd.

**Experimental Methods**

A suspension of 4T1 breast cancer cells was injected subcutaneously into the left shoulder back of 55 healthy female BALB/c mice to create solid tumor models. When the mean tumor volume reached approximately 240 $mm^3$, the mice were randomized into groups based on tumor volume, and were administered the respective drugs or drug solvents via oral gavage or intraperitoneal injection over 25 consecutive days. Longest and shortest diameters of tumors were measured every three days to calculate tumor volume, and mouse weights were recorded every three days. At the end of the experiment, tumors, spleens, and thymuses were harvested and weighed to calculate tumor, spleen, and thymus indices.

### Dosage Design

Based on previous experimental results, Ganoderma lucidum polysaccharide GLP-4 was administered at a low dose of 130 mg/kg and a high dose of 1170 mg/kg as shown in Table 26.

Rationale for cisplatin dosage design: Based on the clinical dosage of cisplatin, which should not exceed 100 $mg/m^2$ per person per day, and considering the tolerance of mice to cisplatin, a dose of 4 mg/kg has been selected as the administration dosage.

Table 26: Experimental groups and dosage design

| Group | Dose (mg/kg) | Method of administration | Administration volume (mL/10 g) | Frequency of administration |
|---|---|---|---|---|
| Model control group | - | Intraperitoneal injection | 0.1 | Once daily |
| Cisplatin group | 4 | Intraperitoneal injection | 0.1 | Once every 3 days |
| Cisplatin + GLP-4 low-dose group | 4+130 | Intraperitoneal injection + oral gavage | 0.1+0.2 | Cisplatin, once every 3 days; GLP-4, once daily |
| Cisplatin + GLP-4 high-dose group | 4+1170 | Intraperitoneal injection + oral gavage | 0.1+0.2 | Cisplatin, once every 3 days; GLP-4, once daily |

### Test Indicators

### Efficacy Indicators

### Relative tumor growth inhibition rate

Relative tumor growth inhibition rate (%) = (1 - $T_{RTV}$ / $C_{RTV}$) × 100%. Where $T_{RTV}$ is the relative tumor volume in the experimental group, and $C_{RTV}$ is the relative tumor volume in the model control group. Relative tumor volume (RTV) = $V_t$ / $V_0$, where $V_t$ is the tumor volume on day t of dosing, and $V_0$ is the tumor volume at the time of grouping. Evaluation criteria: A relative tumor growth inhibition rate of ≥ 40% and a statistical analysis with $P < 0.05$ indicate effective inhibition.

### Tumor growth inhibition rate

Tumor growth inhibition rate (%) = (1 - T / C) × 100%. Where T represents the average tumor weight in the treatment group, and C represents the average tumor weight in the model control group. Evaluation criteria: A tumor growth inhibition rate of ≥ 40% and a statistical analysis with $P < 0.05$ indicate effective inhibition.

**Spleen and thymus organ coefficients:** After the last dose, spleen, thymus, and tumor weights are measured, and organ coefficients are calculated.

### Data Processing and Statistical Analysis

Statistical analyses were performed using SPSS 17.0, with the significance level set at $P \leq 0.05$. Measurement data were expressed as mean ± standard deviation ( $\bar{x} \pm s$ ). Normality and homogeneity of variance were tested using Leven's test. If data met normality and homogeneity of variance ($P > 0.05$), one-way ANOVA and LSD test were used for statistical analysis. If data did not meet normality and homogeneity of variance ($P < 0.05$), the Kruskal-Wallis test was used. If the Kruskal-Wallis test was statistically significant ($P < 0.05$), comparison analysis was performed using Dunnett's Test (a non-parametric method). Evaluation considered statistical differences and biological significance.

### Experimental Results

### Animal Mortality

As shown in Table 27, the mortality rate for the model control group of mice was 60%, while the mortality rates for all other groups were 0.

Table 27: Statistics on the number of surviving animals and mortality rate in each group

| Group | Total number of animals | Number of deaths | Mortality rate (%) | Survival time (days) |
|---|---|---|---|---|
| Model control group | 10 | 6 | 60.0 | 24.3±1 |
| Cisplatin group | 10 | 0 | 0.0 | 25±0 |
| Cisplatin + GLP-4 low-dose group | 10 | 0 | 0.0 | 25±0 |
| Cisplatin + GLP-4 high-dose group | 10 | 0 | 0.0 | 25±0 |

**Effect of Ganoderma Lucidum Polysaccharide GLP-4 Combined with Chemotherapy on Body Weight of 4T1 Breast Tumor-Bearing Mice**

As shown in Table 28, compared to the model control group, the body weight of mice in the cisplatin group, cisplatin + GLP-4 low-dose group, and cisplatin + GLP-4 high-dose group significantly decreased from D3 to D22. Compared to the cisplatin group, the body weight of mice in the cisplatin + GLP-4 high-dose group significantly decreased on D19 and D22.

Table 28: Effect of Ganoderma lucidum polysaccharide GLP-4 combined with chemotherapy on body weight of 4T1 breast tumor-bearing mice ( x ± s)

| Group | Weight ( g) | | | | | | | | |
|---|---|---|---|---|---|---|---|---|---|
| | D0 | D3 | D6 | D9 | D12 | D15 | D19 | D22 | D25 |
| Model control group | 18.9±1. 5 | 20.1±1.4 | 20±1.9 | 20.5±0.9 | 21.3±2.1 | 22.3±1.4 | 20.9±1.8 | 20.8±1.8 | 20.6±2. 3 |
| Cisplatin group | 18.3±1. 3 | **17.7±1.1 *** | **15.5±1*** | **15±1.4*** | **13.8±0.9 *** | **15±1.1*** | **16.8±1.4 *** | **18±1.5*** | 18.1±1. 6 |
| Cisplatin + GLP-4 low-dose group | 18.5±1 | **17.8±1.2 *** | **14.8±1.1 *** | **13.8±1.3 *** | **13.1±1.2 *** | **13.8±1.1 *** | **15.1±1.5 *** | **16.3±1.5 *** | 17±1.5 |
| Cisplatin + GLP-4 high-dose group | 18.9±1 | **17.8±0.7 *** | **15.7+0.7** * | **14.4±0.7 *** | **13.3±0.6 *** | **13.7±1*** | **15±1.6*#** | **15.8±2*#** | 17±1.8 |
| Note: Compared to the model control group, **P* < 0.05; Compared to the cisplatin group, #*P* < 0.05. | | | | | | | | | |

**Effect of Ganoderma Lucidum Polysaccharide GLP-4 Combined with Chemotherapy on Tumor Volume in 4T1 Breast Tumor-Bearing Mice**

As shown in Table 29, compared to the model control group, the tumor volume in the cisplatin group, the cisplatin + GLP-4 low-dose group, and the cisplatin + GLP-4 high-dose group was significantly reduced from D3 to D22.

Compared to the cisplatin group, the tumor volume in the cisplatin + GLP-4 low-dose group was significantly reduced on D12, and the tumor volume in the cisplatin + GLP-4 high-dose group was significantly reduced on D19 and D22.

Table 29: Effect of Ganoderma lucidum polysaccharide GLP-4 combined with chemotherapy on tumor volume in 4T1 breast tumor-bearing mice ( x ± s)

| Group | Tumor volume ($mm^3$) | | | | | | | | |
|---|---|---|---|---|---|---|---|---|---|
| | D0 | D3 | D6 | D9 | D12 | D15 | D19 | D22 | D25 |
| Model control **group** | 238.4±92.8 | 574,2±131.4 | 1111.5±360.1 | **14614±485.3** | 1934.5=787.4 | 2432.4±779.2 | 3273.1±794.8 | 3595.3±755.9 | **2761.6±794.1** |
| Cisplatin group | 225.3±65.4 | **464.9±84.8*** | **590.3±131.9*** | **728.4±203.7*** | **816.9±215.2*** | **1000.±252.1*** | **1653.2±493.9*** | **2023.2±654.6*** | 2026.4±707 |
| Cisplatin + GLP-4 low-dose group | 228.1±66.5 | **386.1±86.6*** | **486.6±136.4*** | **514.3±123.9*** | **460.1±116.9*#** | **658.6±151.4*** | **1961.7±181.3*** | **1371.3±264.2*** | 1624.4±282.7 |
| Cisplatin + GLP-4 high-dose group | 240.8±68.6 | **407.2±93.6*** | **486.4±149*** | **462.9±162.2*** | **490.5±149*** | **632.±246.6*** | **951.4±303.4*#** | **1075.4±364.4*#** | **1319.3±479.4** |

Note: Compared to the model control group, *P < 0.05; Compared to the cisplatin group, #*P* < 0.05.

As shown in Table 30, compared to the model control group, the cisplatin group showed a relative tumor growth inhibition rate of over 40% from D6 to D25, peaking at 56.9%. The cisplatin + GLP-4 low-dose group maintained a relative tumor growth inhibition rate of over 50% from D6 to D25, reaching up to 75.2%, and the cisplatin + GLP-4 high-dose group maintained a relative tumor growth inhibition rate of over 55% from D6 to D18, peaking at 74.0%.

Compared to the cisplatin group, the relative tumor growth inhibition rate for the cisplatin + GLP-4 low-dose group ranged from 22% to 44% *(P* < 0.05) from D6 to D22, and the relative tumor growth inhibition rate for the cisplatin + GLP-4 high-dose group ranged from 24% to 50% *(P* < 0.05) from D6 to D25.

Table 30: Effect of Ganoderma lucidum polysaccharide GLP-4 combined with chemotherapy on relative tumor growth inhibition rate in 4T1 breast tumor-bearing mice (vs. the model control group)

| Group | Relative tumor growth inhibition rate (compared to model control group) (%) | | | | | | | |
|---|---|---|---|---|---|---|---|---|
| | D3 | D6 | D9 | D12 | D15 | D19 | D22 | D25 |
| Model control group | - | - | - | - | - | - | - | - |
| Cisplatin group | 17.2 | **43.5*** | **49.1*** | **55.7*** | **56.9*** | **48.8*** | **44.1*** | **42.1*** |
| Cisplatin + GLP-4 low-dose group | 32.4 | **56.3*** | **65.4*** | **75.2*** | **72.7*** | **66.7*** | **60.9*** | **53.6*** |
| Cisplatin + GLP-4 high-dose group | 33.5 | **57.4*** | **68.6*** | **73.9*** | **74.0*** | **71.7*** | **71.3*** | **63.7*** |

Note: Compared to the model control group, *P < 0.05.

Table 31: Effect of Ganoderma lucidum polysaccharide GLP-4 combined with chemotherapy on relative tumor growth inhibition rate in 4T1 breast tumor-bearing mice (vs. the cisplatin group)

| Group | Relative tumor growth inhibition rate (compared to cisplatin group) (%) | | | | | | | |
|---|---|---|---|---|---|---|---|---|
| | D3 | D6 | D9 | D12 | D15 | D19 | D22 | D25 |
| Cisplatin group | - | - | - | - | - | - | - | - |
| Cisplatin + GLP-4 low-dose group | 18.3 | **22.6#** | **32.0#** | **44.0#** | **36.6#** | **35.0#** | **30.0#** | 19.8 |
| Cisplatin + GLP-4 high-dose group | 19.7 | **24.6#** | **38.3#** | **41.2#** | **39.7#** | **44.7#** | **48.6#** | **37.3#** |

Note: Compared to the cisplatin group, #*P* < 0.05.

**Effect of Ganoderma Lucidum Polysaccharide GLP-4 Combined with Chemotherapy on Organ Coefficients and Tumor Growth Inhibition Rates in 4T1 Breast Tumor-Bearing Mice**

As shown in Table 32, compared to the model control group, the tumor index, spleen index, and thymus index was significantly reduced in the cisplatin + GLP-4 group. Compared to the cisplatin group, the tumor index was significantly reduced in the cisplatin + GLP-4 high-dose group, and the spleen index was significantly reduced in the cisplatin + GLP-4 group.

Compared to the model control group, the tumor growth inhibition rate was 29.6% in the cisplatin group, and 49.5% and 60.1% in the cisplatin + GLP-4 low-dose and high-dose groups, respectively. Compared to the cisplatin group, the tumor growth inhibition rate was 28.2% and 43.3% in the cisplatin + GLP-4 low-dose and high-dose groups, respectively.

Table 32: Effect of Ganoderma lucidum polysaccharide GLP-4 combined with chemotherapy on organ indices and tumor growth inhibition rates in 4T1 breast tumor-bearing mice

| Group | **Tumor index** (%) | Spleen **index** (mg/g) | Thymus index (mg/g) | Tumor growth inhibition rate (%) vs. the model control group | Tumor growth inhibition rate (%) vs. the cisplatin group |
|---|---|---|---|---|---|
| Model control group | 15±5.1 | 3.8±1.1 | 0.07±0.03 | - | - |
| Cisplatin | 11.5±3.3 | 4.2±0.3 | 0.06±0.04 | 29.6 | - |
| Cisplatin + GLP-4 low-dose group | **8.9±2.2*** | **2.8±0.9*#** | 0.04±0.01 | 49.5 | 28.2 |

(continued)

| Group | **Tumor index** (%) | Spleen **index** (mg/g) | Thymus index (mg/g) | Tumor growth inhibition rate (%) vs. the model control group | Tumor growth inhibition rate (%) vs. the cisplatin group |
|---|---|---|---|---|---|
| Cisplatin + GLP-4 high-dose group | **6.8±2.5*#** | **2.8±0.8*#** | 0.04±0.02 | 60.1 | 43.3 |
| Note: Compared to the model control group, *$P$ < 0.05; Compared to the cisplatin group, #$P$ < 0.05. | | | | | |

Figures 47-50 show images of the tumor-bearing mice, corresponding to the groups as follows: Figure 47: model control group, Figure 48: cisplatin group, Figure 49: cisplatin + GLP-4 low-dose group, Figure 50: cisplatin + GLP-4 high-dose group.

Figures 51-54 show images of the tumors in the tumor-bearing mice, corresponding to the groups as follows: Figure 51: model control group, Figure 52: cisplatin group, Figure 53: cisplatin + GLP-4 low-dose group, Figure 54: cisplatin + GLP-4 high-dose group.

Conclusion

Ganoderma Lucidum Polysaccharide GLP-4 combined with cisplatin significantly inhibits tumor growth in 4T1 breast tumor-bearing mice and exhibits a synergistic effect.

**Pharmacodynamic Experiment and Data of Bleomycin-induced Pulmonary Fibrosis Animal Model**

Experimental Objective

To investigate the therapeutic effects of Ganoderma lucidum polysaccharide GLP-4 on pulmonary fibrosis by utilizing a bleomycin-induced pulmonary fibrosis model in mice, providing experimental evidence for its clinical research.

**Experimental Materials**

**Test Sample**

Ganoderma lucidum polysaccharide GLP-4, batch number: OLMJT202208(20,23,24), provided by Shenzhen Aolimei Oncology Medical Technology Co., Ltd.

**Positive Control**

Pirfenidone capsules, batch number: 20220606, provided by Boji Medical Technology Co., Ltd.

**Laboratory Animals**

50 SPF C57BL/6J mice, weighing 21.1-25.0 g, purchased from Guangdong Charles River Laboratory Animal Technology Co., Ltd. Laboratory animal production license number: SCXK (Yue) 2020-0063. Laboratory animal quality certification number: No: 44829700008922.

**Main Reagents**

Bleomycin, batch number: C13661083, product of Macklin.

**Main Instruments**

TW423L Electronic Scale, product of Shimadzu, Japan; YP2001 Electronic Scale, product of Shanghai Jinping; JJ124BC Electronic Scale, product of G&G Measurement Plant; EMKA-WBP Animal Pulmonary Function Test System, product of EMKA.

## Experimental Methods

Fifty male SPF C57BL/6J mice were randomized by weight into the normal control group, model control group, positive control group, GLP-4 low-dose group, and GLP-4 high-dose group, with 10 animals in each group. Ten animals not subjected to modeling served as the normal control group. The remaining animals were modeled by administering bleomycin via tracheal injection at 5 mg/kg, 0.1 mL per mouse, one-time administration. Starting on day 7 post-modeling, the study drug, positive drug, or solvent was administered orally at 10 mL/kg once daily for 21 consecutive days. During the experimental period, daily observations were made for each group's food intake, activity, breathing, and responsiveness; pulmonary function was tested before administration and about 24 h after the last dose. After completing pulmonary function tests, the mice were euthanized for pathological sampling.

## Dosage Design

The intended clinical dose for Ganoderma lucidum polysaccharide GLP-4 is 1.0 g per day. Based on an adult weight of 60 kg and a mouse weight of 20 g, using a conversion coefficient for body surface area of 0.0026, the clinically equivalent dose for mice is approximately 187 mg/kg. The doses for GLP-4 were designed as low (near clinical equivalent) and high doses at 200 mg/kg and 600 mg/kg, respectively.

For the positive control drug, pirfenidone, based on a stable daily dose of 1,200 mg for adults, the clinically equivalent dose for mice was calculated as 225 mg/kg, which was set as the dose for the positive control group. This is shown in Table 33.

Table 33: Dose design and administration

| Group | Test substance | Dose administered (mg/kg) | Concentration administered (mg/mL) | Volume administered (mL/kg) | Number of doses /day | Equivalent to clinical dose (multiple) |
|---|---|---|---|---|---|---|
| Normal control group | 0.5%CMC-Na | / | / | 10 | 1 | / |
| Model control group | 0.5%CMC-Na | / | / | 10 | 1 | / |
| Positive control group | Pirfenidone | 225 | 22.5 | 10 | 1 | 1 |
| GLP-4 low-dose group | GLP-4 | 200 | 20 | 10 | 1 | 1 |
| GLP-4 high-dose group | GLP-4 | 600 | 60 | 10 | 1 | 3 |

Note: The dose conversion formula used is: logS = 0.8762 + 0.698logW (S: body surface area, unit: $cm^2$; W: weight, unit: g), calculated based on human weight of 60 kg and mouse weight of 20 g each.

## Test Indicators

**General behavioral observation:** During the trial period, daily observations were made on the animals' consumption, activity, breathing, and responsiveness.

**Lung function:** Lung function in mice was measured using the EMKA WBP animal lung function assessment system both before administration and approximately 24 h after the last dose. Measurements included inspiratory (expiratory) duration, maximum inspiratory (expiratory) volume, tidal volume, expiratory volume, relaxation time, minute ventilation, respiratory rate, end inspiratory (expiratory) pause, bronchoconstriction parameters, 50% expiratory flow, alveolar ventilation, and number of breaths.

**Pulmonary histopathology:** Following lung function tests, mice were euthanized, and a laparotomy was performed. Whole-body perfusion was conducted using saline through the portal vein followed by thoracotomy and perfusion-fixation with 10% formaldehyde solution for 5 min. The trachea was then ligated, the lungs were harvested, and further fixed in formalin solution for 48 h. Hematoxylin and eosin (H&E) staining was performed to observe pathological changes.

## Data Processing and Statistical Analysis

Experimental data were compiled using Excel and presented as mean ± standard deviation. Analysis was performed using SPSS 28.0 software. Homogeneity of variances was tested using one-way ANOVA. When variances were homogeneous ($P \geq 0.05$), intergroup comparisons were carried out using the LSD test; for heterogenous variances *(P* < 0.05), intergroup comparisons were carried out using the Dunnett's T3 test. Differences with $P < 0.05$ were considered statistically significant.

## Experimental Results

### General behavioral observation

During the experiment, compared to the normal control group, the model control group exhibited poor appetite, reduced activity, increased breathing rate, decreased responsiveness, and dull fur. Compared to the model control group, the positive control group, the GLP-4 low-dose group, and the GLP-4 high-dose group showed noticeable improvements in appetite, activity, breathing, responsiveness, and fur condition.

### Lung function test

Prior to administration (one week post-modeling), lung function indices are shown in Tables 34 and 35. Compared to the normal control group, the model control group exhibited significantly increased inspiratory duration (Ti), peak inspiratory flow (PIF), relaxation time (RT), end inspiratory pause (EIP), and number of breaths (n) ($p < 0.01$ or 0.05), and significantly decreased minute ventilation (MV) and bronchoconstriction parameters (Penh) ($p < 0.01$). The positive control group and GLP-4 low-dose and high-dose groups showed consistent indices with the model control group, indicating successful modeling as evidenced by the presence of lung function abnormalities. Compared to the model control group, the positive control group showed statistically significant differences in peak expiratory flow (PEF), MV, 50% expiratory flow (EF50), and n, likely due to individual animal variability and measurement errors, which were considered clinically insignificant.

21 days post-dosing, lung function indices are shown in Tables 36 and 37. Compared to the normal control group, the model control group showed significantly decreased Ti, PEF, tidal volume (TV), expiratory volume (EV), MV, Penh, and 50% expiratory flow (EF50) ($p < 0.01$ or 0.05). Compared to the model control group, the positive control group, and both GLP-4 low-dose and high-dose groups showed significant increases in PIF, PEF, TV, EV, MV, EF50, and alveolar ventilation (AV) ($p < 0.01$ or 0.05), with Ti also showing some increase. Statistically significant differences were observed between the positive control group and the GLP-4 low-dose group ($p < 0.05$). No statistical differences were noted when compared to the positive drug control group.

Table 34: Lung function test results 1 ($\bar{x} \pm s$)

| Group | Dose (mg/kg ) | Ti (msec) | Te (msec) | PIF (ml/s) | PEF (ml/s) | TV (ml) | EV (ml) | RT (msec) |
|---|---|---|---|---|---|---|---|---|
| Normal control | / | 66.87±5.00 | 59.23±19.74 | 4.07±0.78 | 6.09±1.03 | 0.15+0.0 3 | 0.15+0.0 3 | 43.85±20.28 |
| group | | | | | | | | |
| Model control group | / | 92.06±15.92# # | 93.23±39.87 | 4.99±1.00# # | 6.71±1.18 | 0.15±0.0 3 | 0.15±0.0 3 | 79.42±39.48 # |
| Positiv e control group | 225 | 109.26±23.25 | 120.91±48.1 2 | 4.63±0.69 | 5.94±0.94 * | 0.15±0.0 2 | 0.15±0.0 2 | 105.6±46.61 |
| GLP-4 low-dose | 200 | 101.82±11.29 | 106.16±44.4 7 | 5.09±0.54 | 6.66±0.93 | 0.16±0.0 2 | 0.16±0.0 2 | 91.56±43.65 |
| GLP-4 high-dose | 600 | 94.82±17.45 | 83.34±25.55 | 4.95±0.8 | 6.72±1.09 | 0.16±0.0 2 | 0.16±0.0 3 | 69.00±24.81 |

Note: Compared to the normal control group # $p < 0.05$, ## $p < 0.01$; compared to the model control group * $p < 0.05$, ** $p < 0.01$; compared to the positive drug control group & $p < 0.05$, && $p < 0.01$. Parameters include inspiratory duration (Ti), expiratory duration (Te), peak inspiratory flow (PIF), peak expiratory flow (PEF), tidal volume (TV), expiratory volume (EV), and relaxation time (RT).

Table 35: Lung function test results 2 ($\bar{x} \pm s$)

| Group | Dose (mg/kg) | MV (ml) | EIP (msec) | EEP (msec) | Penh | EF50 (ml/s) | AV (ml) | n |
|---|---|---|---|---|---|---|---|---|
| Normal control group | / | 84.62±18.29 | 3.00±1.40 | 5.48±0.71 | 0.99±0.13 | 5.87±1.02 | 70.05±30.76 | 21.00±2.49 |
| Model control group | / | 62.11±16.92## | 46.58±28.23## | 5.19±0.33 | 0.50±0.18## | 6.49±1.20 | 63.73±39.37 | 27.99±4.31## |
| Positive control group | 225 | 52.78±12.68* | 62.63±33.56 | 5.70±0.71 | 0.44±0.15 | 5.68±0.95* | 70.49±10.76 | 23.82±4.54** |
| GLP-4 low-dose | 200 | 58.44±11.19 | 51.6±22.86 | 5.53±0.52 | 0.44±0.12 | 6.45±0.87 | 79.26±15.27 | 27.59±4.30 |
| GLP-4 high-dose | 600 | 64.64±13.23 | 40.27±23.68 | 5.34±0.49 | 0.53±0.14 | 6.51±1.04 | 84.01±20.41 | 29.19±4.19 |

Note: Compared to the normal control group # $p < 0.05$, ## $p < 0.01$; compared to the model control group * $p < 0.05$, ** $p < 0.01$; compared to the positive drug control group & $p < 0.05$, && $p < 0.01$. Parameters include minute ventilation (MV), end inspiratory pause (EIP), end expiratory pause (EEP), bronchoconstriction parameters (Penh), 50% expiratory flow (EF50), alveolar ventilation (AV), and number of breaths (n).

Table 36: Lung function test results 3 ($\bar{x} \pm s$)

| Group | Dose (mg/kg) | Ti (msec) | Te (msec) | PIF (ml/s) | PEF (ml/s) | TV (ml) | EV (ml) | RT (msec) |
|---|---|---|---|---|---|---|---|---|
| Normal | / | 85.32±7.30 | 74.98±24.01 | 3.75±0.49 | 6.03±0.65 | 0.15±0.02 | 0.15±0.02 | 59.83±24.12 |
| control group | | | | | | | | |
| Model control group | / | 69.55±14.15## | 606.62±882.84 | 3.36±0.66 | 4.07±0.65## | 0.10±0.01## | 0.10±0.01## | 560.50±854.68 |
| Positive control group | 225 | 79.85±12.82* | 73.20±22.54 | 4.49±0.75* | 5.59±0.86** | 0.14±0.01** | 0.14±0.01** | 57.72±22.68 |
| GLP-4 low-dose | 200 | 79.74±6.54* | 67.02±29.38 | 4.92±1.13** | 6.39±1.17** | 0.16±0.03** | 0.16±0.03** | 51.57±28.39 |
| GLP-4 high-dose | 600 | 77.31±10.34 | 67.96±40.13 | 4.73±0.88** | 6.34±1.16** | 0.16±0.02** | 0.16±0.02** | 52.81±37.64 |

Note: Compared to the normal control group # $p < 0.05$, ## $p < 0.01$; compared to the model control group * $p < 0.05$, ** $p < 0.01$; compared to the positive drug control group & $p < 0.05$, && $p < 0.01$. Parameters include inspiratory duration (Ti), expiratory duration (Te), peak inspiratory flow (PIF), peak expiratory flow (PEF), tidal volume (TV), expiratory volume (EV), and relaxation time (RT).

Table 37: Lung function test results 4 ($\bar{x} \pm s$)

| Group | Dose (mg/kg) | MV (ml) | EIP (msec) | EEP (msec) | Penh | EF50 (ml/s) | AV (ml) | n |
|---|---|---|---|---|---|---|---|---|
| Normal control group | / | 70.22±13.31 | 6.87±1.80 | 5.42±0.54 | 0.92±0.12 | 5.72±0.71 | 68.78±44.37 | 19.73±3.94 |
| Model control group | / | 30.29±16.54## | 13.62±6.62 | 12.33±4.13 | 3 0.70±0.24# | 3.57±0.97## | 41.52±47.58 | 29.86±26.53 |
| Positive control group | 225 | 64.98±11.84** | 18.83±9.94 | 5.89±0.57 | 0.56±0.22 | 5.31±0.91** | 119.87±38.33** | 40.22±17.53 |
| GLP-4 low-dose | 200 | 79.04±18.06** | 11.37±6.11 | 5.72±0.99 | 0.68±0.15 | 6.17±1.15** | 124.63±53.9** | 38.96±14.65 |
| GLP-4 high-dose | 600 | 78.74±21.58** | 15.2±18.9 | 5.78±1.20 | 0.71±0.25 | 6.11±1.18** | 117.31±43.62** | 33.13±13.19 |

Note: Compared to the normal control group # $p < 0.05$, ## $p < 0.01$; compared to the model control group * $p < 0.05$, ** $p < 0.01$; compared to the positive drug control group & $p < 0.05$, && $p < 0.01$. Parameters include minute ventilation (MV), end inspiratory pause (EIP), end expiratory pause (EEP), bronchoconstriction parameters (Penh), 50% expiratory flow (EF50), alveolar ventilation (AV), and number of breaths (n).

**Pathological examination**

The pathological test results shown in Table 38 indicate that, compared to the normal control group, the lung pathology scores in the model control group significantly increased ($p < 0.01$). Compared to the model control group, the lung pathology scores in the GLP-4 low-dose group and the GLP-4 high-dose group significantly decreased ($p < 0.01$ or 0.05).

Table 38: Summary of pathology scores by group ($\overline{X} \pm S$)

| Group | Dose (mg/kg) | Pathology score |
|---|---|---|
| Normal control group | / | 0.20±0.63 |
| Model control group | / | 10.50±0.93## |
| Positive control group | 225 | 6.22±3.31 |
| GLP-4 low-dose | 200 | 5.22±3.03* |
| GLP-4 high-dose | 600 | 5.33±2.50** |

Note: Compared to the normal control group # $p < 0.05$, ## $p < 0.01$; compared to the model control group * $p < 0.05$, ** $p < 0.01$; compared to the positive drug control group & $p < 0.05$, && $p < 0.01$.

As shown in Figures 55-59, the normal control group showed normal alveolar structure with no inflammatory cell infiltration or fibrotic tissue proliferation. The model control group exhibited lung tissue damage (40%-60%), inflammatory cell infiltration (40%-60%), and fibrosis around blood vessels and within lung tissue (40%-60%). The positive control group showed lung tissue damage (1%-20%), inflammatory cell infiltration (1%-20%), and no fibrotic tissue proliferation. The GLP-4 low-dose group displayed normal alveolar structure, with inflammatory cell infiltration (1%-20%) and no fibrotic tissue proliferation around blood vessels or within lung tissue. The GLP-4 high dose group showed lung tissue damage (1%-20%), inflammatory cell infiltration (1%-20%), and no fibrotic tissue proliferation around blood vessels or within lung tissue.

In Figures 55-59: Figure 55: Normal control group; Figure 56: Model control group; Figure 57: Positive control group; Figure 58: GLP-4 low-dose group; Figure 59: GLP-4 high-dose group

**Conclusion**

Low doses of Ganoderma lucidum polysaccharide GLP-4 significantly improved the model animals' appetite, activity, respiration, responsiveness, and fur condition, significantly increased Ti, PIF, PEF, TV, EV, MV, EF50, AV, and improved lung tissue pathology, showing benefits in terms of alveolar tissue damage, inflammatory cell infiltration, and fibrotic tissue proliferation. High doses of Ganoderma lucidum polysaccharide GLP-4 improved the model animals' appetite, activity, respiration, responsiveness, and fur condition, significantly increased PIF, PEF, TV, EV, MV, EF50, AV, improved lung tissue pathology, showing benefits in terms of alveolar tissue damage, inflammatory cell infiltration, and fibrotic tissue proliferation.

In summary, under the conditions of this experiment, Ganoderma lucidum polysaccharide GLP-4 has shown therapeutic effects on pulmonary fibrosis by improving the general behavior, lung function, and lung tissue pathology in asthmatic model mice.

The foregoing description concerns merely exemplary embodiments of the present invention and is not intended to limit the invention. Any modifications, equivalent substitutions, and improvements made within the spirit and principles of the invention should be included within the scope of the invention's protection.

**Claims**

1. A type of Ganoderma lucidum polysaccharide GLP-4, **characterized in that** the molecular structural formula of the Ganoderma lucidum polysaccharide GLP-4 is

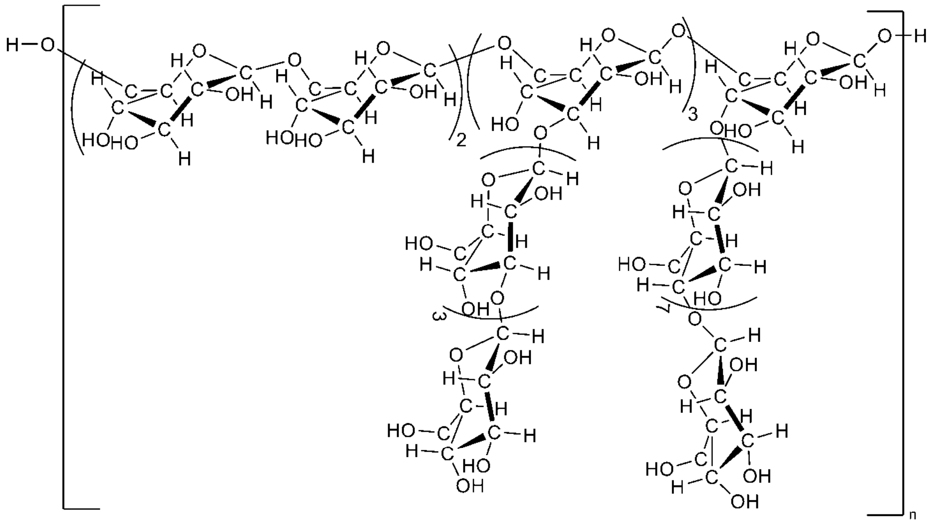

;

the molecular formula is: $(C_{162}H_{270}O_{135})_n$; where n = 10-15.

2. The Ganoderma lucidum polysaccharide GLP-4 according to claim 1, wherein n is selected from 10, 11, 12, 13, 14, or 15.

3. A method for extracting the Ganoderma lucidum polysaccharide GLP-4, comprising the steps of:

S1. dusting and drying Ganoderma lucidum, then crushing Ganoderma lucidum to make Ganoderma lucidum powder;
S2. placing the crushed Ganoderma lucidum in a sealed container mixed with water, heating under high temperature and pressure to fully dissolve the Ganoderma lucidum powder with the water into a medicinal juice solution;
S3. using centrifugation technology to separate the medicinal juice solution to obtain a concentrated solution with an active ingredient and medicinal residue;
S4. mixing the separated medicinal residue with a NaOH solution at a preset ratio followed by addition of HCl for neutralization;
S5. concentrating and desalting the mixed solution using membrane concentration technology to remove NaCl and obtain a concentrated solution with the active ingredient; and
S6. lyophilizing the concentrated solution containing the active ingredient and formulating it at a specific concentration, followed by multiple column chromatography separations to obtain the Ganoderma lucidum polysaccharide GLP-4 with the active ingredient.

4. The method for extracting Ganoderma lucidum polysaccharide GLP-4 according to claim 3, **characterized in that** in step S2, the mixture of the Ganoderma lucidum powder and the water in the sealed container is fully stirred and heated at a high temperature to 105-200°C, with boiling time lasting for 2-6 h, during which the internal pressure in the sealed container gradually increases as the heating temperature rises, forming a high-temperature and high-pressure environment within the sealed container.

5. The method for extracting Ganoderma lucidum polysaccharide GLP-4 according to claim 3, **characterized in that** in step S2, the mixture of the Ganoderma lucidum powder and the water in the sealed container is heated at a high temperature to 105-170°C, with boiling time lasting for 3-6 h, during which the internal pressure in the sealed container gradually increases as the heating temperature rises, forming the high-temperature and high-pressure environment within the sealed container.

6. The method for extracting Ganoderma lucidum polysaccharide GLP-4 according to claim 4 or 5, **characterized in that**, in step S6, pure water is added to the lyophilized powder of the concentrated solution containing the active ingredient to prepare a solution of 40-80 mg/mL, which then undergoes multiple column chromatography separations.

7. The method for extracting Ganoderma lucidum polysaccharide GLP-4 according to claim 6, **characterized in that**, in step S4, the medicinal residue is mixed and soaked with a NaOH solution at a temperature of 40-100°C at a ratio of

1:10 to 1:40, with a soaking time of 1-5 h, where the concentration of NaOH is 0.05-0.5 mol/L.

8. The method for extracting the Ganoderma lucidum polysaccharide GLP-4 according to claim 7, **characterized in that** in step S1, the Ganoderma lucidum is rinsed with clean water to remove surface dust and dried at 105°C, and the dried Ganoderma lucidum is crushed, with the crushed Ganoderma lucidum powder being larger than 60 mesh.

9. The method for extracting the Ganoderma lucidum polysaccharide GLP-4 according to claim 8, **characterized in that** in step S2, the mixed liquid in the sealed container is heated at a high temperature to 105°C, 110°C, 115°C, 120°C,125°C, 130°C, 135°C, 140°C, 145°C, 150°C, 155°C, 160°C, 165°C, 170°C, 175°C, 180°C, 185°C, 190°C, 195°C,or 200°C, with boiling times of 3 h, 3.5 h, 4 h, 4.5 h, 5 h, 5.5 h, or 6 h, during which the internal pressure in the sealed container gradually increases as the heating temperature rises, forming the high-temperature and high-pressure environment within the sealed container.

10. A use of the Ganoderma lucidum polysaccharide GLP-4 as claimed in claim 1 or 2, **characterized in that** the Ganoderma lucidum polysaccharide GLP-4 exhibits excellent water solubility, is easily absorbed by the human body, possesses anti-tumor efficacy, and demonstrates potent effects in preventing tumor occurrence in humans. Particularly when used in combination with chemotherapy drugs, it can eliminate the toxic side effects caused by chemotherapy drugs on the human body, control and reduce tumor masses, and decrease and eliminate tumor cells.

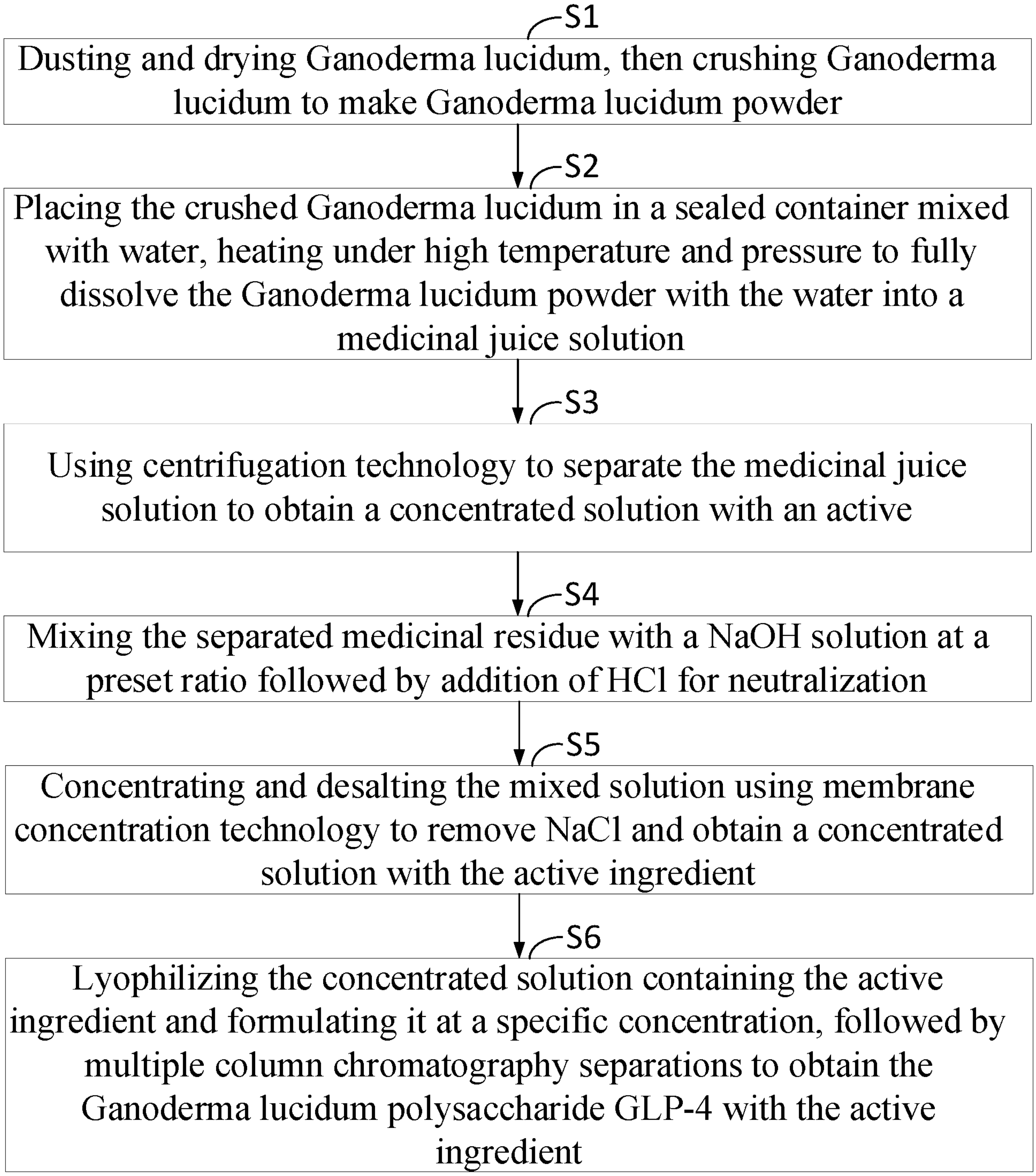
S1
Dusting and drying Ganoderma lucidum, then crushing Ganoderma lucidum to make Ganoderma lucidum powder
S2
Placing the crushed Ganoderma lucidum in a sealed container mixed with water, heating under high temperature and pressure to fully dissolve the Ganoderma lucidum powder with the water into a medicinal juice solution
S3
Using centrifugation technology to separate the medicinal juice solution to obtain a concentrated solution with an active
S4
Mixing the separated medicinal residue with a NaOH solution at a preset ratio followed by addition of HCl for neutralization
S5
Concentrating and desalting the mixed solution using membrane concentration technology to remove NaCl and obtain a concentrated solution with the active ingredient
S6
Lyophilizing the concentrated solution containing the active ingredient and formulating it at a specific concentration, followed by multiple column chromatography separations to obtain the Ganoderma lucidum polysaccharide GLP-4 with the active ingredient

Fig. 1

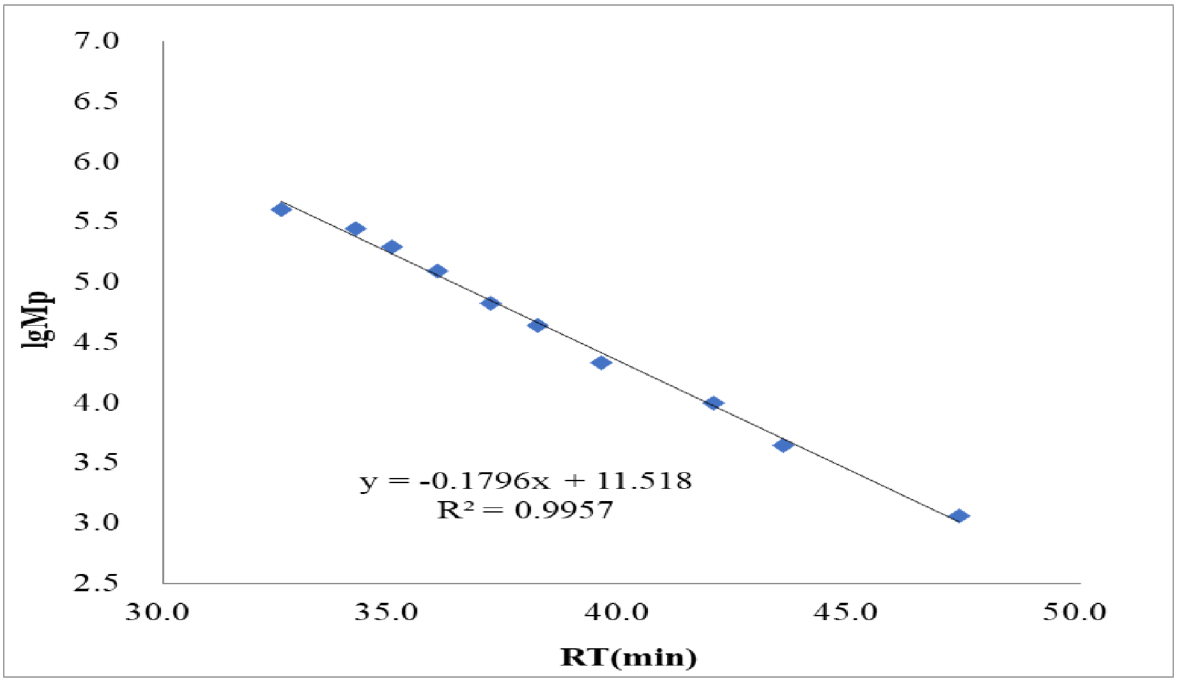
7.0
6.5
6.0
5.5
5.0
4.5
4.0
3.5
3.0
2.5
lgMp
y = -0.1796x + 11.518
R² = 0.9957
30.0
35.0
40.0
45.0
50.0
RT(min)

Fig. 2

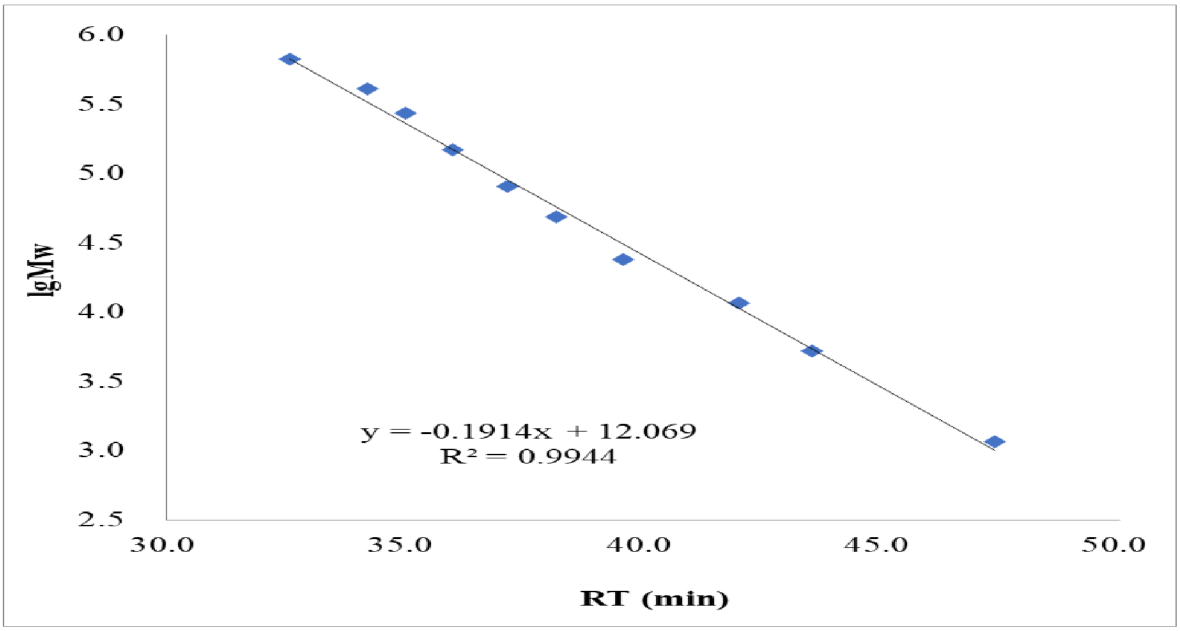
6.0
5.5
5.0
4.5
4.0
3.5
3.0
2.5
lgMw
y = -0.1914x + 12.069
R² = 0.9944
30.0
35.0
40.0
45.0
50.0
RT (min)

Fig. 3

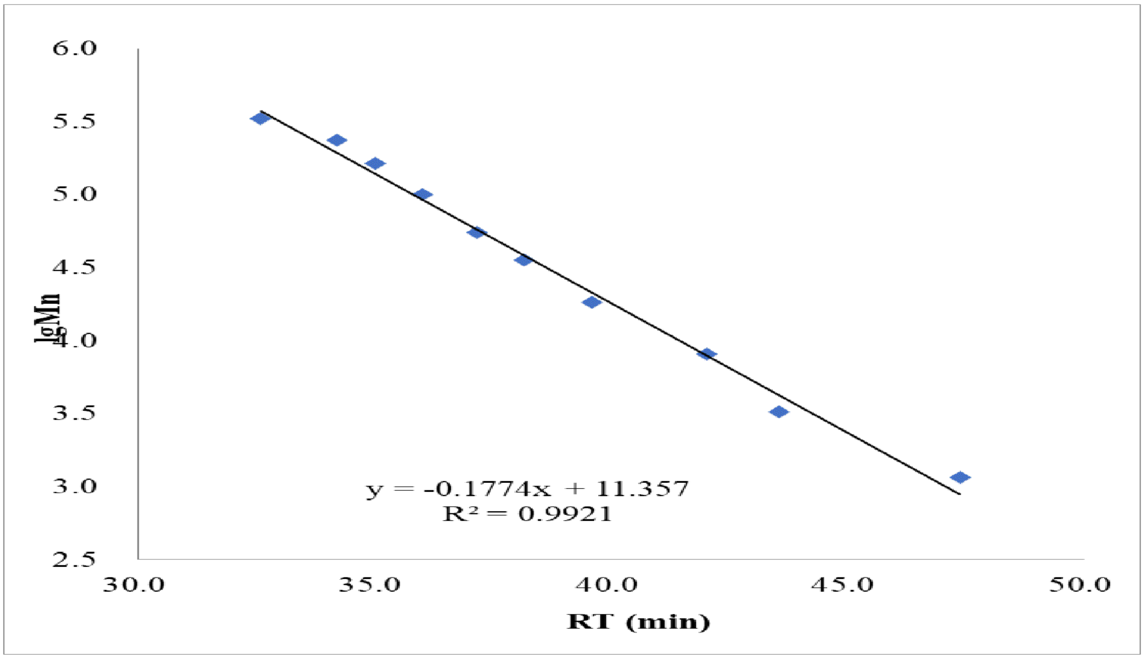


Fig. 4

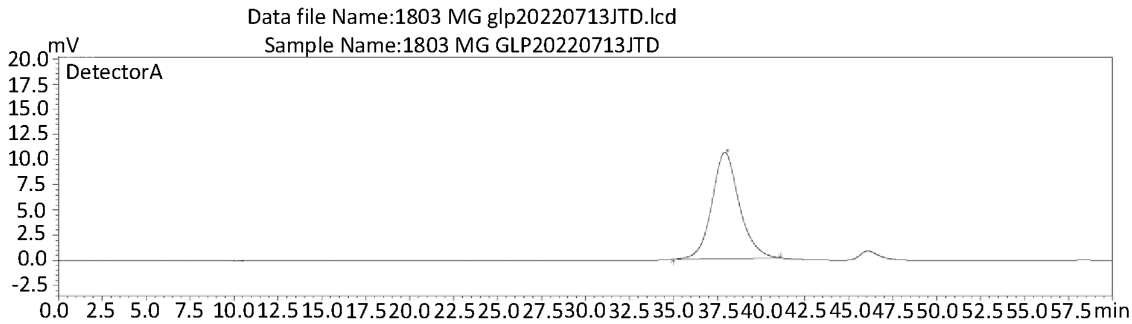


Fig. 5

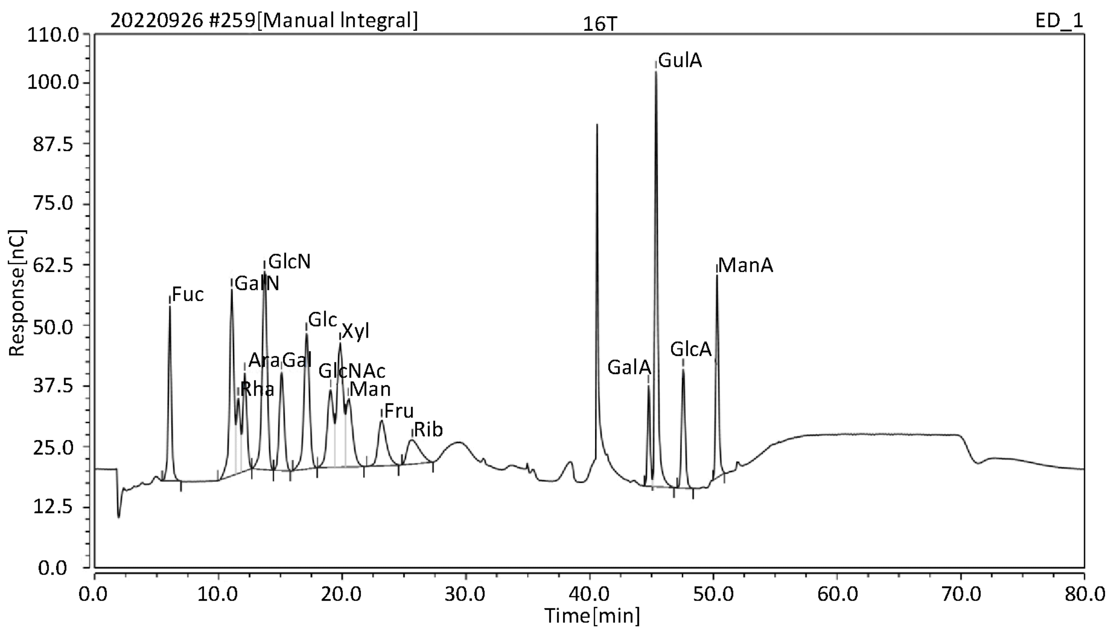


Fig. 6

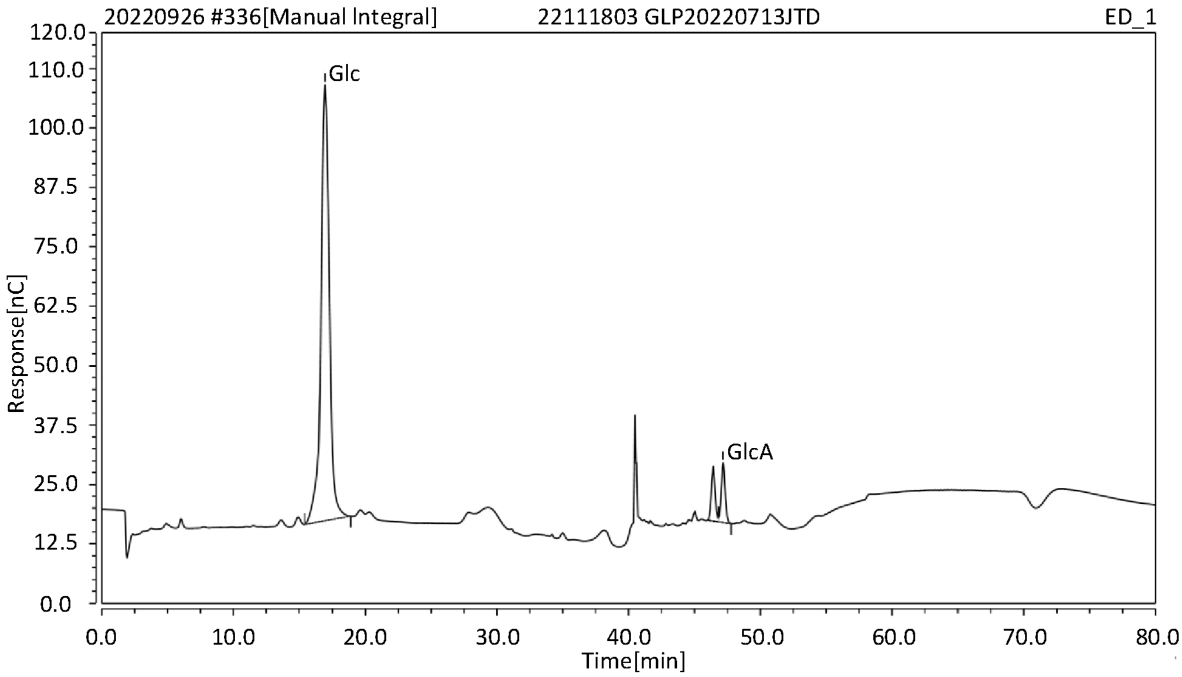

20220926 #336[Manual Integral]
22111803 GLP20220713JTD
ED_1
Glc
GlcA
Response[nC]
Time[min]


Fig. 7

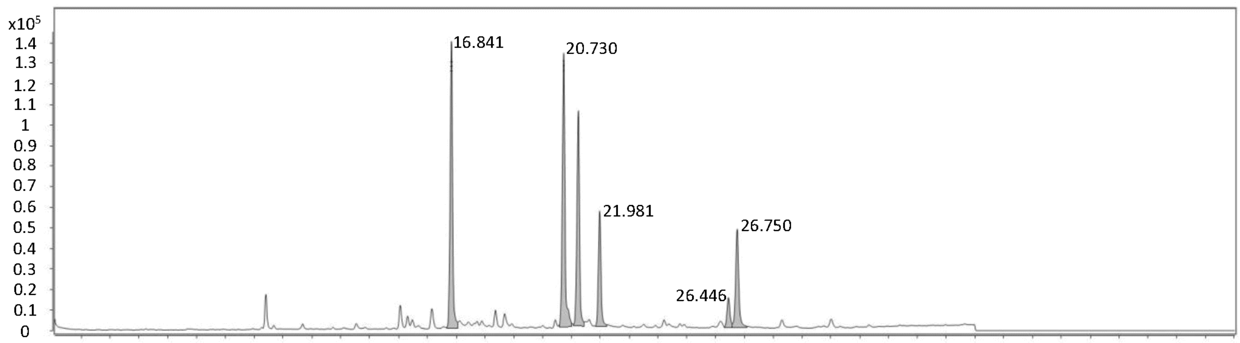

x10^5
16.841
20.730
21.981
26.446
26.750
Counts vs.Collection Time(min)


Fig. 8

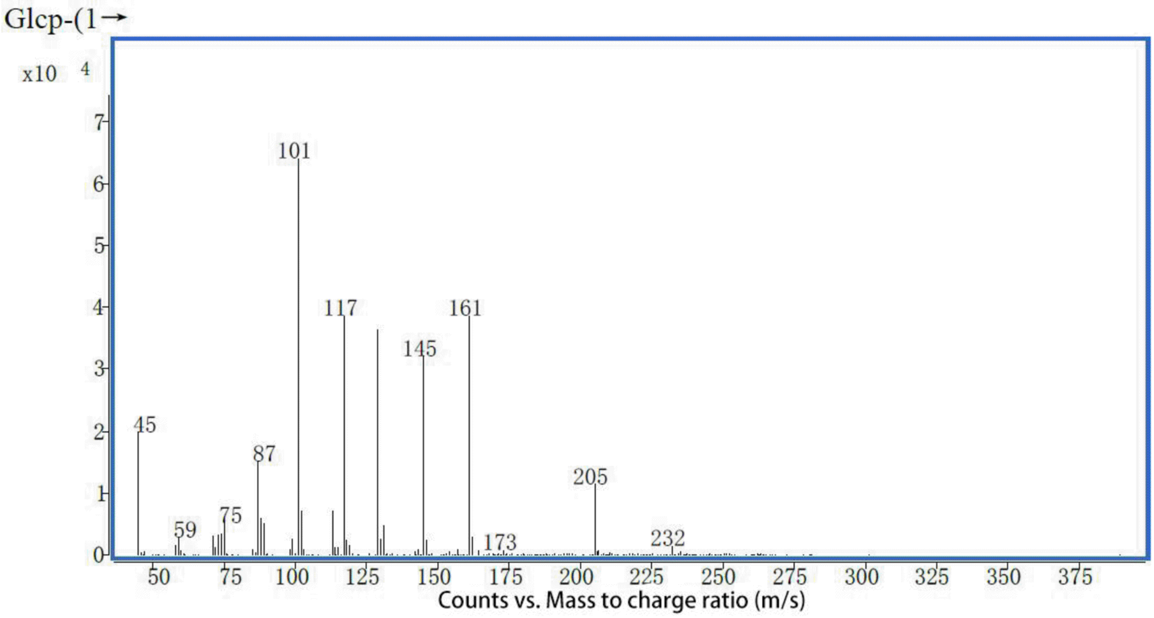
Glcp-(1→
x10 4
7
6
5
4
3
2
1
0
101
117
161
145
45
87
205
75
59
173
232
50
75
100
125
150
175
200
225
250
275
300
325
350
375
Counts vs. Mass to charge ratio (m/s)

Fig. 9

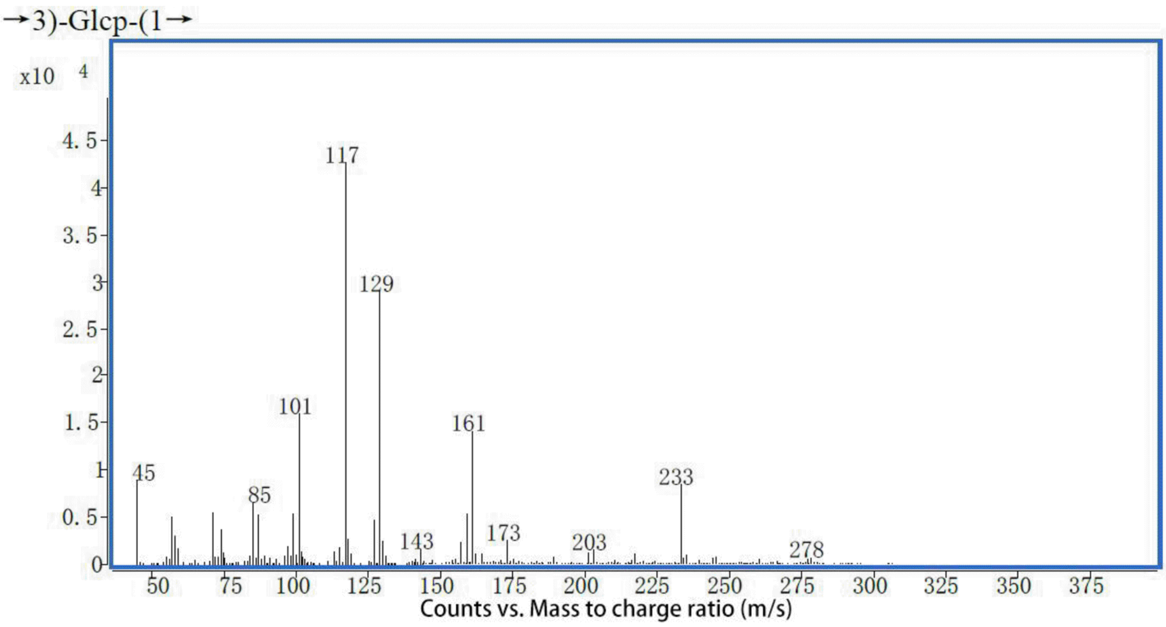
→3)-Glcp-(1→
x10 4
4. 5
4
3. 5
3
2. 5
2
1. 5
1
0. 5
0
117
129
101
161
45
233
85
143
173
203
278
50
75
100
125
150
175
200
225
250
275
300
325
350
375
Counts vs. Mass to charge ratio (m/s)

Fig. 10

→4)-Glcp-(1→

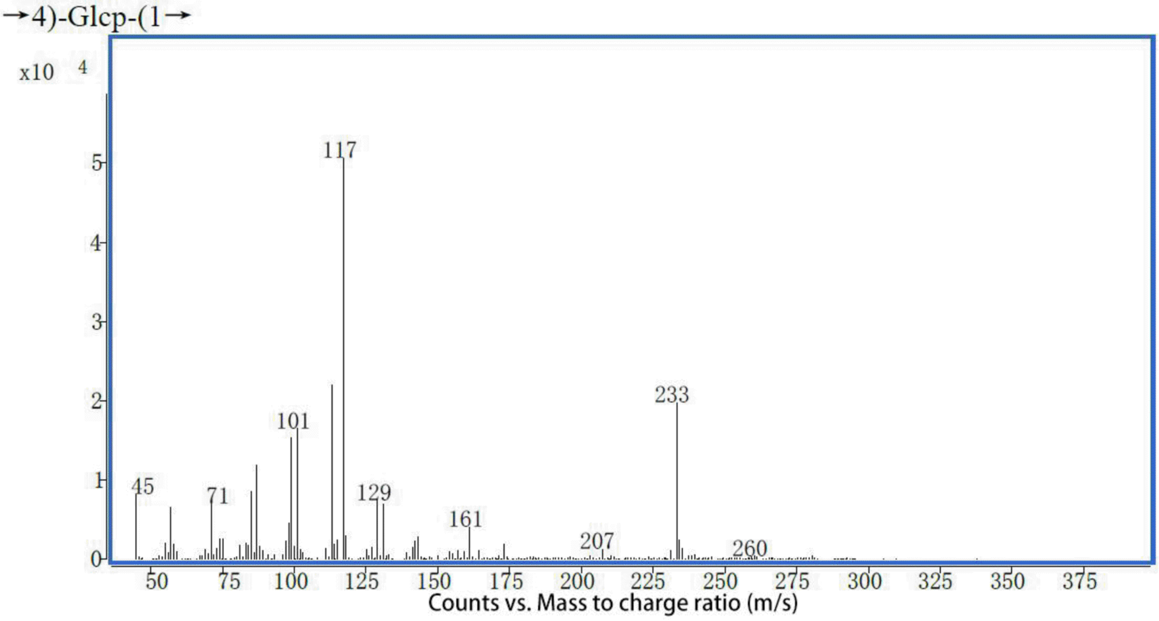


Fig. 11

→6-Glcp-(1→

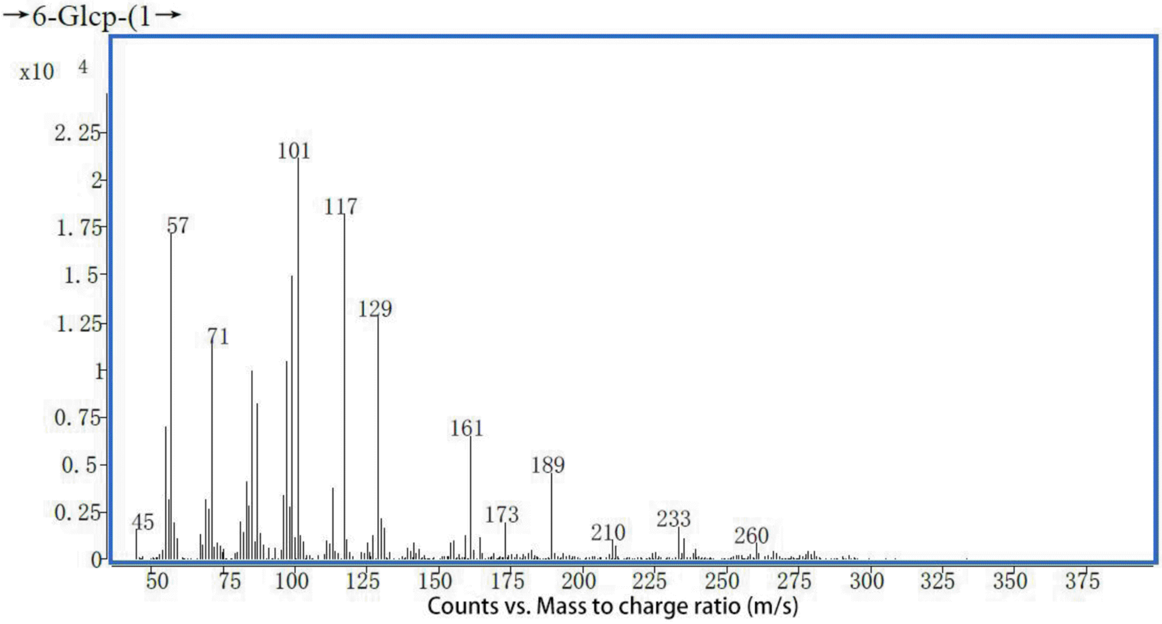


Fig. 12

→4,6)-Glcp-(1→

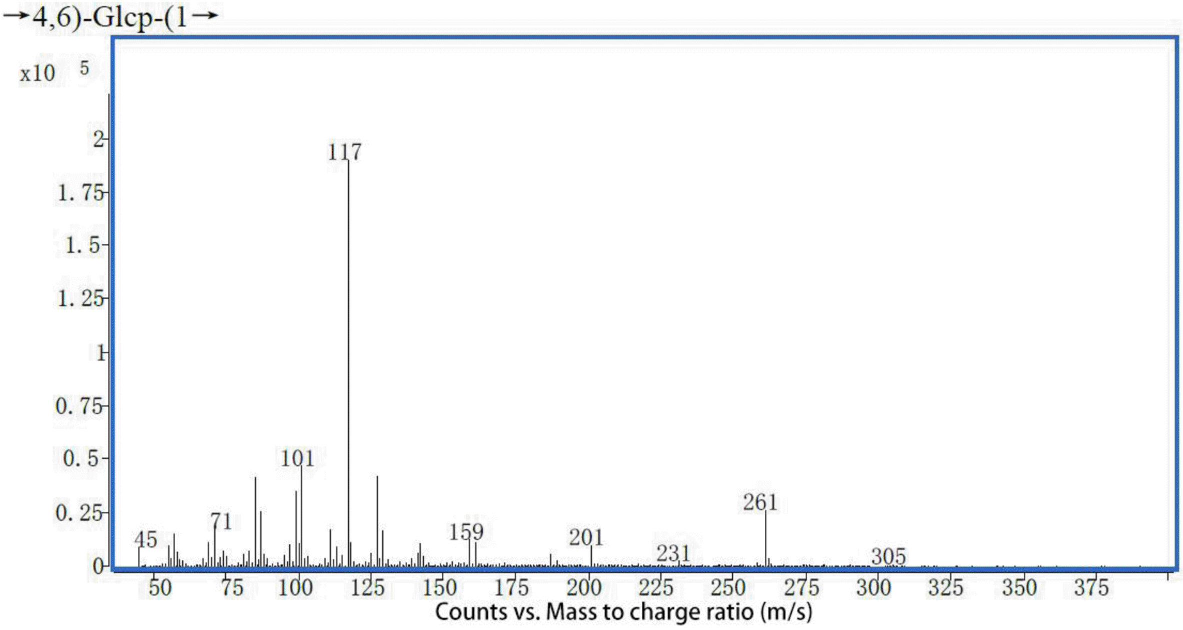


Fig. 13

→3,6)-Glcp-(1→

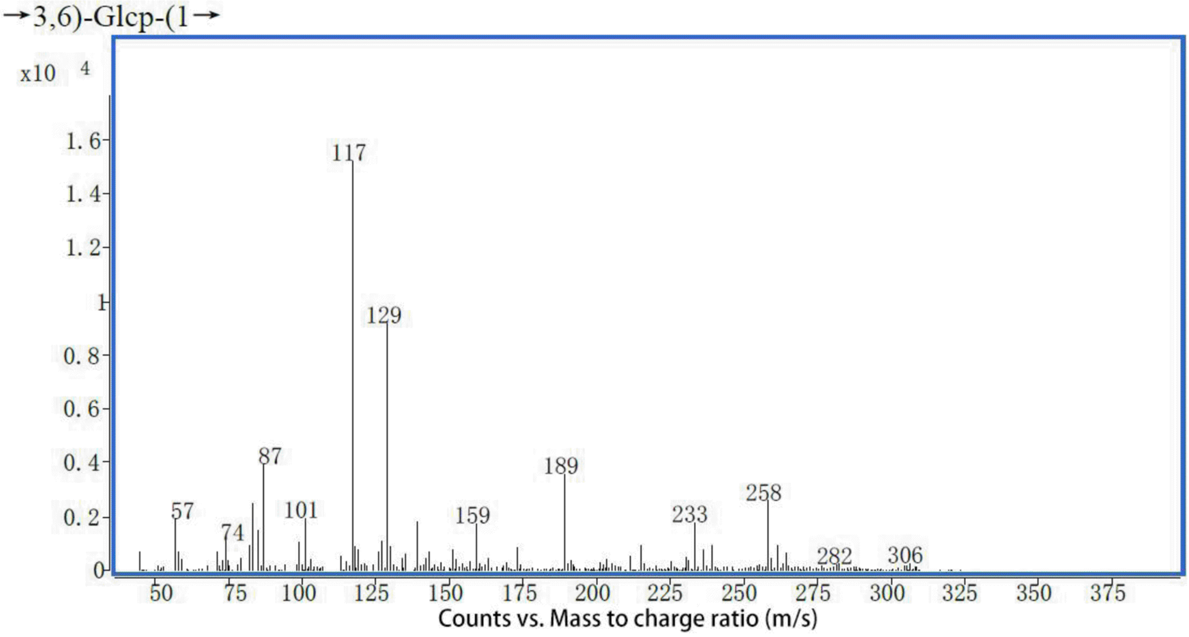


Fig. 14

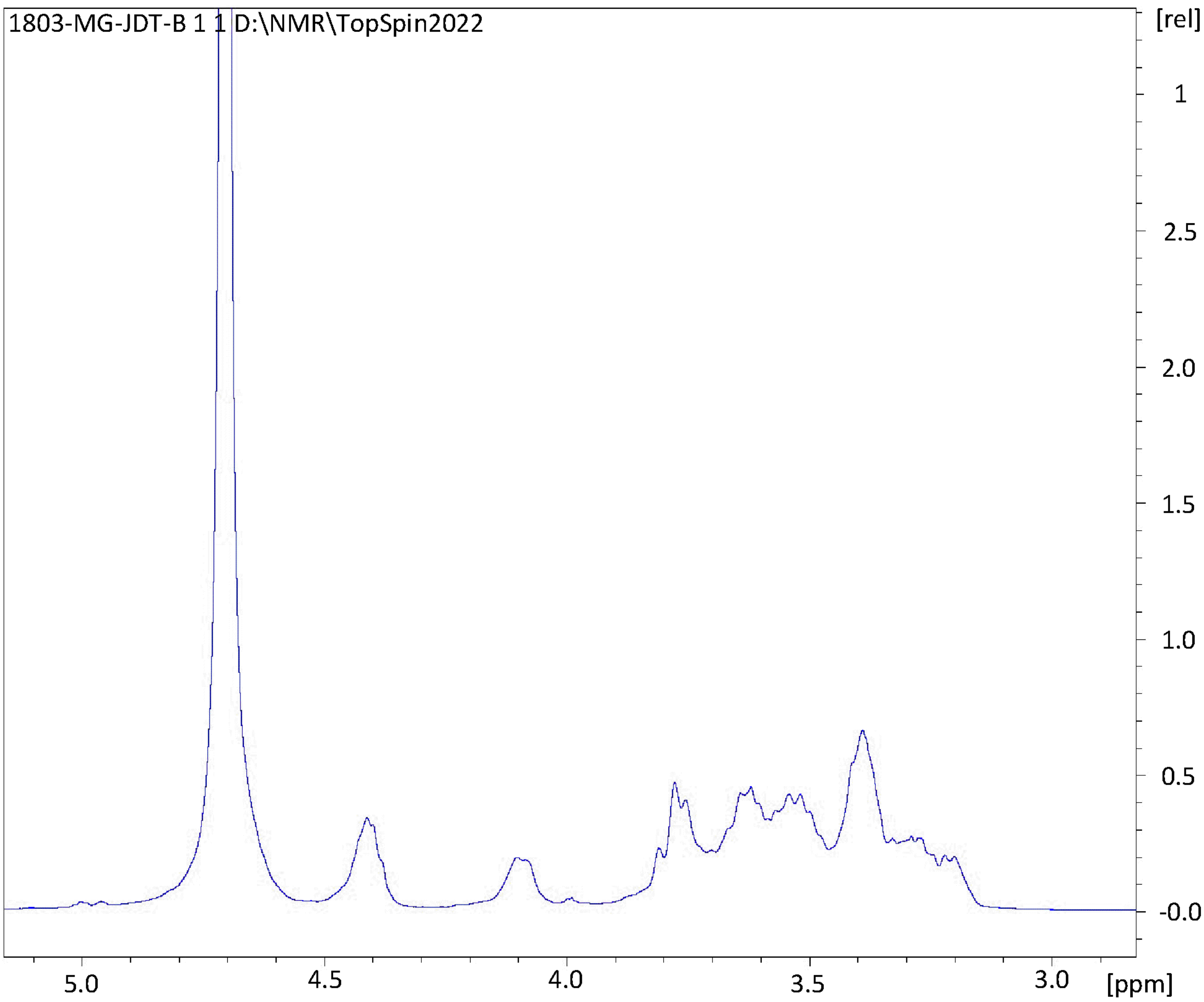
1803-MG-JDT-B 1 1 D:\NMR\TopSpin2022
[rel]
1
2.5
2.0
1.5
1.0
0.5
-0.0
5.0
4.5
4.0
3.5
3.0
[ppm]

Fig. 15

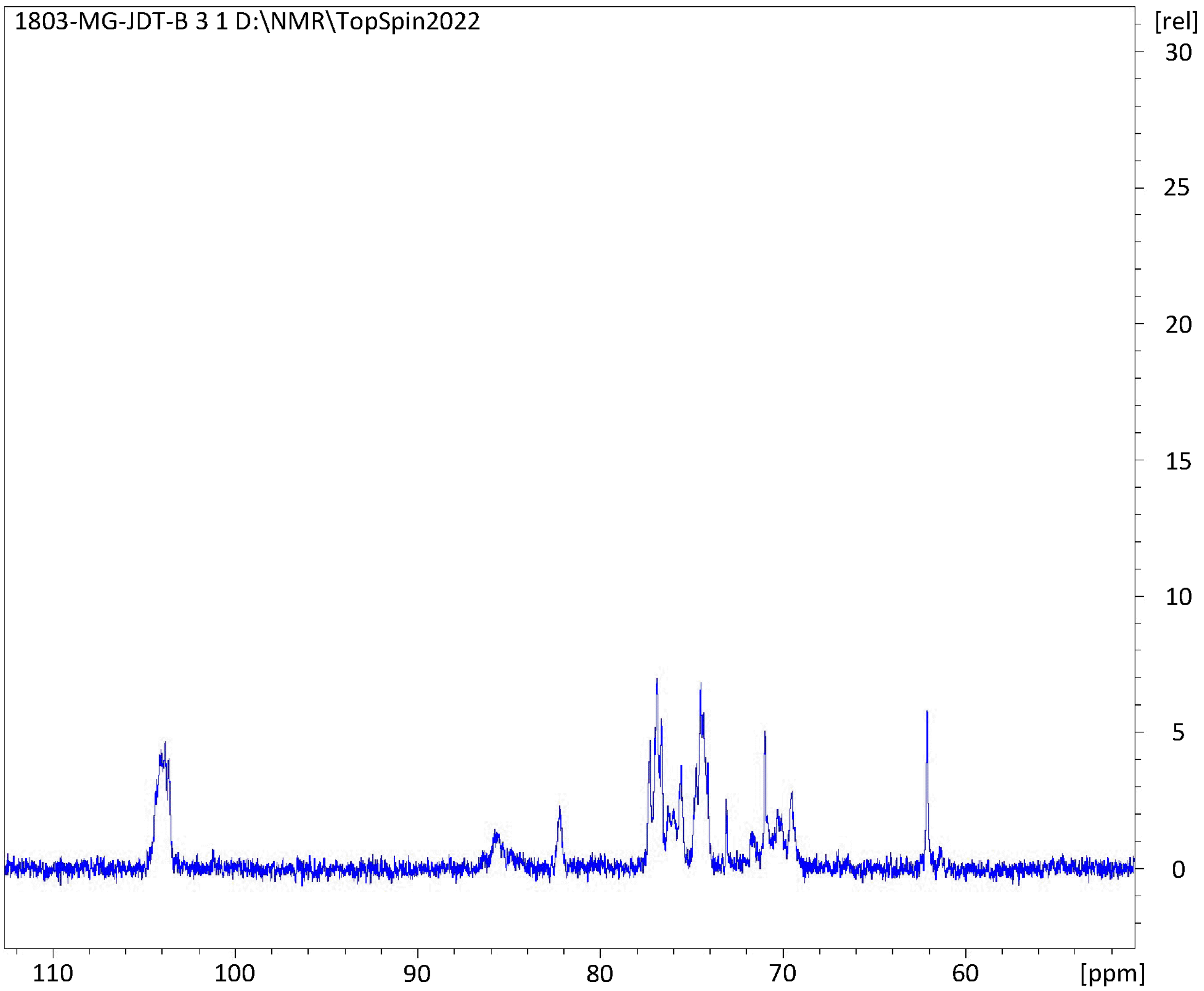
1803-MG-JDT-B 3 1 D:\NMR\TopSpin2022
[rel]
30
25
20
15
10
5
0
110
100
90
80
70
60
[ppm]

Fig. 16

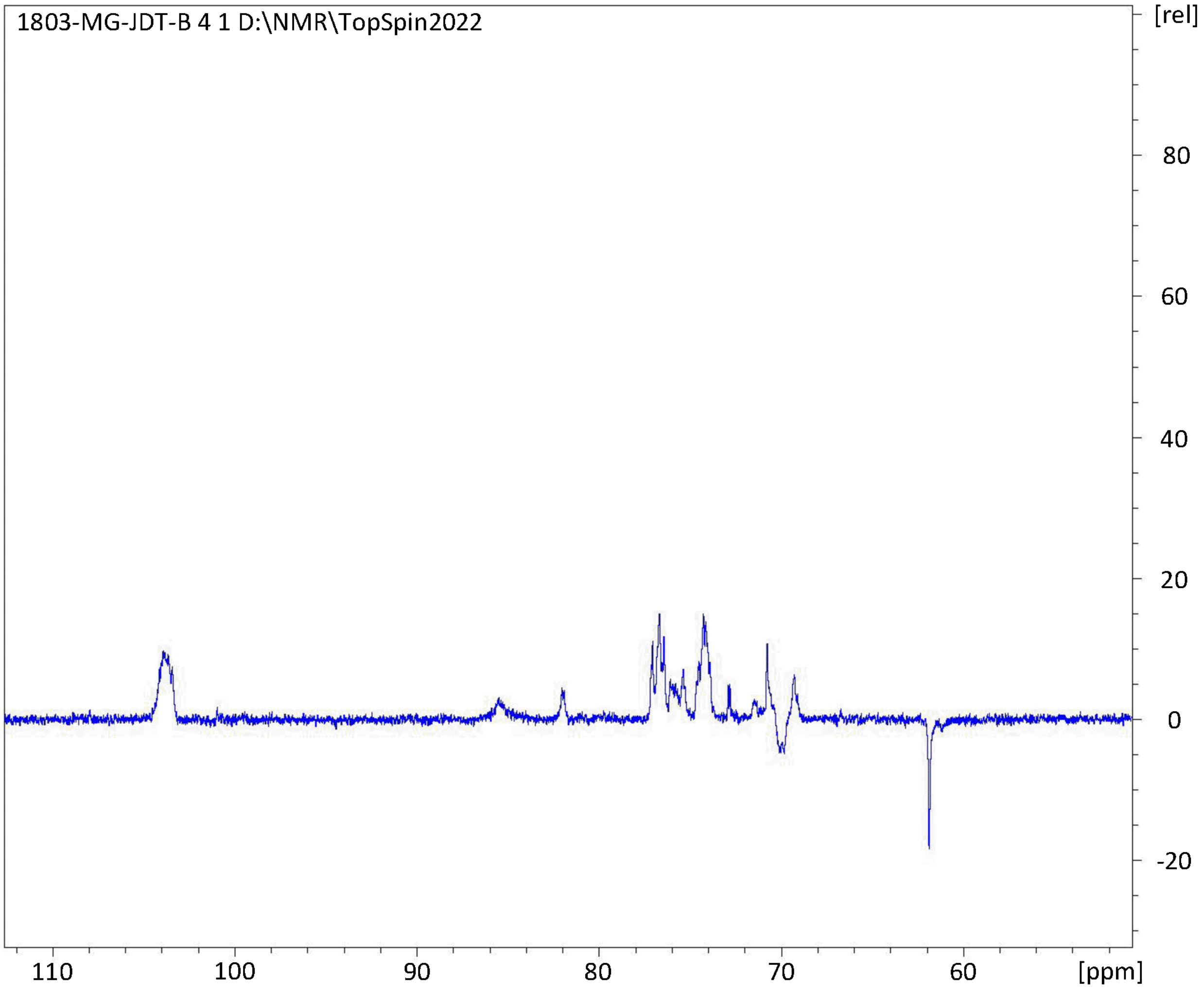
1803-MG-JDT-B 4 1 D:\NMR\TopSpin2022
[rel]
80
60
40
20
0
-20
110
100
90
80
70
60
[ppm]

Fig. 17

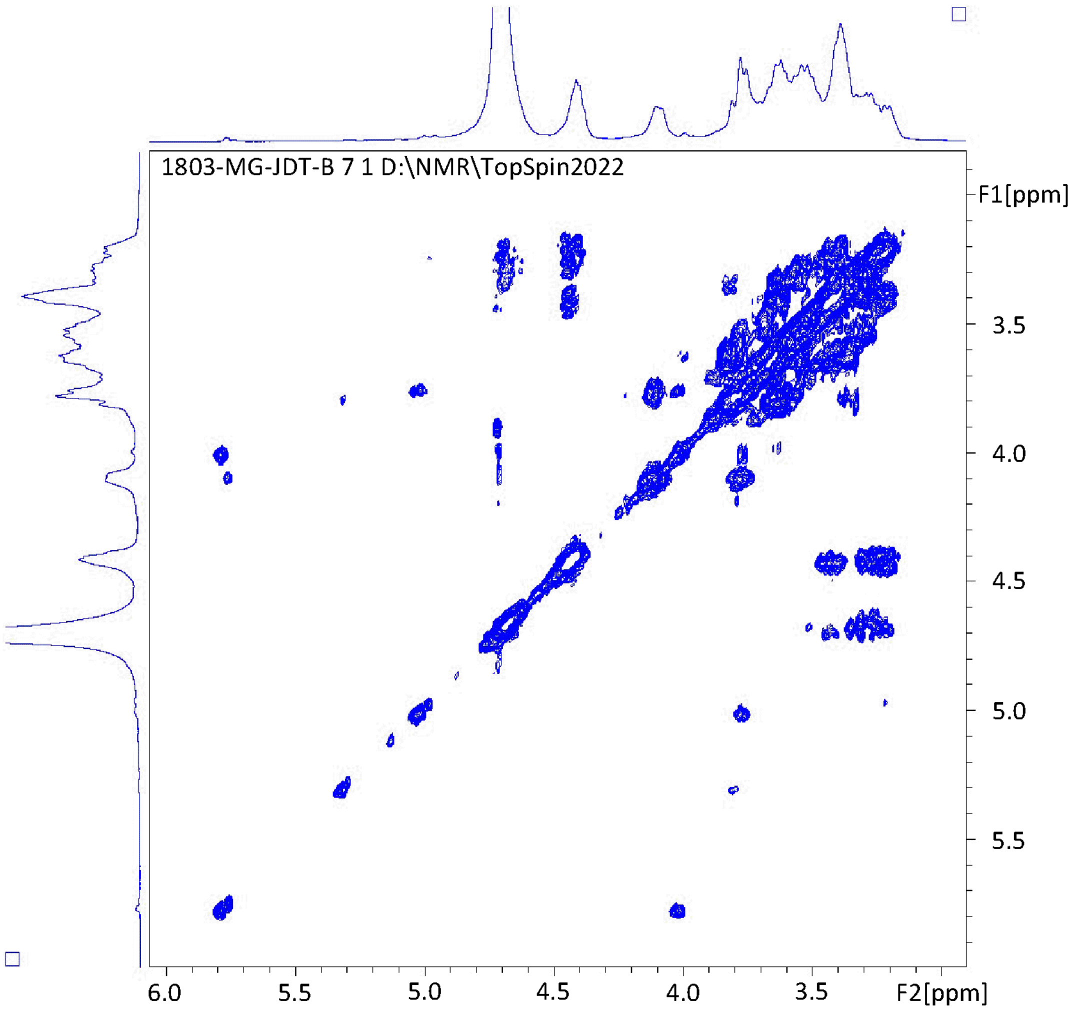
1803-MG-JDT-B 7 1 D:\NMR\TopSpin2022
F1[ppm]
3.5
4.0
4.5
5.0
5.5
6.0
5.5
5.0
4.5
4.0
3.5
F2[ppm]

Fig. 18

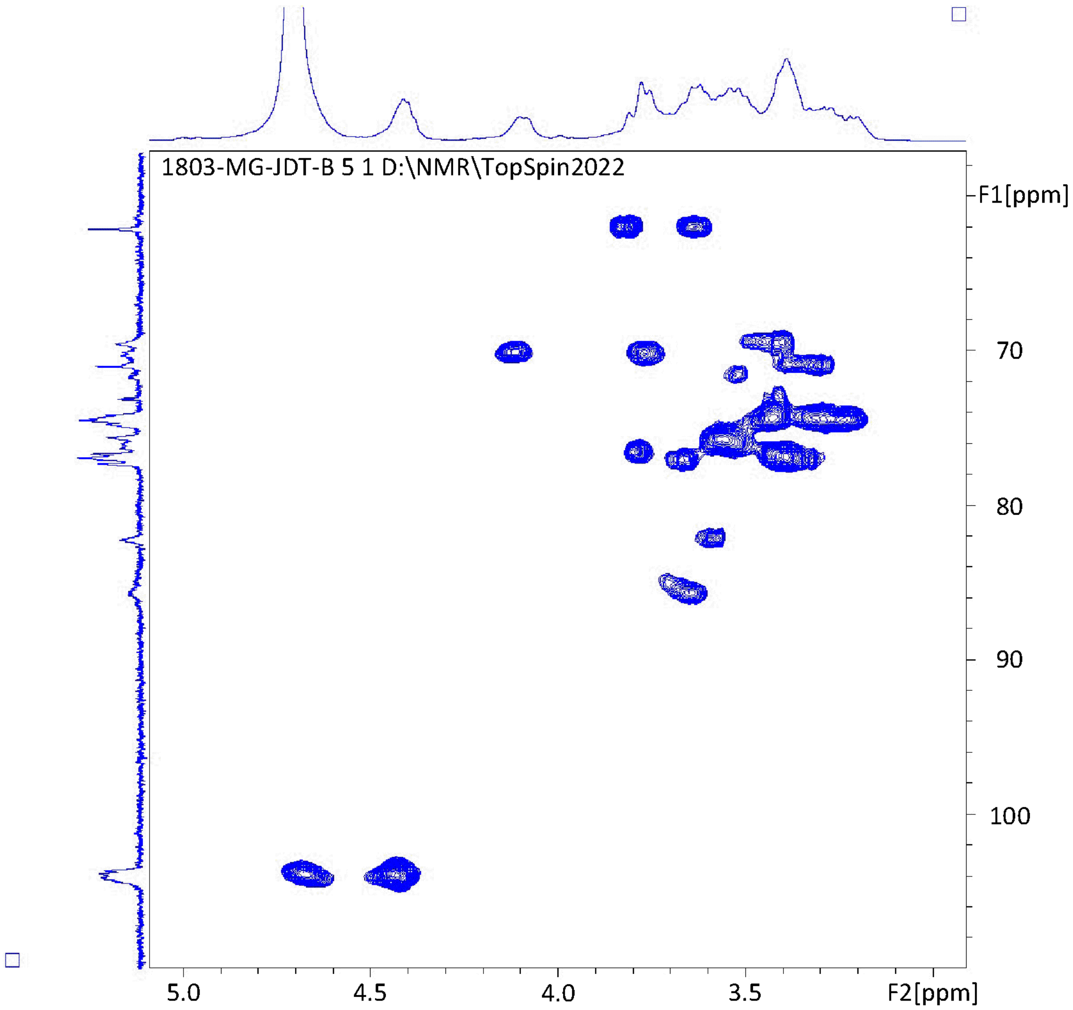
1803-MG-JDT-B 5 1 D:\NMR\TopSpin2022
F1[ppm]
70
80
90
100
5.0
4.5
4.0
3.5
F2[ppm]

Fig. 19

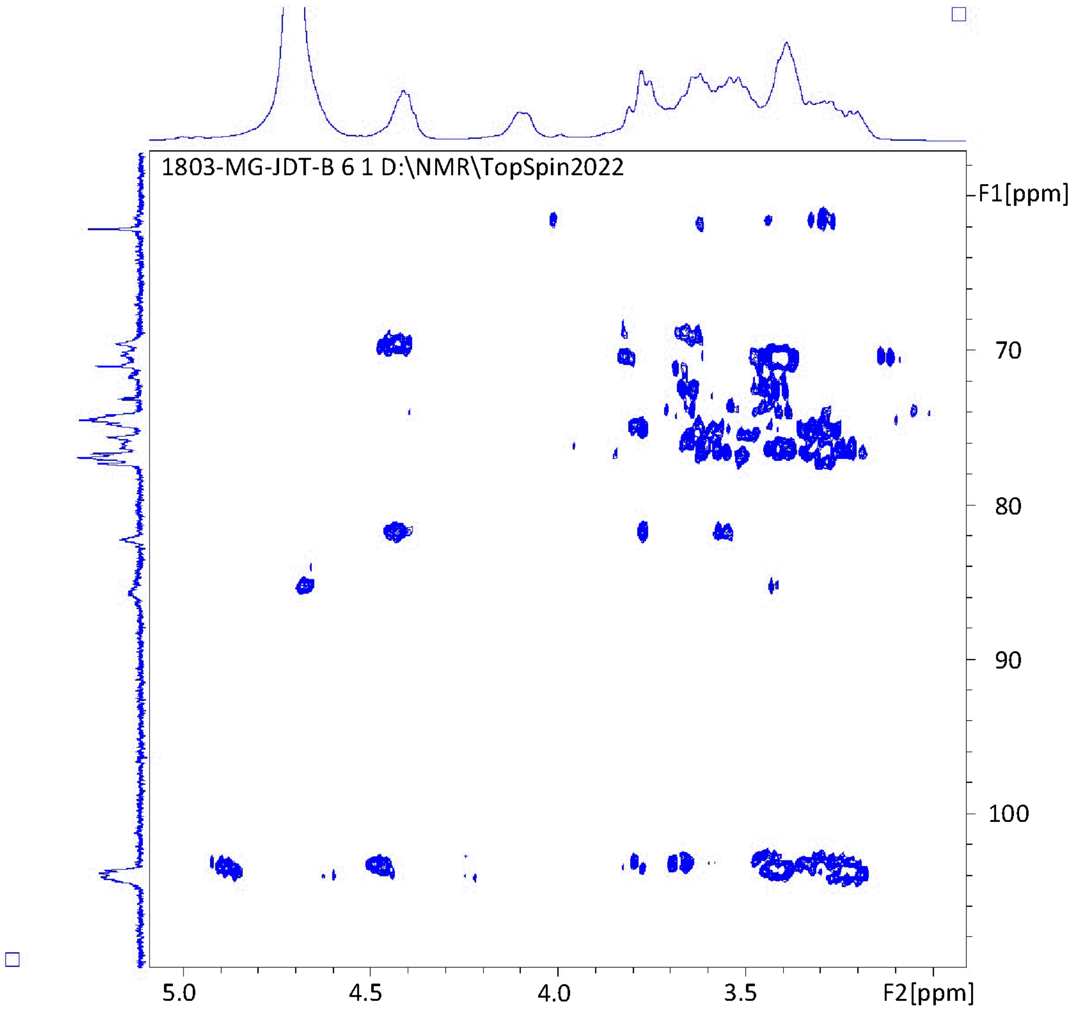
1803-MG-JDT-B 6 1 D:\NMR\TopSpin2022
F1[ppm]
70
80
90
100
5.0
4.5
4.0
3.5
F2[ppm]

Fig. 20

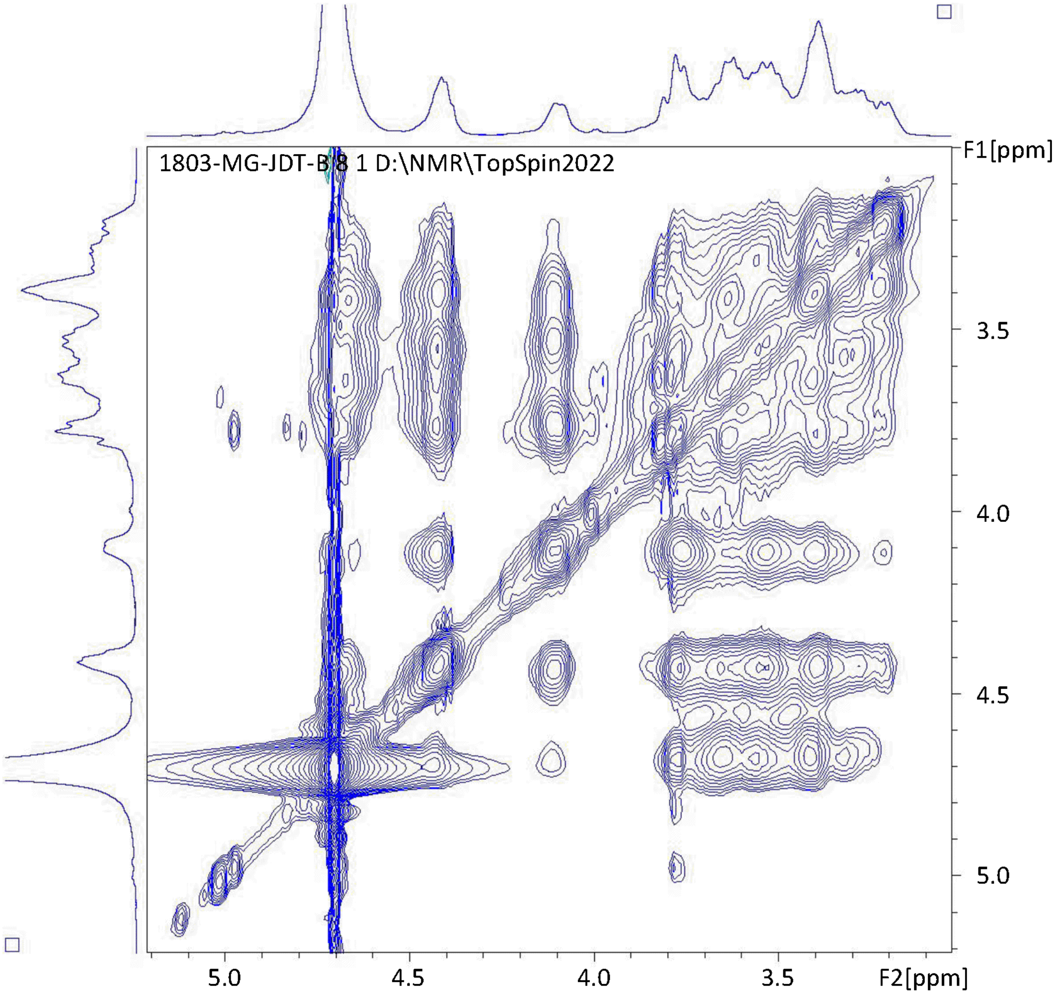
1803-MG-JDT-B 8 1 D:\NMR\TopSpin2022
F1[ppm]
3.5
4.0
4.5
5.0
5.0
4.5
4.0
3.5
F2[ppm]

Fig. 21

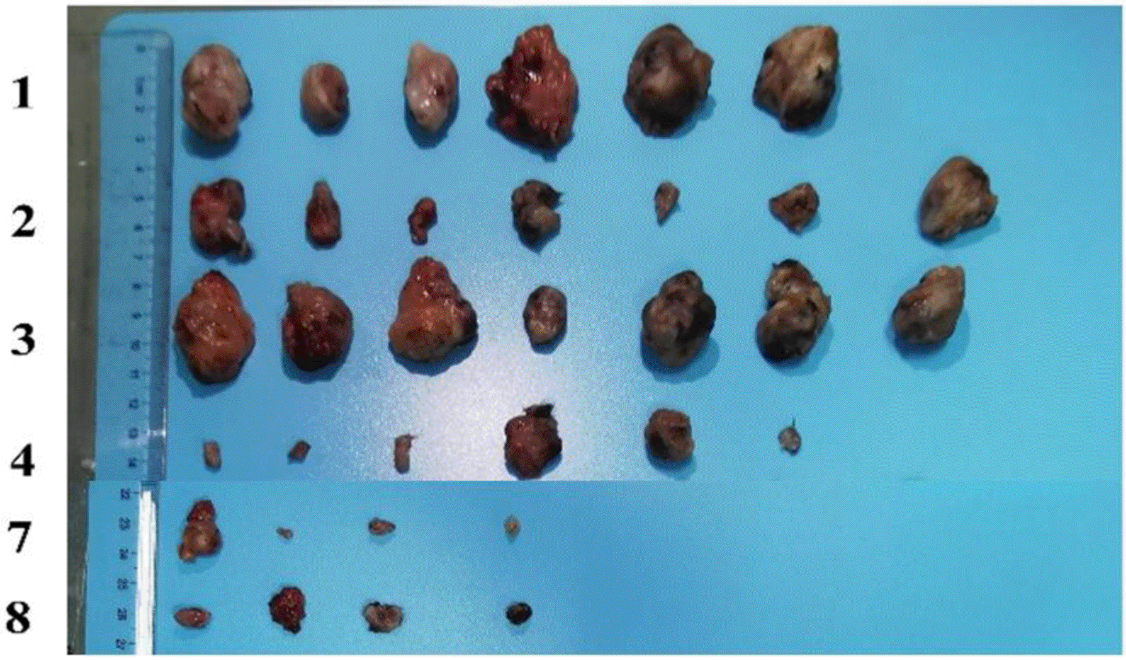
1
2
3
4
7
8

Fig. 22

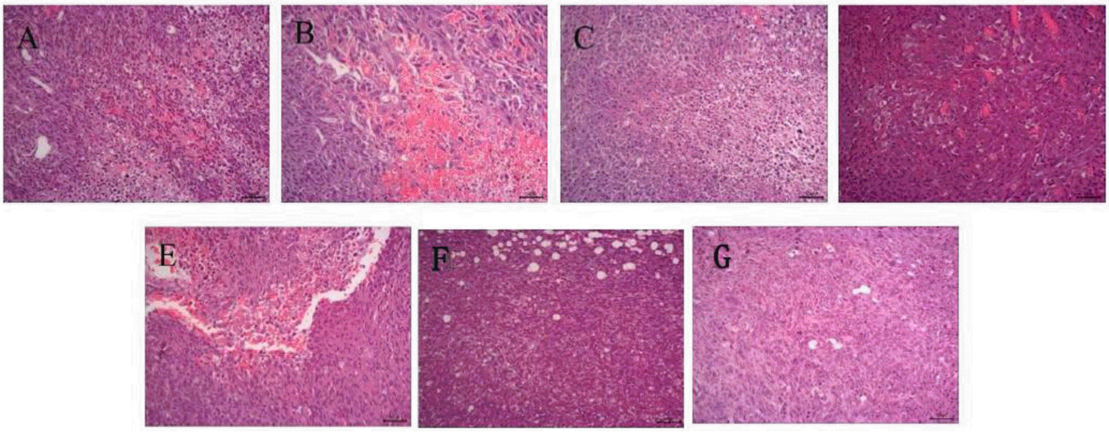


Fig. 23

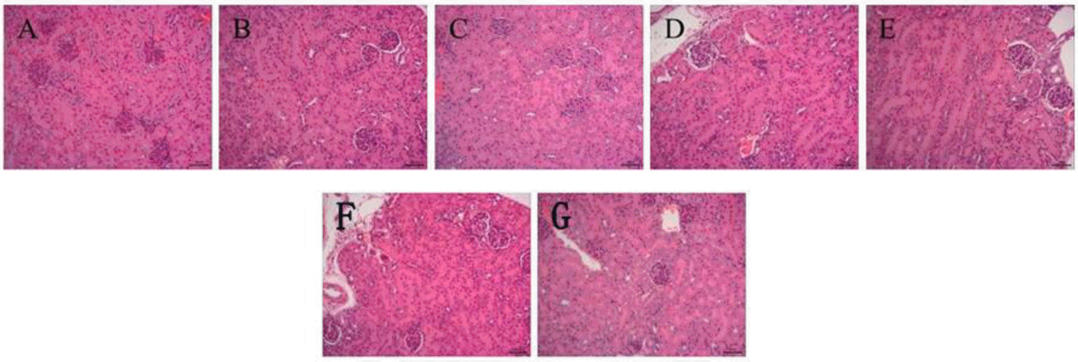


Fig. 24

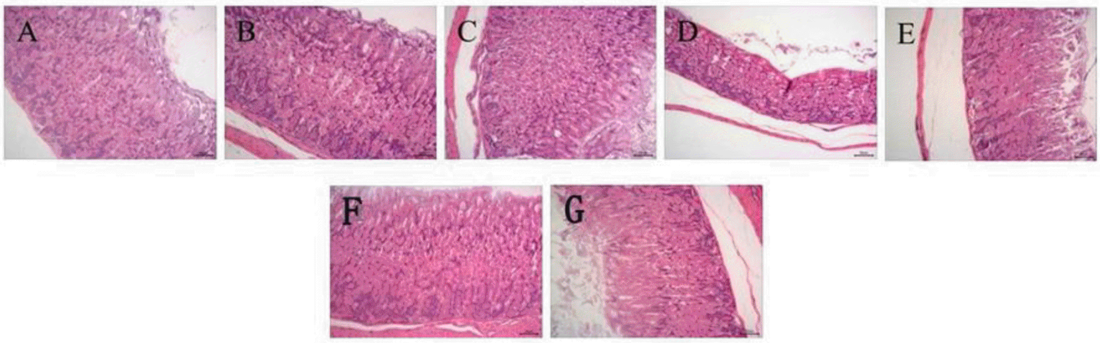


Fig. 25

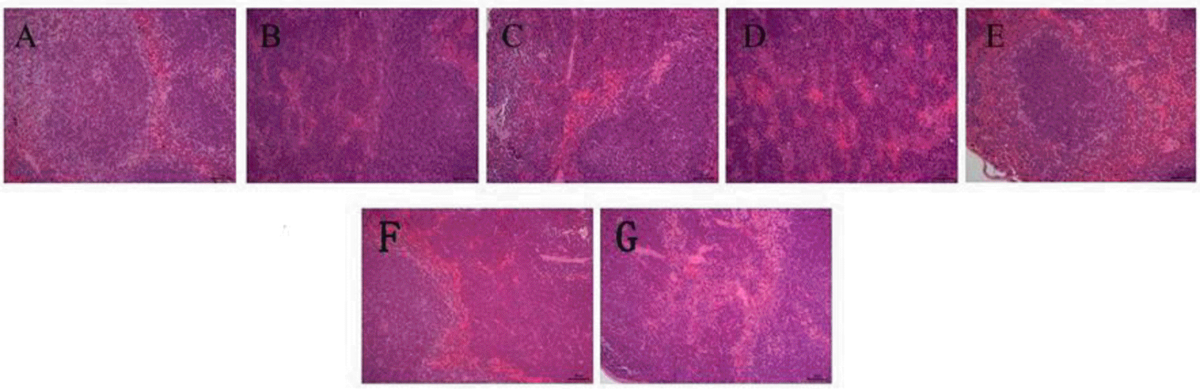


Fig. 26

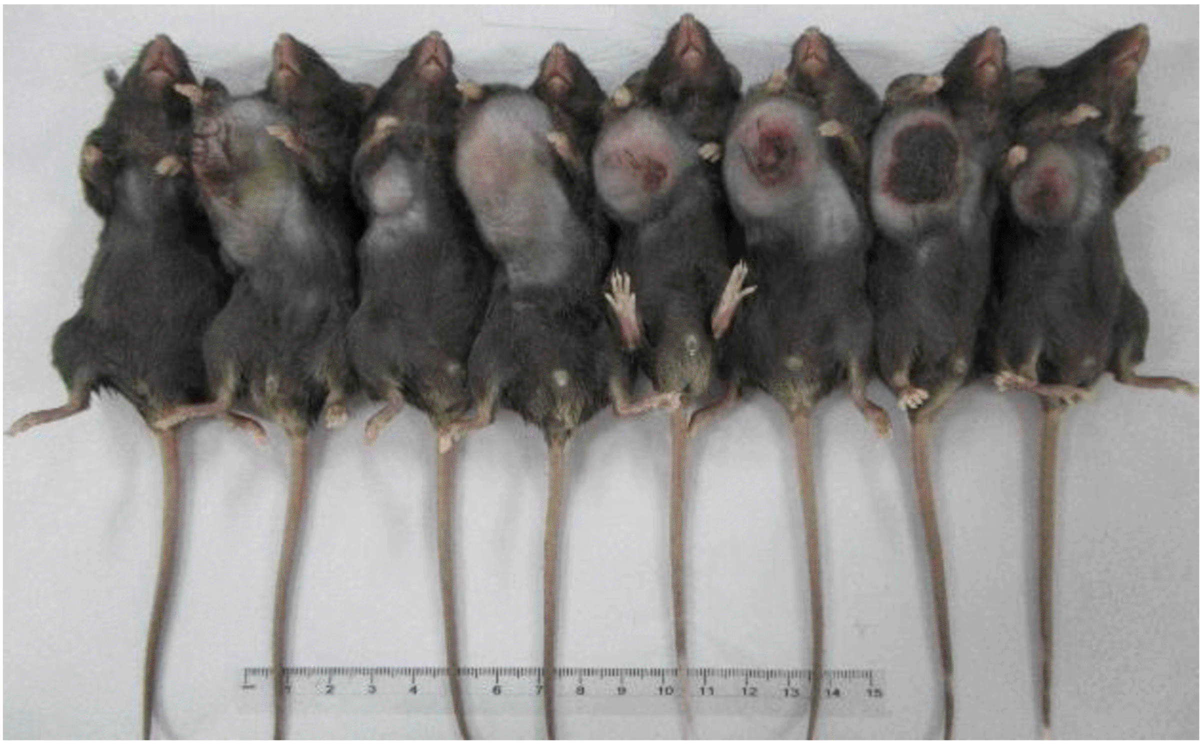

Fig. 27

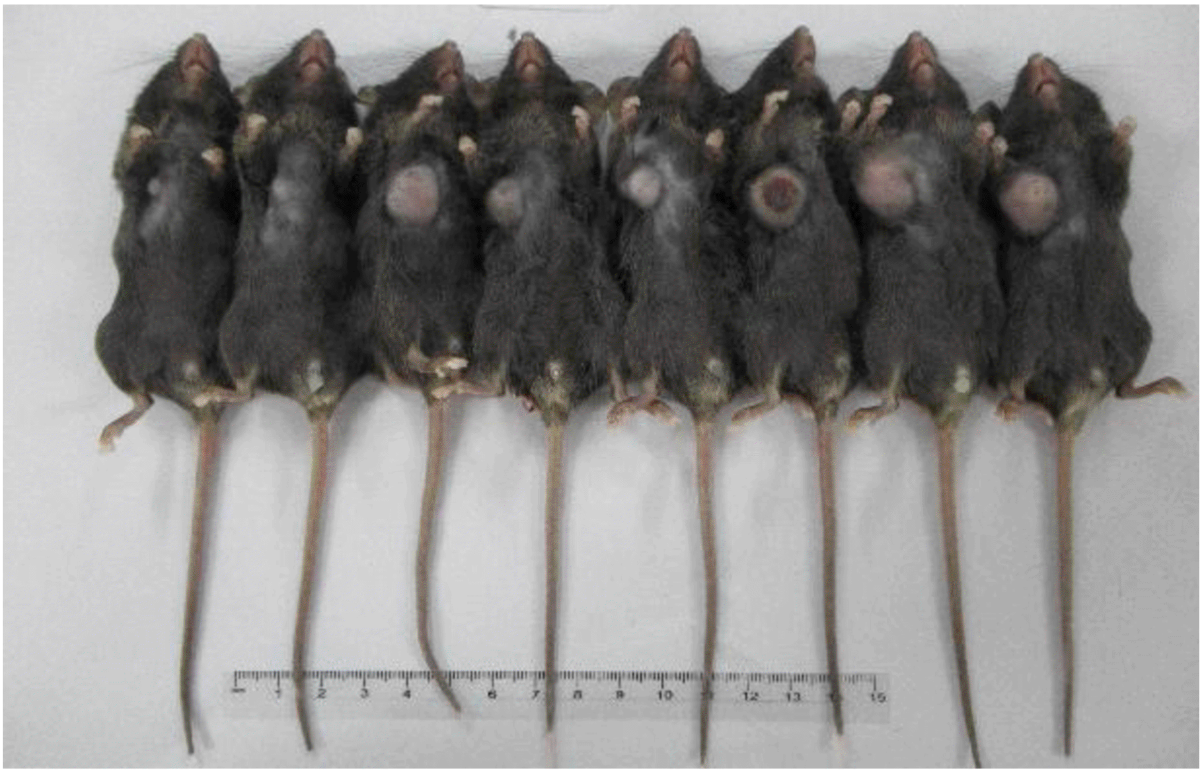

Fig. 28

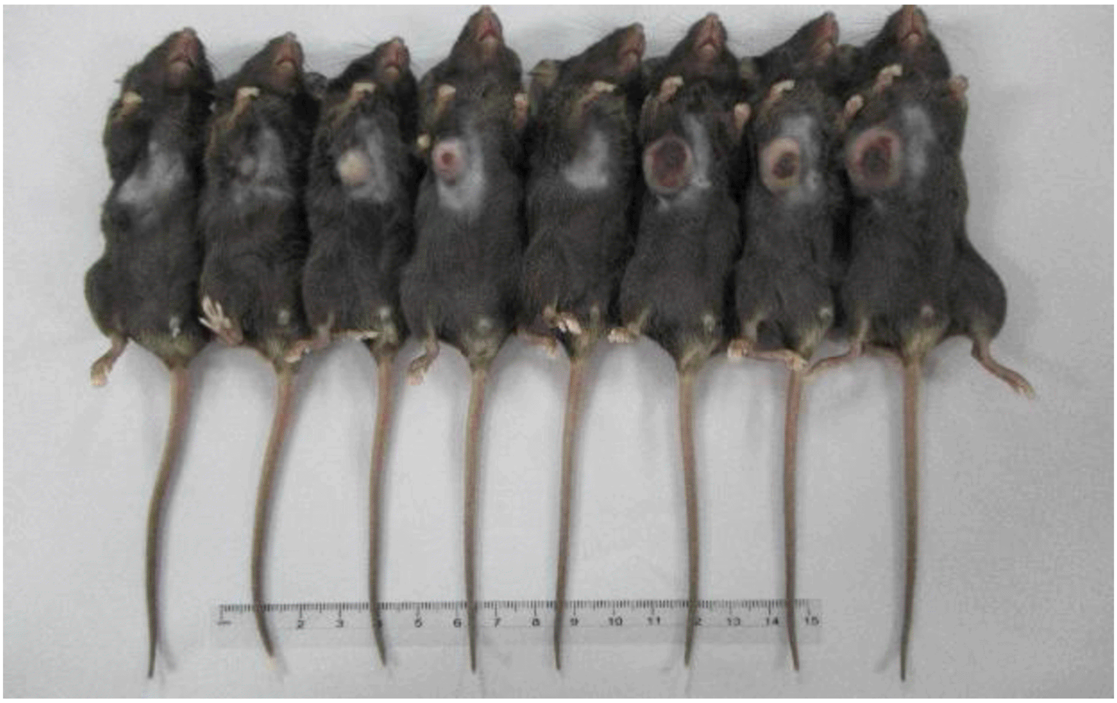

Fig. 29

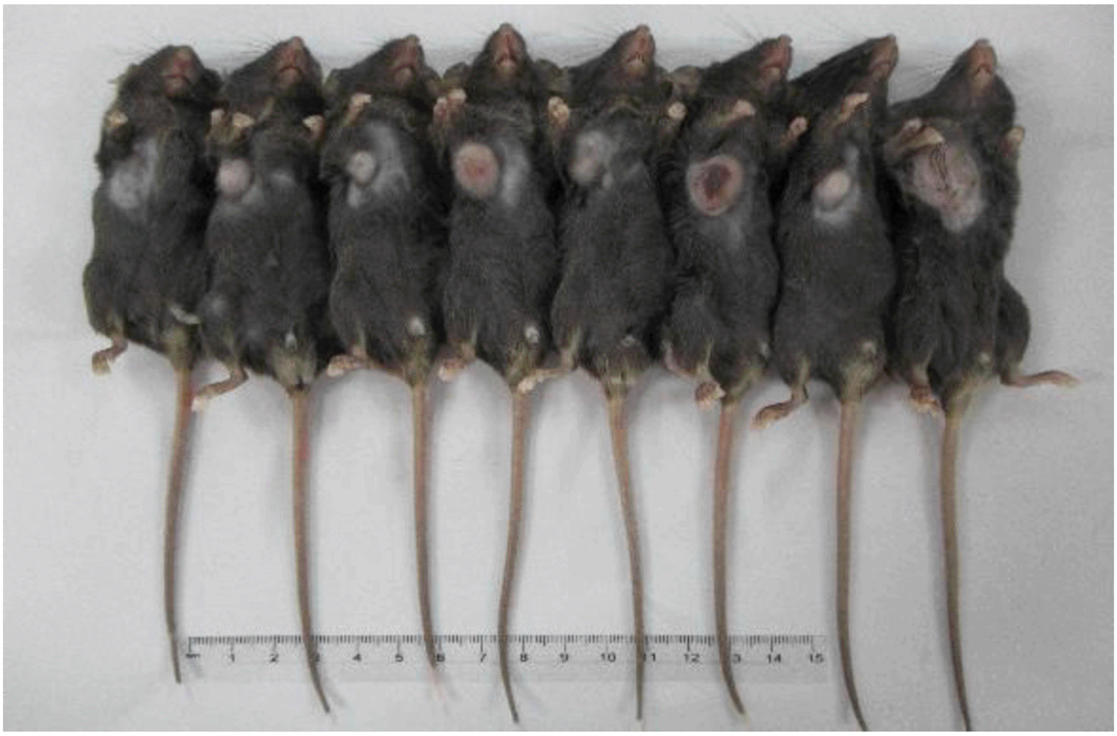

Fig. 30

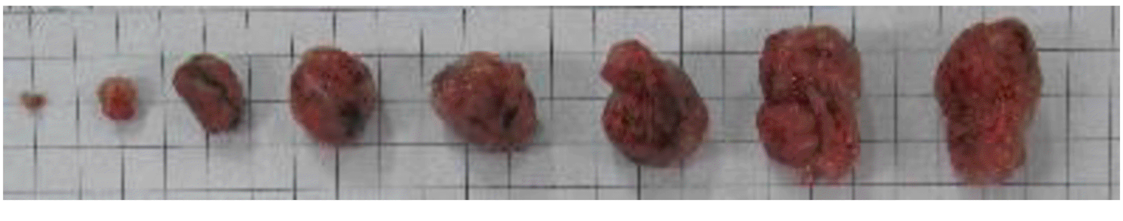

Fig. 31

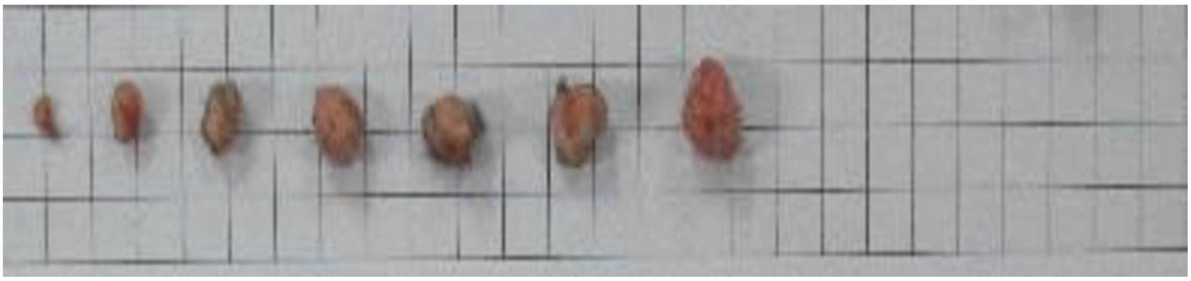

Fig. 32

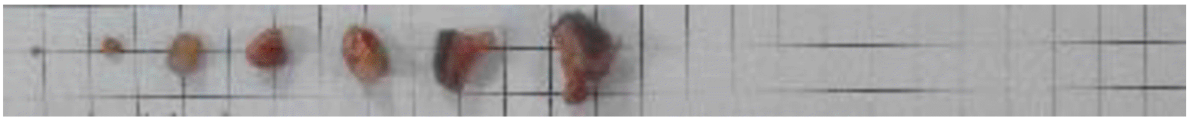

Fig. 33

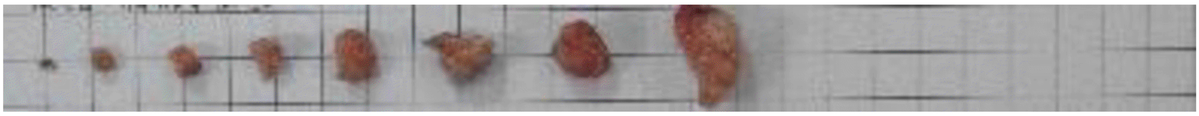

Fig. 34

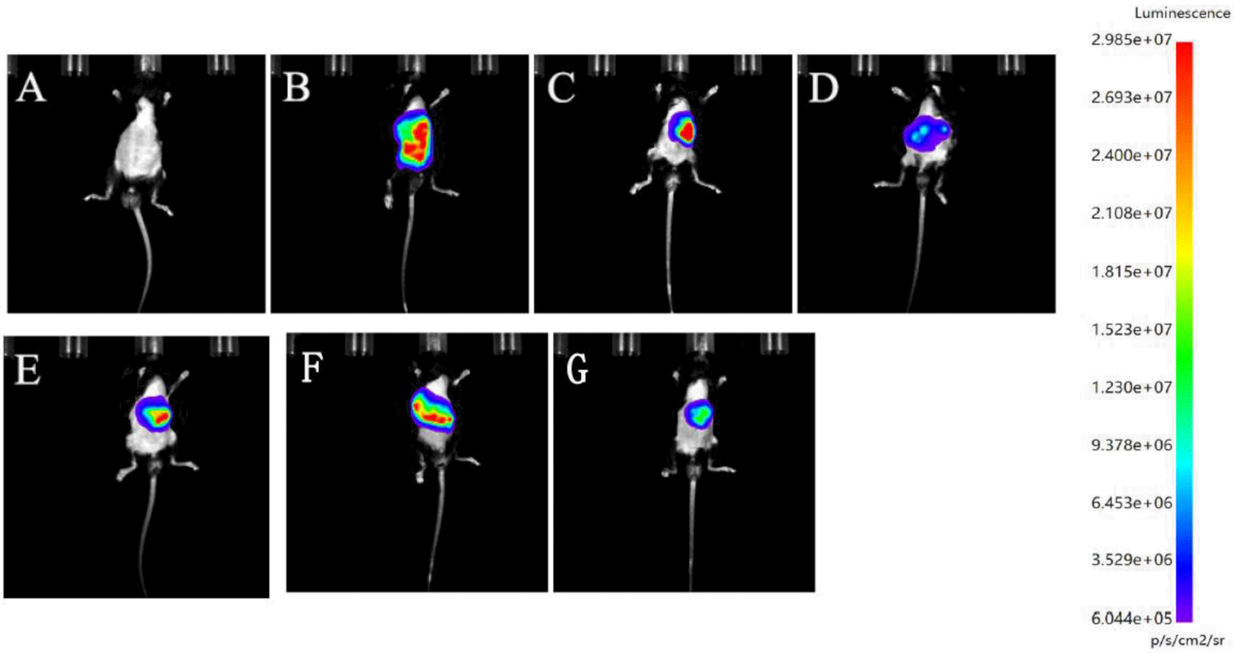


Fig. 35

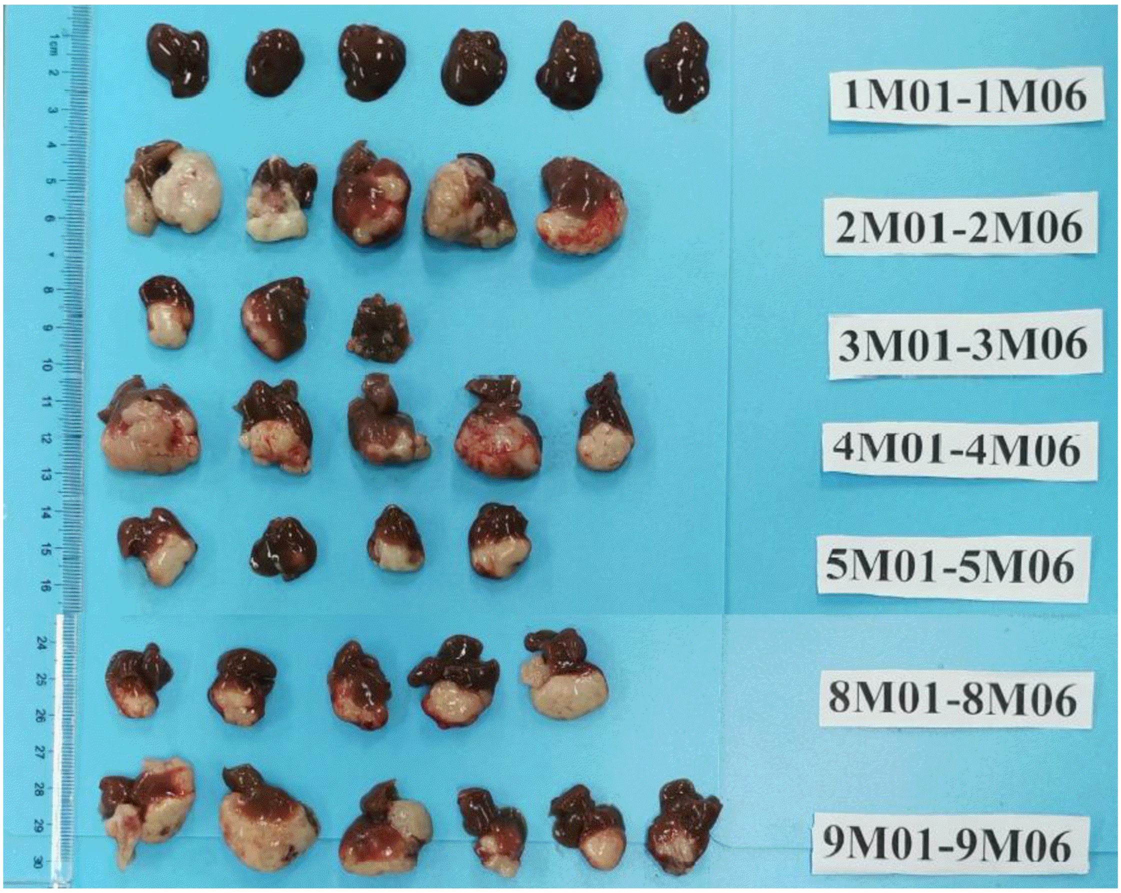


Fig. 36

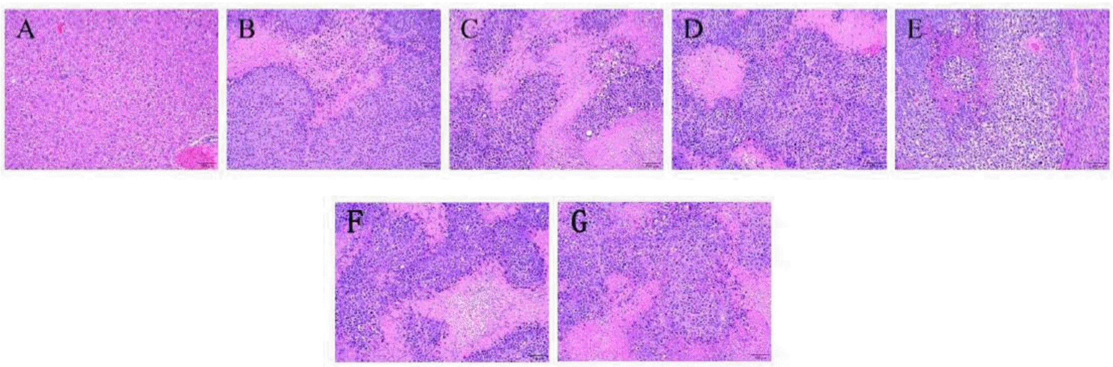


Fig. 37

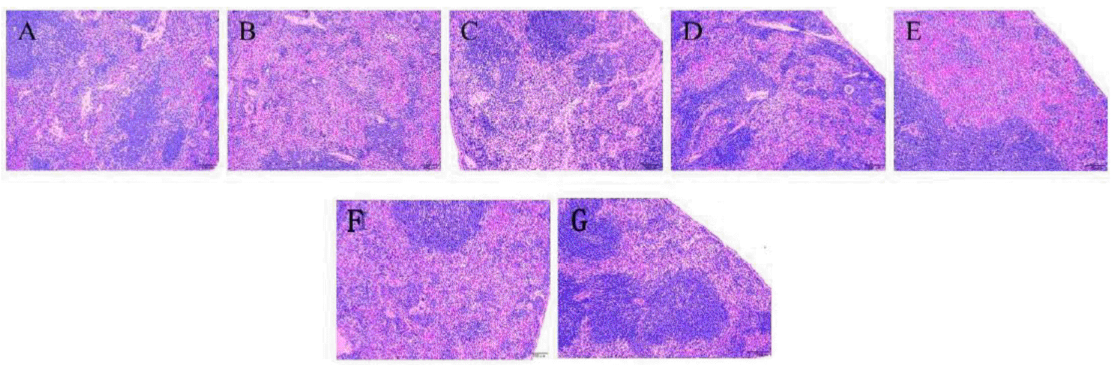


Fig. 38

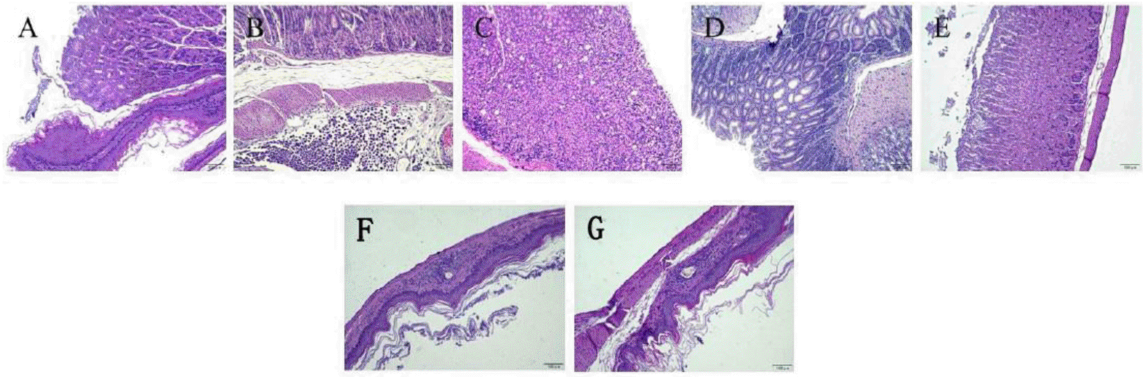

Fig. 39

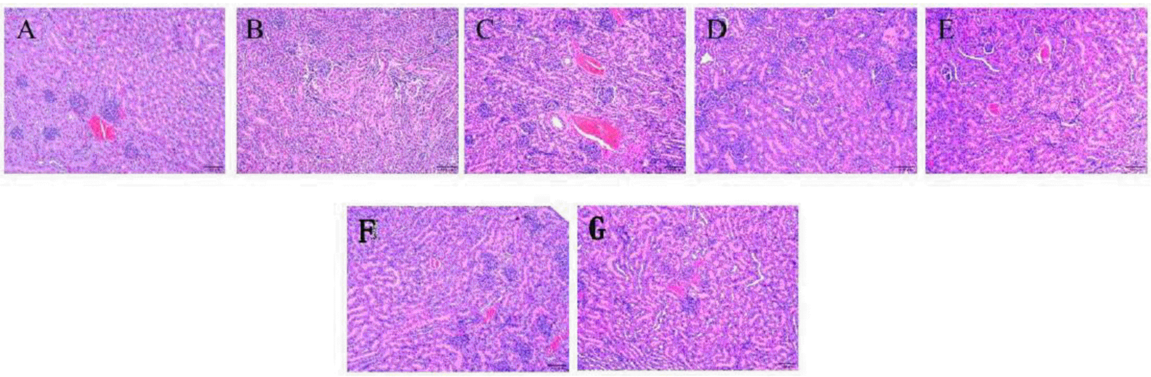

Fig. 40

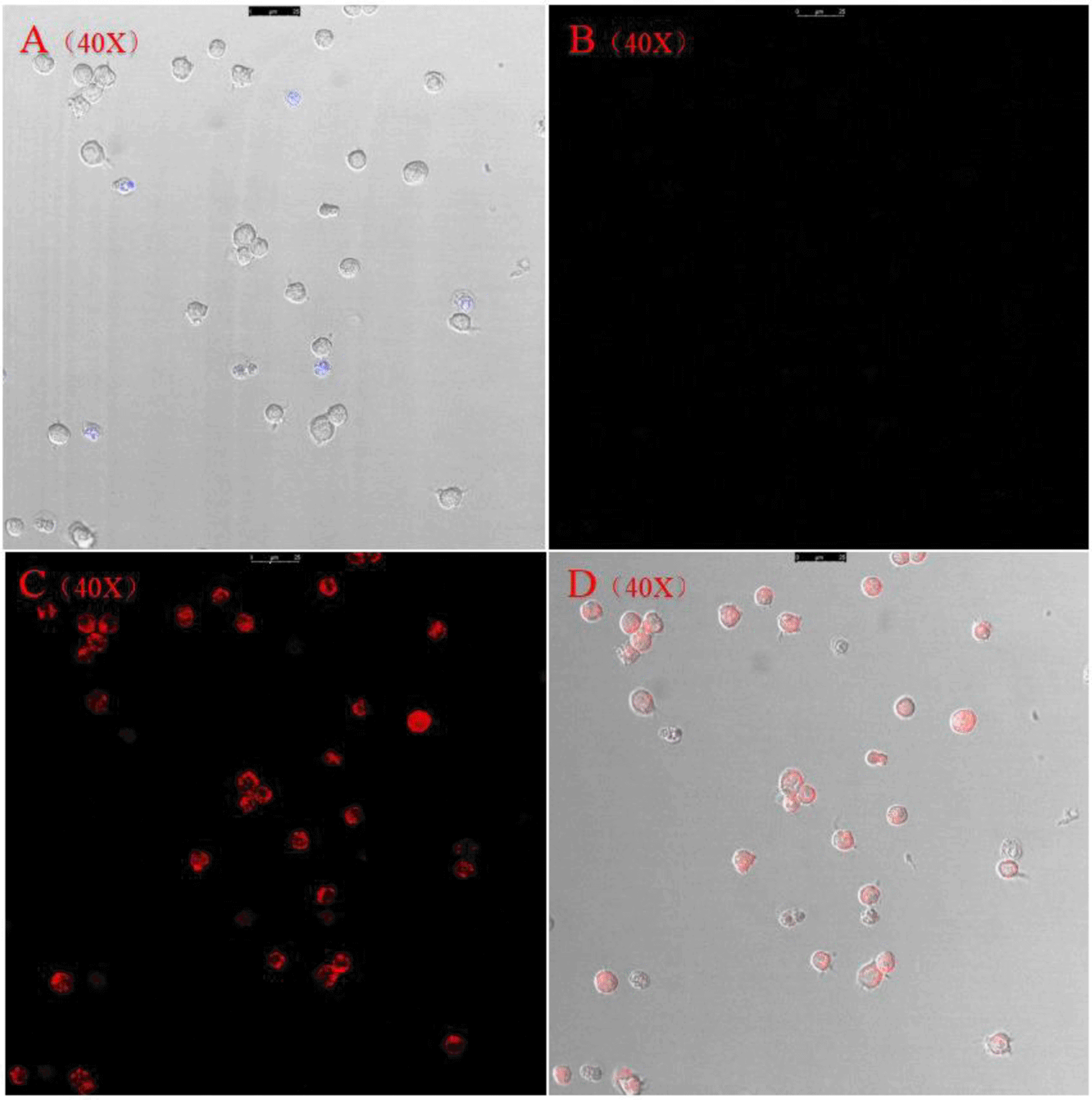


Fig. 41

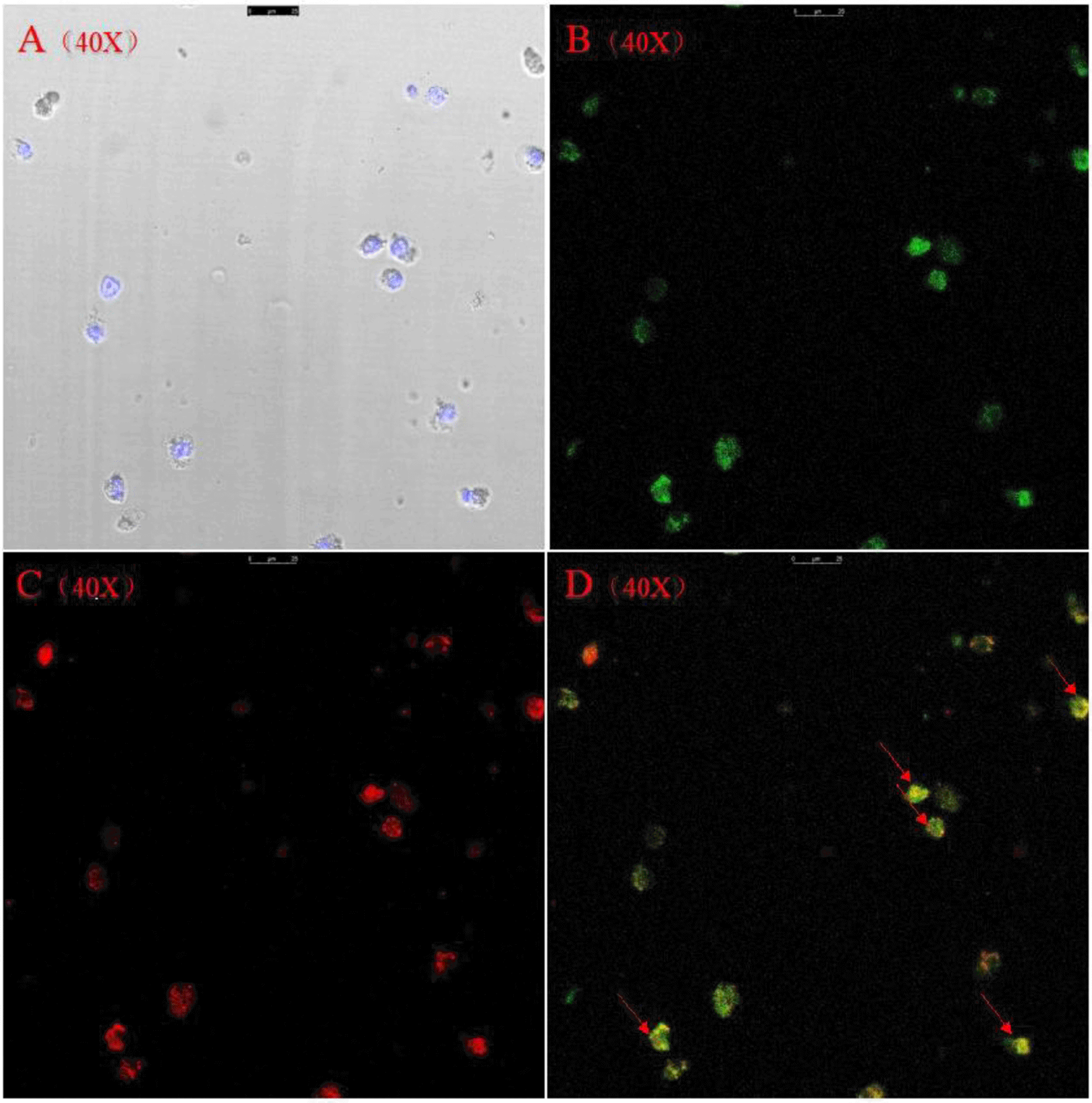


Fig. 42

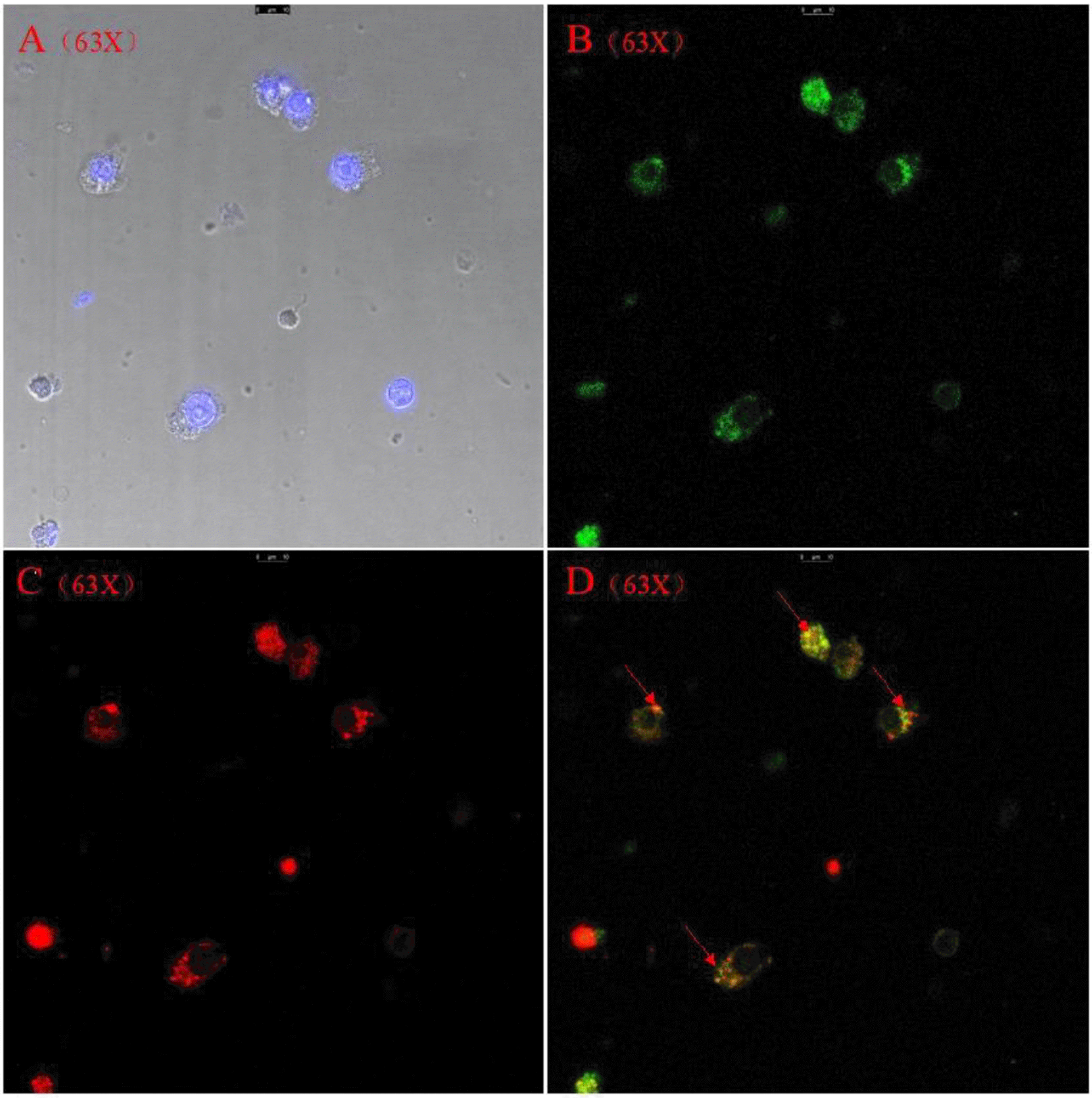


Fig. 43

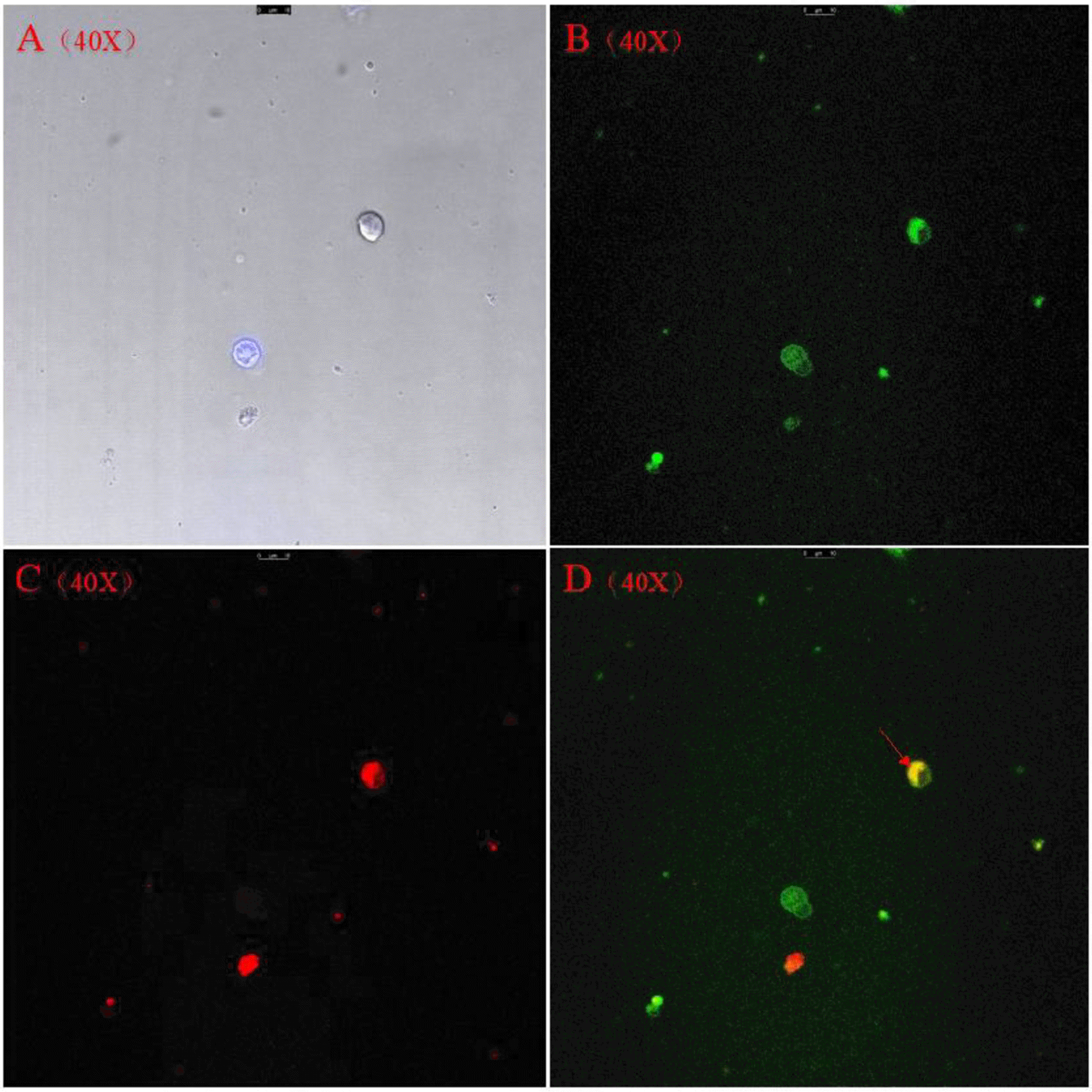


Fig. 44

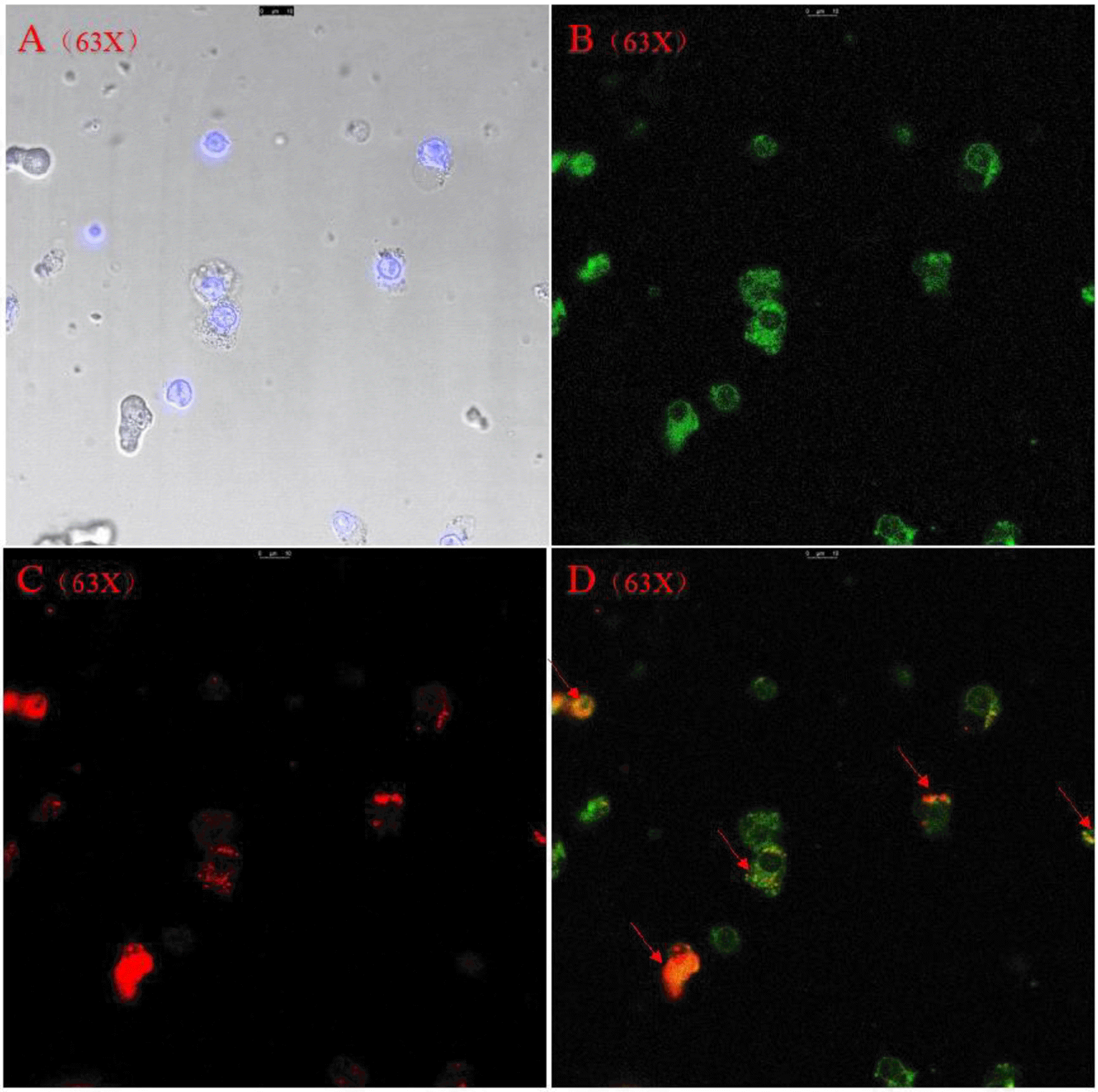
A (63X)
B (63X)
C (63X)
D (63X)

Fig. 45

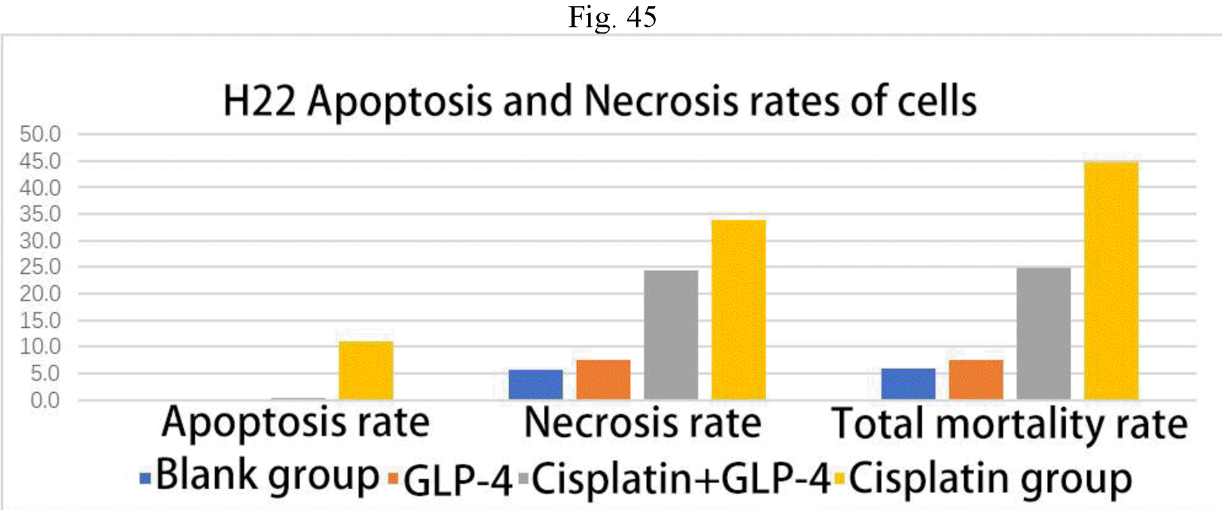
H22 Apoptosis and Necrosis rates of cells
50.0
45.0
40.0
35.0
30.0
25.0
20.0
15.0
10.0
5.0
0.0
Apoptosis rate
Necrosis rate
Total mortality rate
Blank group
GLP-4
Cisplatin+GLP-4
Cisplatin group

Fig. 46

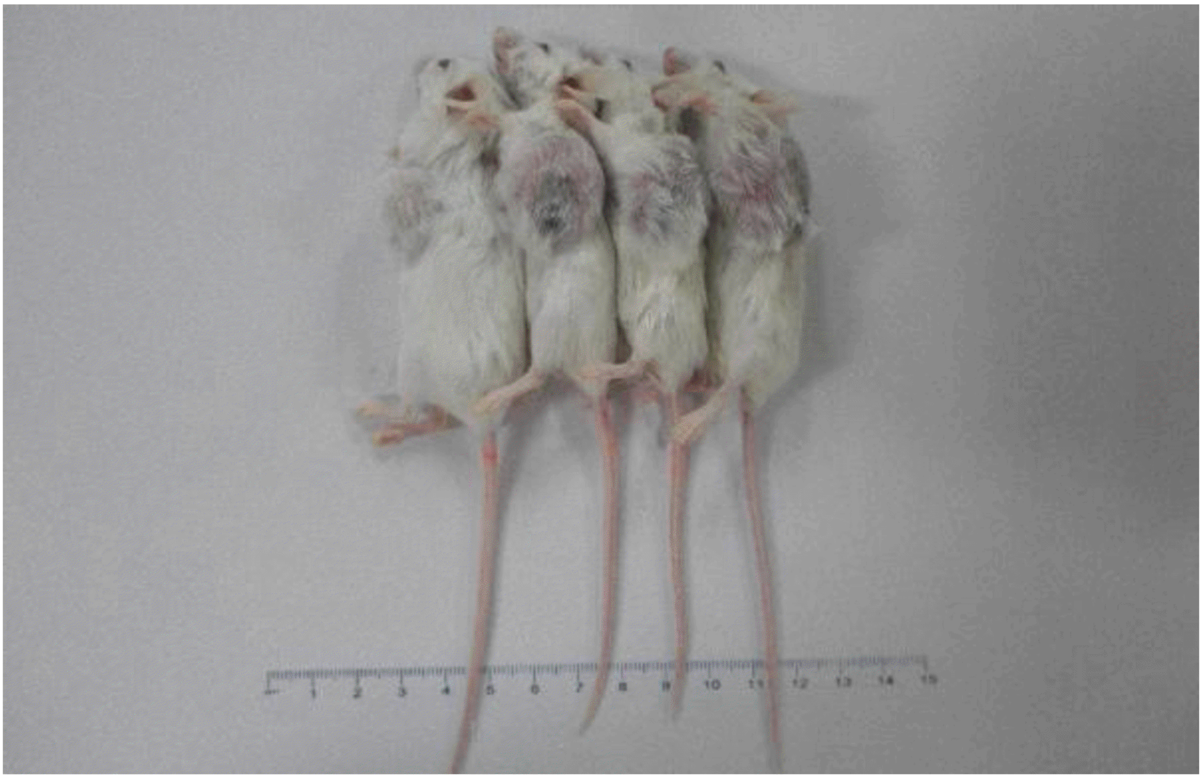

Fig. 47

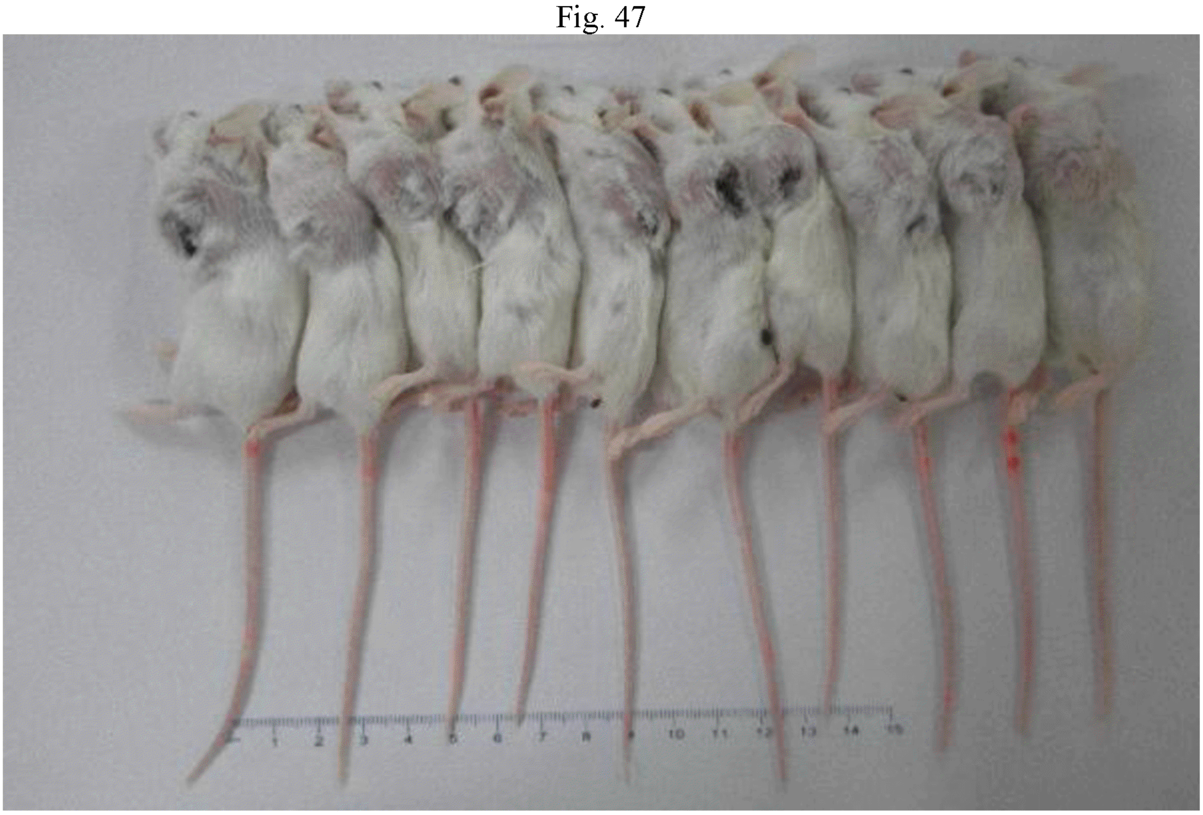

Fig. 48

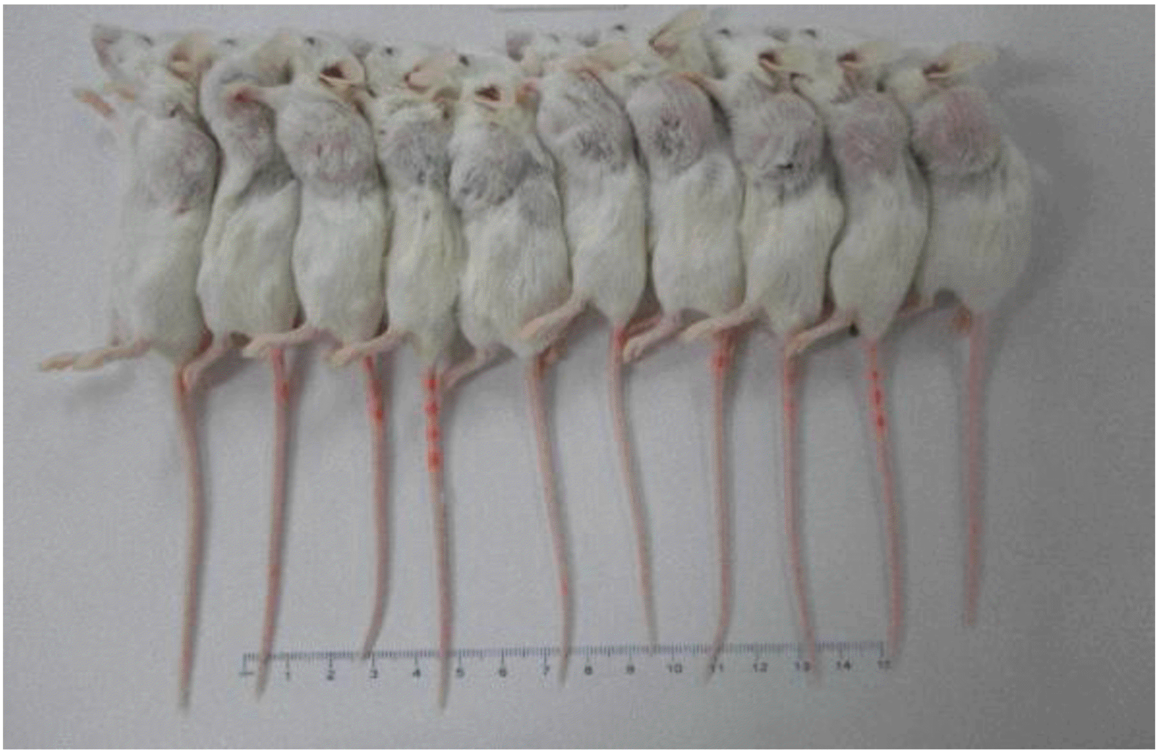

Fig. 49

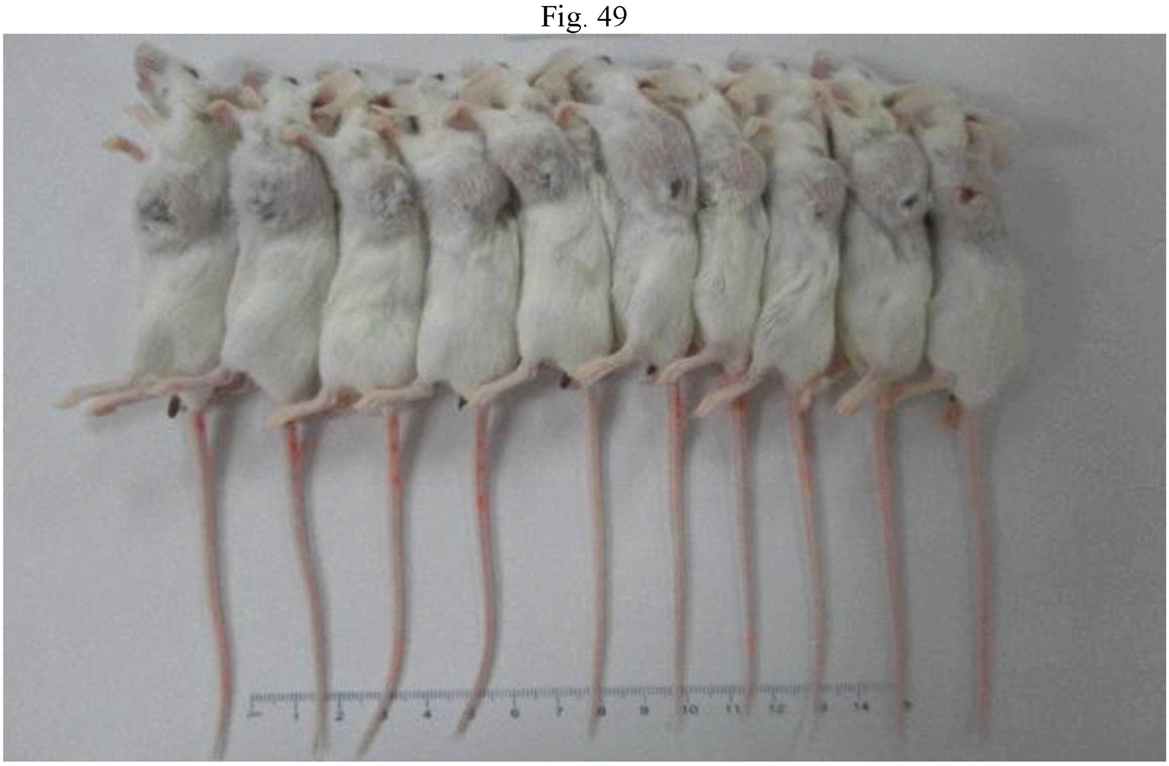

Fig. 50

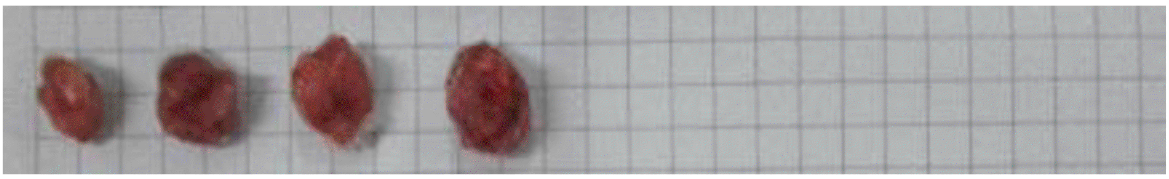

Fig. 51

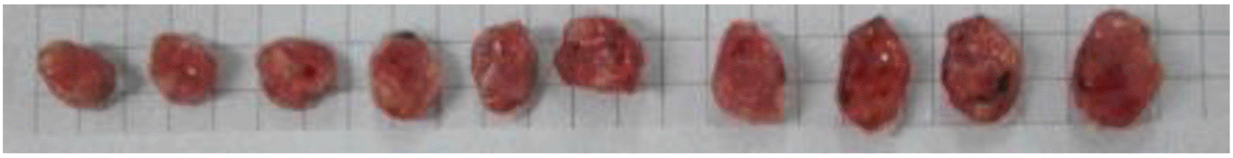

Fig. 52

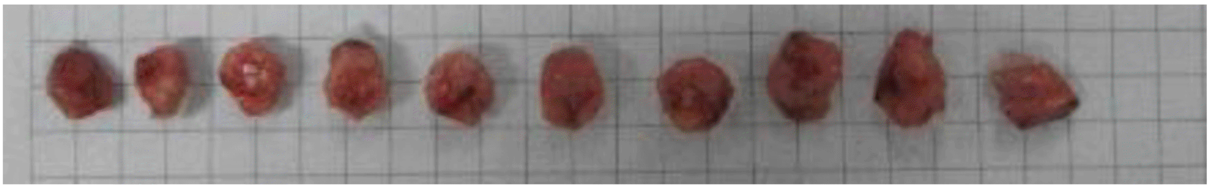

Fig. 53

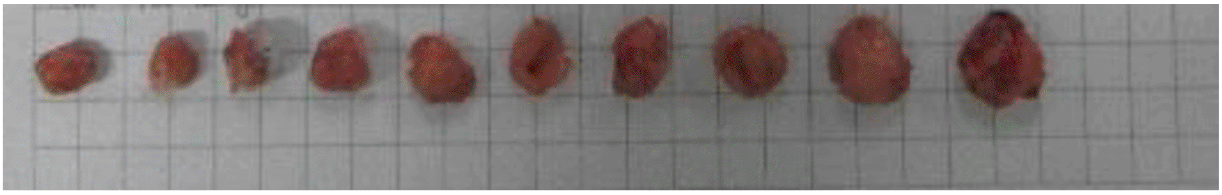

Fig. 54

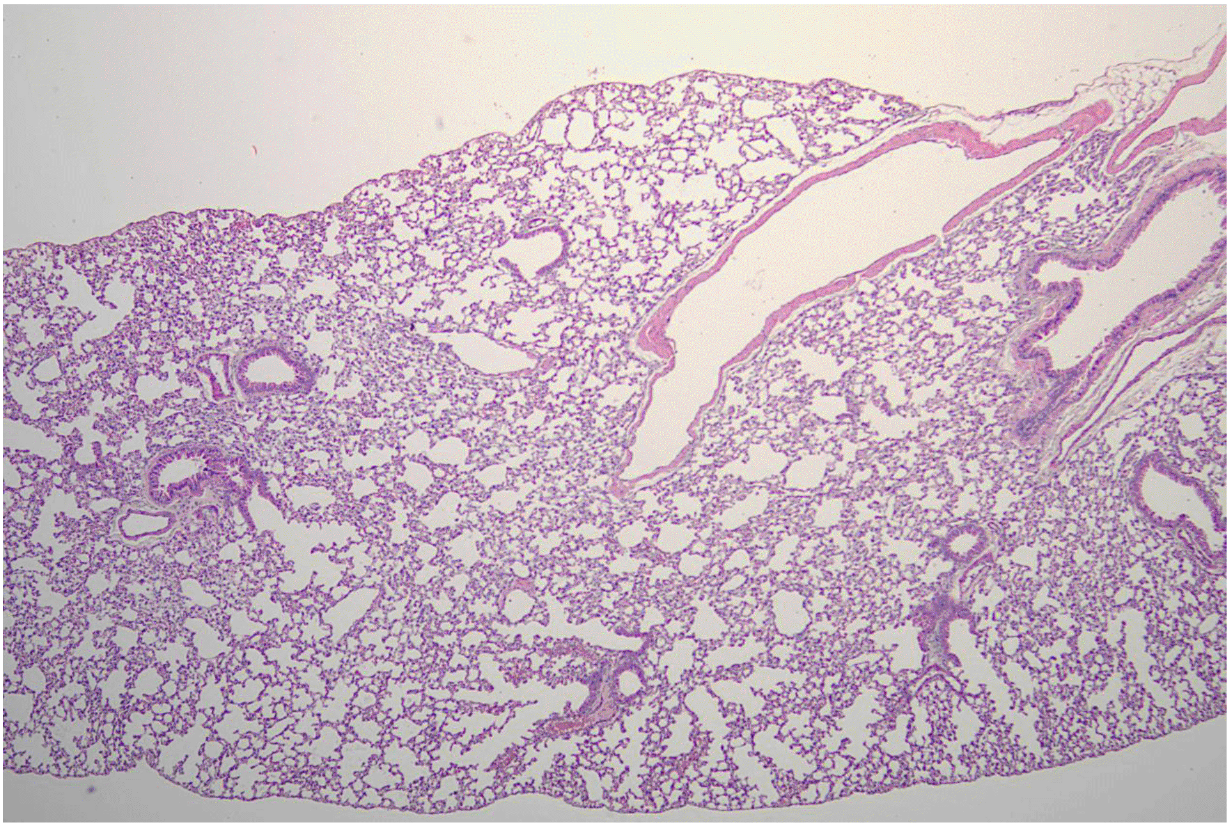

Fig. 55

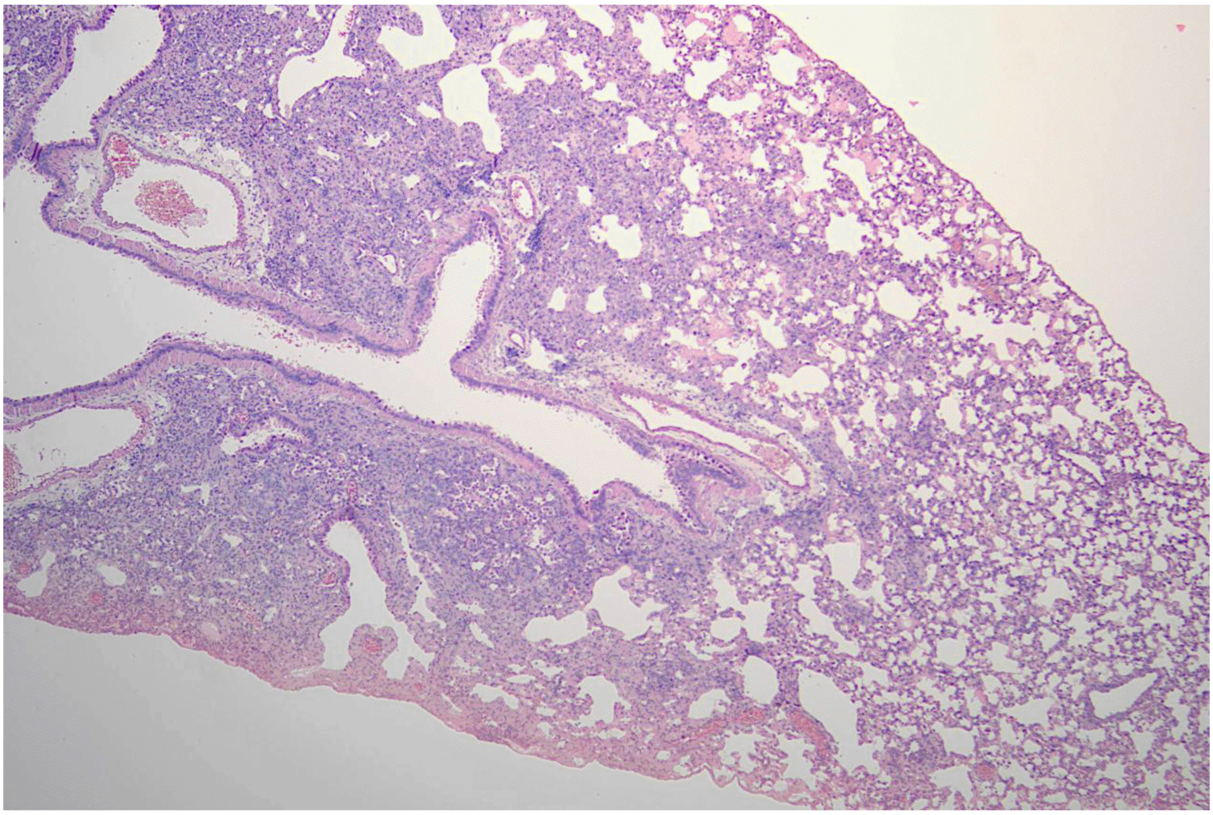

Fig. 56

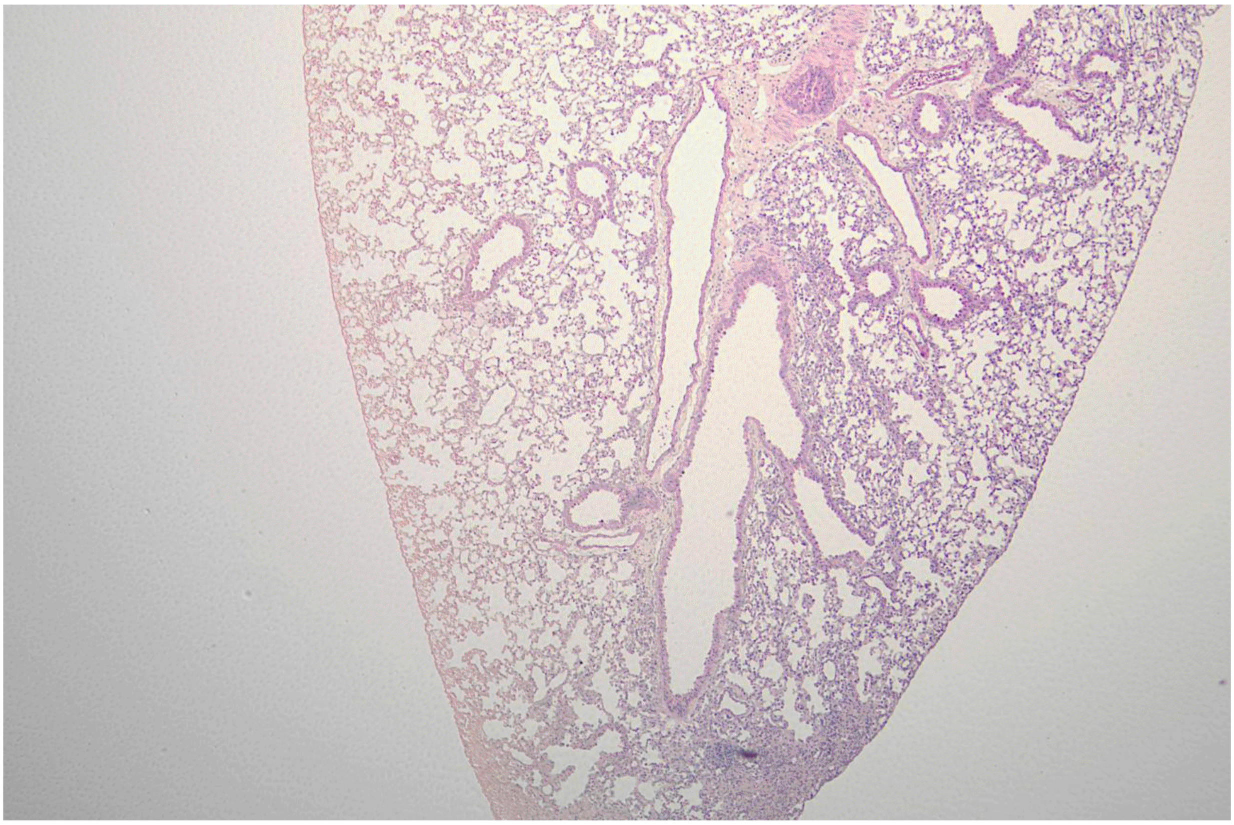

Fig. 57

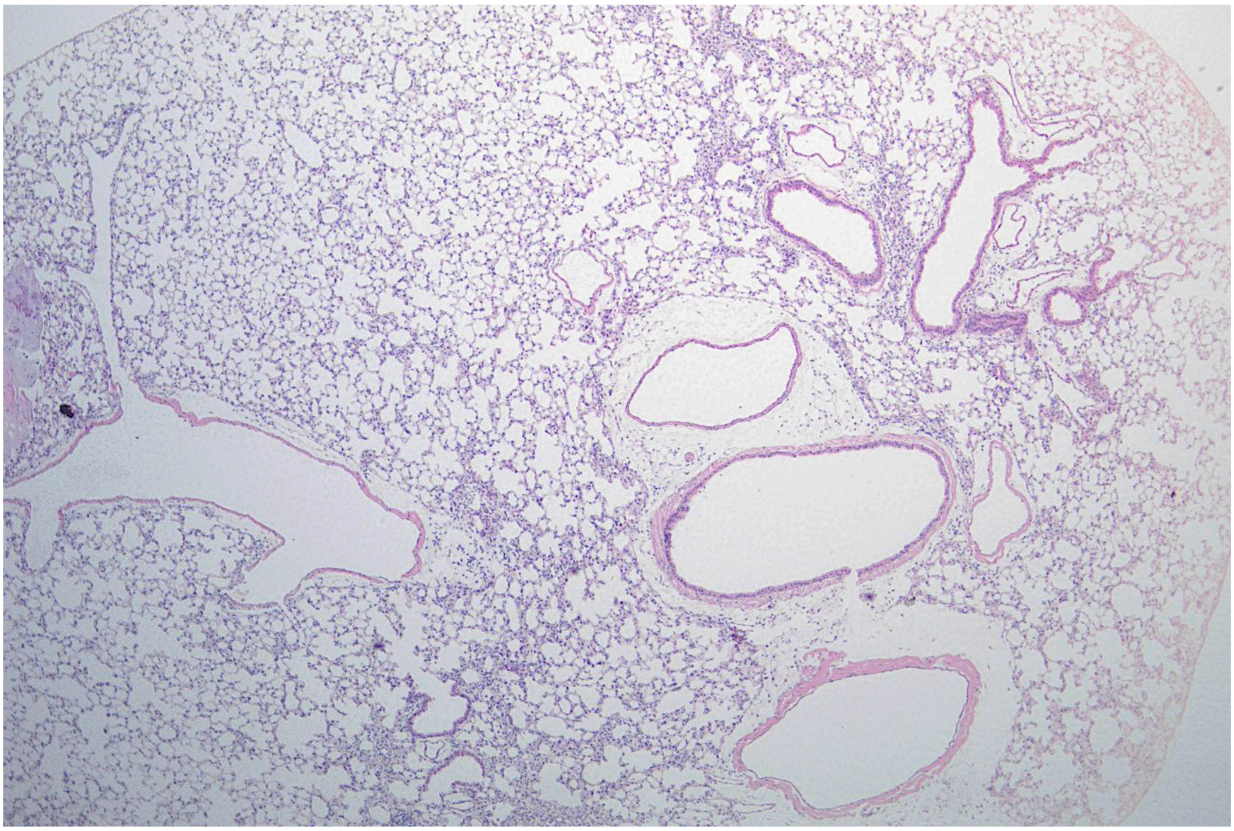

Fig. 58

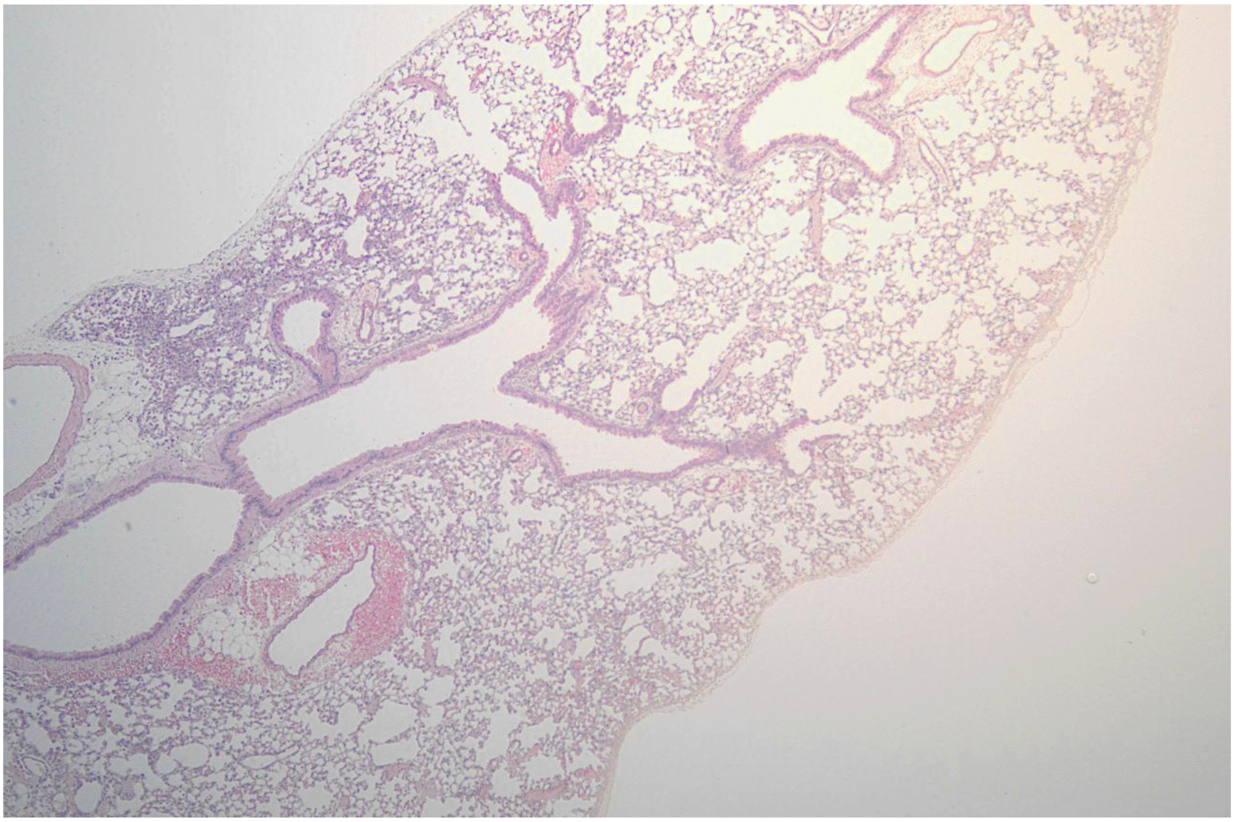

Fig. 59

**INTERNATIONAL SEARCH REPORT**

International application No.
**PCT/CN2024/080444**

**A. CLASSIFICATION OF SUBJECT MATTER**

C08B37/00(2006.01)i； A61K31/715(2006.01)i； A61K36/074(2006.01)i； A61P11/00(2006.01)i； A61P35/00(2006.01)i

According to International Patent Classification (IPC) or to both national classification and IPC

**B. FIELDS SEARCHED**

Minimum documentation searched (classification system followed by classification symbols)

C08B 37/-, A61K 31/-, A61K 36/-, A61 P11/-, A61P 35/-

Documentation searched other than minimum documentation to the extent that such documents are included in the fields searched

Electronic data base consulted during the international search (name of data base and, where practicable, search terms used)

CNTXT, WOTXT, EPTXT, USTXT, JPTXT, DWPI, WPABS, CNKI, WEB of SCIENCE: 灵芝, 多糖, 葡聚糖, 热水, 高温, 高压, 加压, 膜浓缩, 膜分离, 碱提, 柱层析, 阴离子交换, 肺气肿, 肺纤维化, 抗肿瘤, 化疗副作用, ganoderma lucidum, polysaccharide, glucan, hot water, high temperature, high pressure, membrane concentration, membrane separation, alkaline extraction, column chromatography, anion exchange, emphysema, pulmonary fibrosis, anti-tumor, chemotherapy side effects

**C. DOCUMENTS CONSIDERED TO BE RELEVANT**

| Category* | Citation of document, with indication, where appropriate, of the relevant passages | Relevant to claim No. |
|---|---|---|
| Y | CN 108976314 A (OCEAN UNIVERSITY OF CHINA) 11 December 2018 (2018-12-11)<br>description, paragraphs 0007 and 0009-0015 | 1-10 |
| Y | 周岩飞等 (ZHOU, Yanfei et al.). "高温高压水提取灵芝β-葡聚糖工艺优化 (Process Optimization of Extraction of β-glucan from Ganoderma lucidum by High Temperature and High Pressure Water)"<br>*食用菌 (Edible Fungi)*, Vol. 44, No. (1), 31 December 2022 (2022-12-31), 61-64, 69<br>introduction, and section 3 | 1-10 |
| PX | CN 117024623 A (SHENZHEN YANDAI INVESTMENT CO., LTD.) 10 November 2023 (2023-11-10)<br>claims 1-10 | 1-10 |
| A | CN 102617745 A (GUANGDONG INSTITUTE OF MICROBIOLOGY et al.) 01 August 2012 (2012-08-01)<br>entire document | 1-10 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **04 July 2024** | **05 July 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2024/080444**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **10**
because they relate to subject matter not required to be searched by this Authority, namely:

The subject matter of claim 10 relates to a method for using Ganoderma lucidum polysaccharide GLP-4 to treat a living human or animal body (PCT Rule 39.1(iv)). Nevertheless, a search is still carried out on the basis of the technical subject matter of the use of the Ganoderma lucidum polysaccharide GLP-4 in the preparation of a corresponding drug.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/080444**

| Patent document cited in search report | | | Publication date (day/month/year) | Patent family member(s) | | | Publication date (day/month/year) |
|---|---|---|---|---|---|---|---|
| CN | 108976314 | A | 11 December 2018 | None | | | |
| CN | 117024623 | A | 10 November 2023 | None | | | |
| CN | 102617745 | A | 01 August 2012 | CN | 102617745 | B | 13 November 2013 |

Form PCT/ISA/210 (patent family annex) (July 2022)